# EUROPEAN PATENT SPECIFICATION

(11) **EP 1 631 292 B1**
(45) Date of publication and mention of the grant of the patent: **05.08.2009**
(21) Application number: 04728367.6
(22) Date of filing: 20.04.2004
(51) Int. Cl.: A61K 31/517, A61P 35/00, C07D 401/12, C07D 403/12, C07D 239/94, C07D 417/12, C07D 413/12

(54) **4-ANILINO-QUINAZOLINE DERIVATIVES AS ANTIPROLIFERATIVE AGENTS**
4-ANILINO-CHINAZOLIN-DERIVATE ALS PROLIFERATIONSHEMMENDE MITTEL
DERIVES DE 4-ANILINO-QUINAZOLINE UTILISES COMME AGENTS ANTIPROLIFERATIFS

(30) Priority: 22.04.2003 GB 0309009
(43) Date of publication of application: 08.03.2006
(73) Proprietor: Astra Zeneca AB, 151 85 Södertälje (SE)
(72) Inventor: BRADBURY, Robert ,Hugh AstraZeneca R & D Alderley, Cheshire SK10 4TG (GB); KETTLE, Jason, Grant AstraZeneca R & D Alderley, Cheshire SK10 4TG (GB)
(74) Representative: Nelson, Michael Andrew
(86) International application number: PCT/GB2004/001713
(87) International publication number: WO 2004/093880

(56) References cited:
- WO-A-96/15118
- WO-A-03/040108

## Description

The invention concerns certain novel quinazoline derivatives, or pharmaceutically-acceptable salts thereof, which possess anti-tumour activity and are accordingly useful in methods of treatment of the human or animal body. The invention also concerns processes for the manufacture of said quinazoline derivatives, pharmaceutical compositions containing them and their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors as well as paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised in to 20 receptor tyrosine kinase and 10 non-receptor tyrosine kinase sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that is encoded by the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988., 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga et al., Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). Receptor mis-regulation seems to be an independent phenomenon as a diverse range of erbB receptor expression patterns have been detected in tumour tissue e.g. NSCLC (Brabender et al, Clin. Cancer Res., 2001, 7, 1850). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565). Thus, as outlined in these studies, good clinical response rates can be obtained using agents that inhibit only one member of the erbB family of receptors. Indeed, the use of selective receptor modulators will increase dosing regimen flexibility allowing maximal anti-tumour benefit whilst minimising toxicological effects.

Amplification and/or activity of members of the ErbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

International Patent Applications WO 96/33977, WO 96/33978, WO 96/33979, WO 96/33980 and WO 96/33981 disclose that certain quinazoline derivatives which bear an anilino substituent at the 4-position possess receptor tyrosine kinase inhibitory activity.

A review of the structure activity relationship of various quinazoline derivatives is disclosed by G. W. Rewcastle et al (J. Med. Chem. 1995, 38, 3428-3487), including a number of 5-substituted compounds. However, such 5-substituted compounds are stated to have low in-vitro activity as EGFR tyrosine kinase inhibitors compared to quinazolines substituted at the 6- and 7- positions.

WO 96/09294 discloses 4-anilinoquinazoline derivatives, including 5-chloro and 5-methoxy substituted quinazoline derivatives as protein tyrosine kinase inhibitors.

WO96/15118 discloses certain 4-anilinoquinazoline derivatives that are substituted on the aniline by certain aryl or heteroaryl groups. The compounds are stated to be Class 1 receptor tyrosine kinase inhibitors. International Patent Application WO 97/03069 also discloses certain 4-subsituted quinazoline derivatives and states that the compounds are erbB2 tyrosine kinase inhibitors.

WO97/30034 describes 4-anilinoquinazoline derivatives that are substituted on the aniline by certain aryl or heteroaryl groups and which are also substituted at the 6-position on the quinazoline by certain aryl or heteroaryl groups. These compounds are also Class I receptor tyrosine kinase inhibitors.

However, there is no disclosure in WO96/15118, WO 97/03069 or WO97/30034 of compounds substituted at the 5-position on the quinazoline ring.

International Patent Application WO01/94341 discloses that certain quinazoline derivatives which carry a 5-substituent are inhibitors of the Src family of non-receptor tyrosine kinases, such as c-Src, c-Yes and c-Fyn.

None of the prior art discloses 4-anilinoquinazolines which are substituted at the 5-position by a substituted or unsubstituted 2-aminoethoxy group.

We have now found that surprisingly certain 5-substituted quinazoline derivatives possess potent anti-tumour activity. In particular the compounds of the present invention are highly potent erbB2 tyrosine kinase inhibitors whilst showing significantly lower activity as EGFR tyrosine kinase inhibitors. Accordingly the compounds of the present invention are expected to be useful in the selective inhibition of erbB2 tyrosine kinase. Without wishing to imply that the compounds disclosed in the present invention possess pharmacological activity only by virtue of an effect on a single biological process, it is believed that the compounds provide an anti-tumour effect by way of inhibition of the erbB2 receptor tyrosine kinases that are involved in the signal transduction steps which lead to the proliferation of tumour cells. It is believed that the compounds of the present invention provide an anti-tumour effect by way of inhibition of erbB2 receptor tyrosine kinase, whilst possessing less potent inhibitory activity against other kinases such as EGFR tyrosine kinase, thus potentially providing effective treatment for erbB2 driven tumours. Certain of the compounds according to the present invention also exhibit favourable physical properties, such as solubility, whilst retaining a high anti-proliferative activity.

Furthermore, many of the compounds according to the present invention are inactive or only weakly active in a hERG assay.

According to a first aspect of the invention there is provided a quinazoline derivative of the formula I: 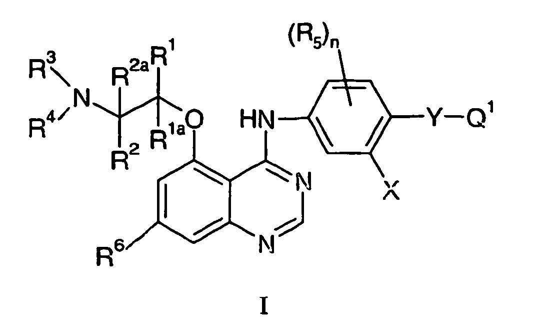 wherein:
**each of R¹ and R²,** which may be the same or different, is selected from hydrogen, carboxy, cyano, formyl, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl;
**each of R^{1a} and R^{2a},** which may be the same or different, is selected from hydrogen and (1-3C)alkyl;
**each of R³ and R⁴,** which may be the same or different, is selected from hydrogen, (1-3C)alkyl and (2-4C)alkenyl;
and wherein any CH or CH₂ or CH₃ within any of R¹, R^{1a}, R², R^{2a}, R³ and R⁴ optionally bears on each said CH or CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino; **X** is selected from hydrogen, halogeno, (1-4C)alkyl, (1-4C)alkoxy, (2-4C)alkenyl and (2-4C)alkynyl;
**each R⁵,** which may be the same or different, is selected from halogeno, hydroxy, (1-4C)alkyl, (1-4C)alkoxy, (2-4C)alkenyl and (2-4C)alkynyl;
**Y** is selected from a direct bond, O, S, OC(R⁷)₂, SC(R⁷)₂, SO, SO₂, N(R⁷), CO and N(R⁷)C(R⁷)₂ wherein each R⁷ is, independently, hydrogen or (1-6C)alkyl;
**Q¹** is selected from phenyl, pyridyl, pyrazinyl, 1,3-thiazolyl, 1H-imidazolyl, 1H-pyrazolyl, 1,3-oxazolyl and isoxazolyl,
and wherein Q¹ optionally bears one or more substituents (for example 1, 2 or 3), which may be the same or different, selected from halogeno, cyano, nitro, hydroxy, amino, carboxy, carbamoyl, sulfamoyl, formyl, mercapto, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino, and N-(1-6C)alkyl-(1-6C)alkanesulfonylamino, or from a group of the formula:

   -X¹-R⁸
wherein X¹ is a direct bond or is selected from O, CO and N(R⁹), wherein R⁹ is hydrogen or (1-6C)alkyl, and R⁸ is halogeno-(1-6C)alkyl, hydroxy-(1-6C)alkyl, carboxy-(1-6C)alkyl, (1-6C)alkoxy-(1-6C)alkyl; cyano-(1-6C)alkyl, amino-(1-6C)alkyl, N-(1-6C)alkylamino-(1-6C)alkyl, N,N-di-[(1-6C)alkyl]amino-(1-6C)alkyl, (2-6C)alkanoylamino-(1-6C)alkyl, (1-6C)alkoxycarbonylamino-(1-6C)alkyl, carbamoyl-(1-6C)alkyl, N-(1-6C)alkylcarbamoyl-(1-6C)alkyl N,N-di-[(1-6C)alkyl]carbamoyl-(1-6C)alkyl, (2-6C)alkanoyl-(1-6C)alkyl or (1-6C)alkoxycarbonyl-(1-6C)alkyl,
and wherein any CH₂ or CH₃ within a substituent on Q¹ optionally bears on each said CH₂ or CH₃ one or more (for example 1, 2, or 3) halogeno or (1-6C)alkyl substituents or a substituent selected from hydroxy, cyano, amino, (1-4C)alkoxy, (1-4C)alkylamino and di-[(1-4C)alkyl]amino;
**R⁶** is selected from hydrogen, (1-6C)alkoxy, (2-6C)alkenyloxy and (2-6C)alkynyloxy,
and wherein any CH₂ or CH₃ group within a R⁶ substituent optionally bears on each said CH₂ or CH₃ group one or more halogeno or (1-6C)alkyl substituents, or a substituent selected from hydroxy and (1-6C)alkoxy;
**n** is 0, 1, 2 nor 3;
or a pharmaceutically acceptable salt thereof.

According to a further aspect of the invention there is provided a quinazoline derivative of the formula I wherein each of **X, R⁵, Y, Q¹, R⁶ and n** has any of the meaning as hereinbefore defined;
**each of R¹ and R²,** which may be the same or different, is selected from hydrogen, carboxy, cyano, formyl, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl;
**each of R^{1a}, R^{2a}, R³ and R⁴,** which may be the same or different, is selected from hydrogen and (1-3C)alkyl;
and wherein any CH₂ or CH₃ within any of R¹, R^{1a}, R², R^{2a}, R³ and R⁴ optionally bears on each said CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino;
or a pharmaceutically acceptable salt thereof.

In this specification the generic term "alkyl" includes both straight-chain and branched-chain alkyl groups such as propyl, isopropyl and tert-butyl, and (3-6C)cycloalkyl groups such as cyclopropyl, cyclobutyl, cyclopentyl and cyclohexyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclohexyl" are specific for that 6-membered ring only. An analogous convention applies to other generic terms, for example (1-6C)alkoxy includes methoxy, ethoxy, cyclopropyloxy and cyclopentyloxy, (1-6C)alkylamino includes methylamino, ethylamino, cyclobutylamino and cyclohexylamino, and di-[(1-6C)alkyl]amino includes dimethylamino, diethylamino, N-cyclobutyl-N-inethylamino and N-cyclohexyl-N-ethylamino.

It is to be understood that, insofar as certain of the compounds of formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

It is to be understood that the present invention includes in its definition any and all tautomeric forms of the compounds of the formula I which possess the above mentioned activity.

It is also to be understood that in so far as certain compounds of the formula 1 may exist in solvated forms as well as unsolvated forms, for example, hydrated forms, the present invention includes any and all such solvated forms, which possess the above mentioned activity.

Suitable values for the generic radicals referred to above and hereinafter include those set out below.

Suitable values for any of the 'R' groups (R¹ to R⁹), X, Y or for any of the various groups within Q¹ include:

| | |
|---|---|
| for halogeno | fluoro, chloro, bromo and iodo; |
| for (1-6C)alkyl: | methyl, ethyl, propyl, isopropyl, butyl and tert-butyl; |
| for (2-8C)alkenyl: | vinyl, isopropenyl, allyl and but-2-enyl; |
| for (2-8C)alkynyl: | ethynyl, 2-propynyl and but-2-ynyl; |
| for (1-6C)alkoxy: | methoxy, ethoxy, propoxy, isopropoxy and butoxy; |
| for (2-6C)alkenyloxy: | vinyloxy and allyloxy; |
| for (2-6C)alkynyloxy: | ethynyloxy and 2-propynyloxy; |
| for (1-6C)alkylthio: | methylthio, ethylthio and propylthio; |
| for (1-6C)alkylsulfinyl: | methylsulfinyl and ethylsulfinyl; |
| for (1-6C)alkylsulfonyl: | methylsulfonyl and ethylsulfonyl; |
| for (1-6C)alkylamino: | methylamino, ethylamino, propylamino, |
| | isopropylamino and butylamino; |
| for di-[(1-6C)alkyl]amino: | dimethylamino, diethylamino, N-ethyl- |
| | N-methylamino and diisopropylamino; |
| for (1-6C)alkoxycarbonyl: | methoxycarbonyl, ethoxycarbonyl, propoxycarbonyl |
| | and tert-butoxycarbonyl; |
| for N-(1-6C)alkylcarbamoyl: | N-methylcarbamoyl, N-ethylcarbamoyl, |
| | N-propylcarbamoyl and N-isopropylcarbamoyl; |
| for N,N-di-[(1-6C)alkyl]carbamoyl: | N,N-dimethylcarbamoyl, N-ethyl- |
| | N-methylcarbamoyl and N,N-diethylcarbamoyl; |
| for (2-6C)alkanoyl: | acetyl and propionyl; |
| for (2-6C)alkanoyloxy: | acetoxy and propionyloxy; |
| for (2-6C)alkanoylamino: | acetamido and propionamido; |
| for N-(1-6C)alkyl-(2-6C)alkanoylamino: | N-methylacetamido and N-methylpropionamido; |
| for (3-6C)alkenoylamino: | acrylamido, methacrylamido and crotonamido; |
| for N-(1-6C)alkyl-(3-6C)alkenoylamino: | N-methylacrylamido and N-methylcrotonamido; |
| for (3-6C)alkynoylamino: | propiolamido; |
| for N-(1-6C)alkyl-(3-6C)alkynoylamino: | N-methylpropiolamido; |
| for N-(1-6C)alkylsulfamoyl: | N-methylsulfamoyl and N-ethylsulfamoyl; |
| for N,N-di-[(1-6C)alkyl]sulfamoyl: | N,N-dimethylsulfamoyl; |
| for (1-6C)alkanesulfonylamino: | methanesulfonylamino and ethanesulfonylamino; |
| for N-(1-6C)alkyl-(1-6C)alkanesulfonylamino: | N-methylmethanesulfonylamino and |
| | N-methylethanesulfonylamino; |
| for amino-(1-6C)alkyl: | aminomethyl, 2-aminoethyl, 1-aminoethyl and |
| | 3-aminopropyl; |
| for N-(1-6C)alkylamino-(1-6C)alkyl: | methylaminomethyl, ethylaminomethyl, |
| | 1-methylaminoethyl, 2-methylaminoethyl, |
| | 2-ethylaminoethyl and 3-methylaminopropyl; |
| for N,N-di-[(1-6C)alkyl]amino-(1-6C)alkyl: | dimethylaminomethyl, diethylaminomethyl, |
| | 1-dimethylaminoethyl, 2-dimethylaminoethyl and |
| | 3-dimethylaminopropyl; |
| for halogeno-(1-6C)alkyl: | chloromethyl, 2-chloroethyl, 1-chloroethyl and |
| | 3-chloropropyl; |
| for hydroxy-(1-6C)alkyl: | hydroxymethyl, 2-hydroxyethyl, 1-hydroxyethyl, |
| | 2-hydroxypropyl and 3-hydroxypropyl; |
| for (1-6C)alkoxy-(1-6C)alkyl: | methoxymethyl, ethoxymethyl, 1-methoxyethyl, |
| | 2-methoxyethyl, 2-ethoxyethyl and |
| | 3-methoxypropyl; |
| for carboxy-(1-6C)alkyl: | carboxymethyl and 2-carboxyethyl; |
| for cyano-(1-6C)alkyl: | cyanomethyl, 2-cyanoethyl, 1-cyanoethyl and |
| | 3-cyanopropyl; |
| for carbamoyl-(1-6C)alkyl: | carbamoylmethyl and 2-carbamoylethyl; |
| for alkanoyl-(1-6C)alkyl: | acetylmethyl and 2-acetylethyl; |
| N-(1-6C)alkylcarbamoyl-(1-6C)alkyl: | N-methylcarbamoylmethyl, |
| | N-ethylcarbamoylmethyl and 2- |
| | N-methylcarbamoylethyl; |
| for N,N-di-[(1-6C)alkyl]carbamoyl-(1-6C)alkyl: | N,N-dimethylcarbamoylmethyl, N-ethyl- |
| | N-methylcarbamoylmethyl and 2- |
| | N,N-diethylcarbamoylethyl; |
| for (1-6C)alkoxycarbonyl-(1-6C)alkyl: | methoxycarbonylmethyl, 2-methoxycarbonylethyl |
| | and 2-ethoxycarbonylethyl; |
| for (2-6C)alkanoylamino-(1-6C)alkyl: | acetamidomethyl, propionamidomethyl and |
| | 2-acetamidoethyl; |
| for (1-6C)alkoxycarbonylamino-(1-6C)alkyl: | methoxycarbonylaminomethyl, |
| | ethoxycarbonylaminomethyl, |
| | tert-butoxycarbonylaminomethyl and |
| | 2-methoxycarbonylaminoethyl; |

When in this specification reference is made to a (1-3C)aikyl or (1-4C)alkyl group it is to be understood that such groups refer to alkyl groups containing up to 3 or 4 carbon atoms respectively, for example methyl, ethyl, isopropyl or, in the case of C4 alkyl butyl and tert-butyl. A similar convention is adopted for the other groups listed above, for example (1-3C)alkoxy, (2-4C)alkenyl, (2-4C)alkynyl and (2-3C)alkanoyl.

When, as defined hereinbefore, in the group of the formula Y is, for example, a OC(R⁷)₂ linking group, it is the oxygen atom, not the carbon atom, of the OC(R⁷)₂ linking group which is attached to the phenyl ring in the formula I and the carbon atom is attached to the Q¹ group. A similar convention applies when Y is SC(R⁷)₂ or N(R⁷)C(R⁷)₂.

When, as defined hereinbefore, any CH or CH₂ or CH₃ group within a R¹, R^{1a}, R², R^{2a}, R³ or R⁴ group optionally bears on each said CH or CH₂ or CH₃ group one or more halogeno substituents, there is suitably 1 halogeno substituent on each said CH group, there are suitably 1 or 2 halogeno substituents present on each said CH₂ group and there are suitably 1, 2 or 3 such substituents present on each said CH₃ group.

When, as defined hereinbefore, any CH or CH₂ or CH₃ group within a R¹, R^{1a}, R², R^{2a}, R³ or R⁴ group optionally bears on each said CH or CH₂ or CH₃ group a substituent as defined hereinbefore, suitable substituents so formed include, for example, hydroxy-substituted alkyl groups such as hydroxymethyl or 2-hydroxyethyl, halogen substituted alkyl groups such as di-fluoromethyl, trifluoromethyl and 2,2-difluoroethyl, (1-3C)alkoxy substituted alkyl groups such as 2-methoxyethyl or amino substituted alkyl groups such as 2-aminoethyl.

When, as defined hereinbefore, any CH₂ or CH₃ group within a substituent on Q¹ optionally bears on each said CH₂ or CH₃ group a substituent as defined hereinbefore, suitable substituents so formed include, for example, hydroxy-substituted alkyl groups such as hydroxymethyl or 2-hydroxyethyl, hydroxy substituted (2-6C)alkanoyl groups such as hydroxyacetyl, halogen substituted alkyl groups, for example di-fluoromethyl and 2,2-difluoroethyl, halogen substituted (2-6C)alkanoyl groups such as fluoroacetyl or trifluoroacetyl, amino substituted alkyl groups such as 2-aminoethyl or amino substituted (2-6C)alkanoyl groups such as aminoacetyl.

Similar considerations apply to the substitutions within the R⁶ group.

A suitable pharmaceutically-acceptable salt of a quinazoline derivative of the formula I is, for example, an acid-addition salt of a quinazoline derivative of the formula I, for example an acid-addition salt with an inorganic or organic acid such as hydrochloric, hydrobromic, sulfuric, trifluoroacetic, citric or maleic acid; or, for example, a salt of a quinazoline derivative of the formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel compounds of the invention include, for example, quinazoline derivatives of the formula I, or pharmaceutically-acceptable salts thereof, wherein, unless otherwise stated, each of R¹, R^{1a}, R², R^{2a} R³, R⁴, R⁵, R⁶, n, X, Y and Q¹ has any of the meanings defined hereinbefore or in paragraphs (a) to (xxxxxxx) hereinafter:
(a) each of R^{1a} and R^{2a}, which may be the same or different, is selected from hydrogen and methyl;
(b) R^{1a} is (1-3C)alkyl (particularly methyl) and R^{2a} is hydrogen;
(c) R^{2a} is (1-3C)alkyl (particularly methyl) and R^{1a} is hydrogen;
(d) R^{1a} and R^{2a} are both hydrogen;
(e) R¹ is selected from hydrogen and (1-3C)alkyl (particularly hydrogen and methyl);
(f) R² is selected from hydrogen and (1-3C)alkyl (particularly hydrogen and methyl);
(g) R¹ is selected from hydrogen and (1-3C)alkyl, and R² is selected from hydrogen, carboxy, cyano, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl, and wherein any CH₂ or CH₃ within any of R¹ and R² optionally bears on each said CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino;
(h) R¹ is selected from hydrogen, methyl and ethyl, and R² is selected from hydrogen, carboxy, cyano, methyl, ethyl, acetyl, methoxycarbonyl, carbamoyl, N-methylcarbamoyl and N, N-di-methylcarbamoyl, and wherein any CH₂ which is attached to two carbon atoms or any CH₃ which is attached to a carbon atom within any of R¹ and R² optionally bears on each said CH₂ or CH₃ one or more (for example 1, 2 or 3) halogeno substituents or a substituent selected from hydroxy, (1-3C)alkoxy, amino, (1-3C)alkylamino and di-[(1-3C)alkyl]amino;
(i) R¹ is selected from hydrogen, methyl and ethyl, R² is selected from hydrogen, carboxy, cyano, methyl, ethyl, acetyl, methoxycarbonyl, carbamoyl, N-methylcarbamoyl and N, N-di-methylcarbamoyl, and R^{1a} and R^{2a} are hydrogen;
(j) R¹ and R^{1a} are hydrogen, R² is selected from hydrogen, carboxy, cyano, methyl, ethyl, acetyl, methoxycarbonyl, carbamoyl, N-methylcarbamoyl and N, N-di-methylcarbamoyl, and R^{2a} is selected from hydrogen and (1-3C)alkyl;
(k) R² is selected from hydrogen and (1-3C)alkyl, and R¹ is selected from hydrogen, carboxy, cyano, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl, and wherein any CH₂ or CH₃ within any of R¹ and R² optionally bears on each said CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino;
(l) R² is selected from hydrogen, methyl and ethyl, and R¹ is selected from hydrogen, carboxy, cyano, methyl, ethyl, acetyl, methoxycarbonyl, carbamoyl, N-methylcarbamoyl and N, N-di-methylcarbamoyl, and wherein any CH₂ which is attached to two carbon atoms or any CH₃ which is attached to a carbon atom within any of R¹ and R² optionally bears on each said CH₂ or CH₃ one or more (for example 1, 2 or 3) halogeno substituents or a substituent selected from hydroxy, (1-3C)alkoxy, amino, (1-3C)alkylamino and di-[(1-3C)alkyl]amino;
(m) R² is selected from hydrogen, methyl and ethyl, R¹ is selected from hydrogen, carboxy, cyano, methyl, ethyl, acetyl, methoxycarbonyl, carbamoyl, N-methylcarbamoyl and N, N-di-methylcarbamoyl, and R^{1a} and R^{2a} are hydrogen;
(n) R² and R^{2a} are hydrogen, R¹ is selected from hydrogen, carboxy, cyano, methyl, ethyl, acetyl, methoxycarbonyl, carbamoyl, N-methylcarbamoyl and N, N-di-methylcarbamoyl, and R^{1a} is selected from hydrogen and (1-3C)alkyl;
(o) R¹ is hydrogen or methyl, and R^{1a} and R^{2a} are hydrogen;
(p) R² is hydrogen or methyl, and R^{1a} and R^{2a} are hydrogen;
(q) each of R¹ and R^{1a}, which may be the same or different, is selected from hydrogen and methyl, and R^{2a} is hydrogen;
(r) each of R² and R^{2a}, which may be the same or different, is selected from hydrogen and methyl, and R^{1a} is hydrogen;
(s) R¹ is (1-3C)alkyl, and R², R^{1a} and R^{2a} are hydrogen;
(t) R² is (1-3C)alkyl, and R¹, R^{1a} and R^{2a} are hydrogen;
(u) each of R¹ and R^{1a}, which may be the same or different, is selected from (1-3C)alkyl, and R² and R^{2a} are hydrogen;
(v) each of R² and R^{2a}, which may be the same or different, is selected from (1-3C)alkyl, and R¹ and R^{1a} are hydrogen;
(w) R¹ is methyl, and R², R^{1a} and R^{2a} are hydrogen;
(x) R² is methyl, and R¹, R^{1a} and R^{2a} are hydrogen;
(y) R¹ and R^{1a} are methyl, and R² and R^{2a} are hydrogen;
(z) R² and R^{2a} are methyl, and R¹ and R^{1a} are hydrogen;

(aa) each of R³ and R⁴, which may be the same or different, is selected from hydrogen and (1-3C)alkyl, and wherein any CH₂ or CH₃ within any of R³ and R⁴ optionally bears on each said CH₂ or CH₃ a substituent selected from cyano and (2-3C)alkanoyl, and wherein any CH₂ or CH₃ which is not attached to a nitrogen atom within any of R³ and R⁴ optionally bears on each said CH₂ or CH₃ one or more (for example 1, 2 or 3) halogeno substituents or a substituent selected from hydroxy, (1-3C)alkoxy, amino, (1-3C)alkylamino and di-[(1-3C)alkyl]amino;
(bb) each of R³ and R⁴, which may be the same or different, is selected from hydrogen, (1-3C)alkyl and (2-4C)alkenyl, and wherein any CH or CH₂ or CH₃ within any of R³ and R⁴ optionally bears on each said CH or CH₂ or CH₃ a substituent selected from cyano and (2-3C)alkanoyl, and wherein any CH or CH₂ or CH₃ which is not attached to a nitrogen atom within any of R³ and R⁴ optionally bears on each said CH or CH₂ or CH₃ one or more (for example 1, 2 or 3) halogeno substituents or a substituent selected from hydroxy, (1-3C)alkoxy, amino, (1-3C)alkylamino and di-[(1-3C)alkyl]amino;
(cc) each of R³ and R⁴, which may be the same or different, is selected from hydrogen and (1-3C)alkyl, and wherein any CH₂ or CH₃ within any of R³ and R⁴ optionally bears on each said CH₂ or CH₃ a substituent selected from cyano and acetyl, and wherein any CH₂ or CH₃ which is not attached to a nitrogen atom within any of R³ and R⁴ optionally bears on each said CH₂ or CH₃ one or more (for example 1, 2 or 3) substituents selected from fluoro and chloro, or a substituent selected from hydroxy and methoxy;
(dd) each of R³ and R⁴, which may be the same or different, is selected from hydrogen, (1-3C)alkyl and (2-4C)alkenyl, and wherein any CH or CH₂ or CH₃ within any of R³ and R⁴ optionally bears on each said CH or CH₂ or CH₃ a substituent selected from cyano and acetyl, and wherein any CH or CH₂ or CH₃ which is not attached to a nitrogen atom within any of R³ and R⁴ optionally bears on each said CH or CH₂ or CH₃ one or more (for example 1, 2 or 3) substituents selected from fluoro and chloro, or a substituent selected from hydroxy and methoxy;
(ee) each of R³ and R⁴, which may be the same or different, is selected from (1-3C)alkyl,
and wherein any CH or CH₂ or CH₃ within any of R³ and R⁴ optionally bears on each said CH or CH₂ or CH₃ one or more substituents selected from hydroxy and (1-3C)alkoxy; (ff) R³ is selected from hydrogen, methyl and ethyl;
(gg) R³ is selected from hydrogen and methyl;
(hh) R³ is selected from methyl and ethyl;
(ii) R³ is selected from hydrogen and methyl and R⁴ is (1-3C)alkyl, and wherein any CH₂ or CH₃ which is not attached to a nitrogen atom within any of R³ and R⁴ optionally bears on each said CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkyl]amino;
(jj) R³ is selected from methyl and ethyl (particularly methyl) and R⁴ is selected from (1-3C)alkyl and (2-4C)alkenyl, and wherein any CH or CH₂ or CH₃ which is not attached to a nitrogen atom within any of R³ and R⁴ optionally bears on each said CH or CH₂ or CH₃ one or more (for example 1, 2 or 3) halogeno substituents or a substituent selected from hydroxy, cyano, (1-3C)alkoxy, amino, (1-3C)alkylanvno and di-[(1-3C)alkyl]amino;
(kk) R³ is selected from hydrogen and methyl (particularly methyl) and R⁴ is (1-3C)alkyl, and wherein any CH₂ or CH₃ which is not attached to a nitrogen atom within any of R³ and R⁴ optionally bears on each said CH₂ or CH₃ one or more (for example 1, 2 or 3) substituents selected from fluoro and chloro or a substituent selected from hydroxy and cyano;
(II) R³ is selected from methyl and ethyl (particularly methyl) and R⁴ is selected from (1-3C)alkyl and (2-4C)alkenyl, and wherein any CH or CH₂ or CH₃ which is not attached to a nitrogen atom within any of R³ and R⁴ optionally bears on each said CH or CH₂ or CH₃ one or more (for example 1, 2 or 3) substituents selected from fluoro and chloro or a substituent selected from hydroxy, methoxy and cyano;
(mm) R³ is selected from hydrogen and methyl (particularly methyl) and R⁴ is (1-2C)alkyl, and wherein any CH₃ which is not attached to a nitrogen atom within any of R³ and R⁴ optionally bears on each CH₃ a substituent selected from fluoro, chloro, hydroxy, cyano and methoxy;
(nn) R³ is selected from methyl and ethyl (particularly methyl) and R⁴ is (1-2C)alkyl, and wherein any CH₃ which is not attached to a nitrogen atom within any of R³ and R⁴ optionally bears on each CH₃ a substituent selected from fluoro, chloro, hydroxy, cyano and methoxy;
(oo) R³ is selected from methyl and ethyl (particularly methyl) and R⁴ is (2-4C)alkenyl, and wherein any CH or CH₂ or CH₃ which is not attached to a nitrogen atom within any of R³ and R⁴ optionally bears on each said CH or CH₂ or CH₃ one or more (for example 1, 2 or 3) substituents selected from fluoro and chloro or a substituent selected from hydroxy, methoxy and cyano;
(pp) R³ is methyl and R⁴ is selected from methyl, ethyl, 2-fluoroethyl, 2-chloroethyl, 2-hydroxyethyl, 2-methoxyethyl, cyanomethyl and 2-cyanoethyl;
(qq) R³ is methyl and R⁴ is selected from methyl, ethyl, 2-fluoroethyl, 2-chloroethyl, 2-hydroxyethyl, 2-methoxyethyl, propenyl, cyanomethyl and 2-cyanoethyl;
(rr) R³ is methyl and R⁴ is selected from methyl, ethyl, 2-hydroxyethyl, 2-methoxyethyl and propenyl;
(ss) R³ is methyl and R⁴ is selected from methyl and 2-hydroxyethyl;
(tt) R³ is methyl and R⁴ is selected from methyl and propenyl;
(uu) R³ is methyl and R⁴ is selected from methyl and 2-methoxyethyl;
(vv) R³ is methyl and R⁴ is selected from methyl and ethyl;
(ww) R³ and R⁴ are methyl;
(xx) R³ is ethyl and R⁴ is 2-hydroxyethyl;
(yy) each of R³ and R⁴, which may be the same or different, is selected from hydrogen, methyl, ethyl and 2-hydroxyethyl;
(zz) each of R³ and R⁴, which may be the same or different, is selected from hydrogen, methyl, ethyl, propenyl, 2-methoxyethyl and 2-hydroxyethyl;

(aaa) each of R³ and R⁴, which may be the same or different, is selected from methyl, ethyl, propenyl, 2-methoxyethyl and 2-hydroxyethyl;
(bbb) R³ is methyl, R⁴ is selected from methyl, ethyl, 2-fluoroethyl, 2-chloroethyl, 2-hydroxyethyl, 2-methoxyethyl, cyanomethyl and 2-cyanoethyl and (i) R¹, R², R^{1a} and R^{2a} are hydrogen, or (ii) R¹ is methyl and R², R^{1a} and R^{2a} are hydrogen, or (iii) R¹, R^{1a} and R^{2a} are hydrogen and R² is methyl;
(ccc) R³ is methyl, R⁴ is selected from methyl, ethyl, 2-fluoroethyl, 2-chloroethyl, 2-hydroxyethyl, 2-methoxyethyl, propenyl, cyanomethyl and 2-cyanoethyl and (i) R¹, R², R^{1a} and R^{2a} are hydrogen, or (ii) R¹ is methyl and R², R^{1a} and R^{2a} are hydrogen, or (iii) R¹, R^{1a} and R^{2a} are hydrogen and R² is methyl, or (iv) R¹ and R^{1a} are methyl and R² and R^{2a} are hydrogen, or (v) R¹ and R^{1a} are hydrogen and R² and R^{2a} are methyl;
(ddd) R³ is methyl, R⁴ is selected from methyl, ethyl, 2-hydroxyethyl, 2-methoxyethyl and propenyl and (i) R¹, R², R^{1a} and R^{2a} are hydrogen, or (ii) R¹ is methyl and R², R^{1a} and R^{2a} are hydrogen, or (iii) R¹, R^{1a} and R^{2a} are hydrogen and R² is methyl, or (iv) R¹ and R^{1a} are methyl and R² and R^{2a} are hydrogen, or (v) R¹ and R^{1a} are hydrogen and R² and R^{2a} are methyl;
(eee) R³ and R⁴ are methyl and (i) R¹, R², R^{1a} and R^{2a} are hydrogen, or (ii) R¹ is methyl and R², R^{1a} and R^{2a} are hydrogen, or (iii) R¹, R^{1a} and R^{2a} are hydrogen and R² is methyl, or (iv) R¹ and R^{1a} are methyl and R² and R^{2a} are hydrogen, or (v) R¹ and R^{1a} are hydrogen and R² and R^{2a} are methyl;
(fff) R³ is ethyl, R⁴ is 2-hydroxyethyl and (i) R¹, R², R^{1a} and R^{2a} are hydrogen, or (ii) R¹ is methyl and R², R^{1a} and R^{2a} are hydrogen, or (iii) R¹, R^{1a} and R^{2a} are hydrogen and R² is methyl, or (iv) R¹ and R^{1a} are methyl and R² and R^{2a} are hydrogen, or (v) R¹ and R^{1a} are hydrogen and R² and R^{2a} are methyl;
(ggg) X is selected from hydrogen, halogeno, (1-4C)alkyl, (1-4C)alkoxy and (2-4C)alkynyl;
(hhh) X is selected from hydrogen, halogeno, (1-4C)alkyl and (1-4C)alkoxy;
(iii) X is selected from hydrogen, halogeno, (1-4C)alkoxy and (2-4C)alkynyl;
(jjj) X is selected from hydrogen and halogeno;
(kkk) X is selected from hydrogen, fluoro, chloro and bromo;
(lll) X is selected from hydrogen, fluoro, chloro, methyl, methoxy and ethynyl;
(mmm)X is selected from hydrogen, fluoro, chloro, methyl and methoxy;
(nnn) X is selected from hydrogen, chloro, methyl and methoxy;
(ooo) X is selected from hydrogen, fluoro, chloro and methoxy;
(ppp) X is selected from hydrogen, fluoro, chloro and methyl;
(qqq) X is selected from hydrogen, chloro and methoxy;
(rrr) X is selected from hydrogen, chloro and methyl;
(sss) X is selected from hydrogen and chloro;
(ttt) X is selected from methyl and chloro;
(uuu) X is selected from methoxy and chloro;
(vvv) X is hydrogen;
(www) X is chloro;
(xxx) X is fluoro;
(yyy) X is methyl
(zzz) X is methoxy;

(aaaa) Y is selected from O, S, OC(R⁷)₂ and N(R⁷)C(R⁷)₂ wherein each R⁷ is, independently, hydrogen or (1-4C)alkyl;
(bbbb) Y is selected from O, S and OC(R⁷)₂ wherein each R⁷ is, independently, hydrogen or (1-4C)alkyl;
(cccc) Y is selected from S and OC(R⁷)₂ wherein each R⁷ is, independently, hydrogen or (1-4C)alkyl;
(dddd) Y is selected from O and OC(R⁷)₂ wherein each R⁷ is, independently, hydrogen or (1-4C)alkyl;
(eeee) Y is selected from O, S, OCH₂ and NHCH₂;
(ffff) Y is selected from O, S and OCH₂;
(gggg) Y is selected from S and OCH₂ ;
(hhhh) Y is selected from O and OCH₂ ;
(iiii) Y is O;
(jjjj) Y is S;
(kkkk) Y is OCH₂;
(llll) Y is OCH₂ and X is selected from hydrogen, methyl, methoxy, fluoro and chloro;
(mmmm)Y is OCH₂ and X is selected from methyl and chloro;
(nnnn)Y is OCH₂ and X is selected from hydrogen and chloro;
(oooo) Y is OCH₂ and X is selected from methoxy and chloro;
(pppp) Y is OCH₂ and X is chloro;
(qqqq) Y is OCH₂ and X is methyl;
(rrrr) Y is S and X is chloro;
(ssss) Y is O and X is selected from chloro and methoxy;
(tttt) Y is O and X is chloro;
(uuuu) Y is O and X is methoxy;
(vvvv) n is 0, 1 or 2 and each R⁵, which may be the same or different, is selected from halogeno;
(wwww) n is 0 or 1 and R⁵ is selected from fluoro and chloro;
(xxxx) n is 0;
(yyyy) n is 1 and R⁵ is fluoro or chloro, and R⁵ is in an ortho position to the NH group;
(zzzz) Q¹ is selected from phenyl, 2-,3- or 4-pyridyl, 2-pyrazinyl, 1,3-thiazol-2-yl, 1,3-thiazol-4-yl, 1,3-thiazol-5-yl, 1H-pyrazol-3-yl, 1H-pyrazol-4-yl, 1H-pyrazol-5-yl, 1H-imidazol-2-yl, 1H-imidazol-4-yl, 1H-imidazol-5-yl, 1,3-oxazol-2-yl, 1,3-oxazol-4-yl, 1,3-oxazol-5-yl, isoxazol-3-yl, isoxazol-4-yl and isoxazol-5-yl,
   and wherein Q¹ optionally bears one or more substituents (for example 1, 2 or 3), which may be the same or different, selected from halogeno, cyano, nitro, hydroxy, amino, carboxy, carbamoyl, sulfamoyl, formyl, mercapto, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino, and N-(1-6C)alkyl-(1-6C)alkanesulfonylamino, or from a group of the formula:

   -X¹-R⁸
wherein X¹ is a direct bond or is selected from O, CO and N(R⁹), wherein R⁹ is hydrogen or (1-6C)alkyl, and R⁸ is halogeno-(1-6C)alkyl, hydroxy-(1-6C)alkyl, carboxy-(1-6C)alkyl, (1-6C)alkoxy-(1-6C)alkyl, cyano-(1-6C)alkyl, amino-(1-6C)alkyl, N-(1-6C)alkylamino-(1-6C)alkyl, N,N-di-[(1-6C)alkyl]amino-(1-6C)alkyl, (2-6C)alkanoylamino-(1-6C)alkyl, (1-6C)alkoxycarbonylamino-(1-6C)alkyl, carbamoyl-(1-6C)alkyl, N-(1-6C)alkylcarbamoyl-(1-6C)alkyl, N,N-di-[(1-6C)alkyl]carbamoyl-(1-6C)alkyl, (2-6C)alkanoyl-(1-6C)alkyl or (1-6C)alkoxycarbonyl-(1-6C)alkyl,
and wherein any CH₂ or CH₃ within a substituent on Q¹ optionally bears on each said CH₂ or CH₃ one or more (for example 1, 2, or 3) halogeno or (1-6C)alkyl substituents or a substituent selected from hydroxy, cyano, amino, (1-4C)alkoxy, (1-4C)alkylamino and di-[(1-4C)alkyl]amino;
(aaaaa) Q¹ is selected from phenyl, 2-,3- or 4-pyridyl, 2-pyrazinyl, 1,3-thiazol-2-yl, 1,3-thiazol-4-yl, 1,3-thiazol-5-yl, 1H-pyrazol-3-yl, 1H-pyrazol-4-yl, 1H-pyrazol-5-yl, 1H-imidazol-2-yl, 1H-imidazol-4-yl, 1H-imidazol-5-yl, isoxazol-3-yl, isoxazol-4-yl and isoxazol-5-yl, and wherein Q¹ optionally bears one or more substituents (for example 1, 2 or 3), which may be the same or different, as defined above in (zzzz);
(bbbbb) Q¹ is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl, 1,3-oxazol-2-yl and isoxazol-3-yl, and wherein Q¹ optionally bears one or more substituents (for example 1, 2 or 3), which may be the same or different, as defined above in (zzzz);
(ccccc) Q¹ is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl and isoxazol-3-yl, and wherein Q¹ optionally bears one or more substituents (for example 1, 2 or 3), which may be the same or different, as defined above in (zzzz);
(ddddd) Q¹ is selected from phenyl, 2-pyridyl, 2-pyrazinyl, 1,3-thiazol-4-yl, 1,3-thiazol-5-yl, 1H-imidazol-2-yl and isoxazol-3-yl, and wherein Q¹ optionally bears one or more substituents (for example 1, 2 or 3), which may be the same or different, as defined above in (zzzz);
(eeeee) Q¹ is phenyl, which optionally bears one or more substituents (for example 1, 2 or 3), which may be the same or different, as defined above in (zzzz);
(fffff) Q¹ is pyrazinyl (particularly 2-pyrazinyl), which optionally bears one or more substituents (for example 1, 2 or 3), which may be the same or different, as defined above in (zzzz);
(ggggg) Q¹ is 1H-imidazolyl (particularly 1H-imidazol-2-yl), which optionally bears one or more substituents (for example 1, 2 or 3), which may be the same or different, as defined above in (zzzz);
(hhhhh) Q¹ is 1H-pyrazolyl (particularly 1H-pyrazol-3-yl), which optionally bears one or more substituents (for example 1, 2 or 3), which may be the same or different, as defined above in (zzzz);
(iiiii) Q¹ is isoxazolyl (particularly isoxazol-3-yl), which optionally bears one or more substituents (for example 1, 2 or 3), which may be the same or different, as defined above in (zzzz);
(jjjjj) Q¹ is pyridyl (particularly 2-pyridyl), which optionally bears one or more substituents (for example 1, 2 or 3), which may be the same or different, as defined above in (zzzz);
(kkkkk) Q¹ is 1,3-thiazolyl (particularly 1,3-thiazol-4-yl or 1,3-thiazol-5-yl), which optionally bears one or more substituents (for example 1, 2 or 3), which may be the same or different, as defined above in (zzzz);
(lllll) Q¹ is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl and isoxazol-3-yl,
   and wherein Q¹ optionally bears one or more substituents (for example 1, 2 or 3), which may be the same or different selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl;
(mmmmm) Q¹ is selected from phenyl, 2-pyridyl, 2-pyrazinyl, 1,3-thiazol-4-yl, 1,3-thiazol-5-yl, 1H-imidazol-2-yl and isoxazol-3-yl, and wherein Q¹ optionally bears one or more substituents (for example 1, 2 or 3), which may be the same or different, as defined above in (lllll);
(nnnnn) Q¹ is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl and isoxazol-3-yl, and wherein Q¹ optionally bears one or more substituents (for example 1, 2 or 3), which may be the same or different, selected from fluoro, chloro, bromo, hydroxy, carboxy, cyano, nitro, amino, methyl, ethyl, isopropyl, methoxy, ethoxy, vinyl, allyl, ethynyl, 2-propynyl, methylthio, methylsulfinyl, methylsulfonyl, acetyl, propionyl methylamino, ethylamino, N,N-dimethylamino, N,N-diethylamino, N-methyl-N-ethylamino methoxycarbonyl, ethoxycarbonyl, carbamoyl, N-methylcarbamoyl, N,N-dimethylcarbamoyl, acetoxy, acetamido, fluoromethyl, 2-fluoroethyl, chloromethyl, 2-chloroethyl, hydroxymethyl, 2-hydroxyethyl, methoxymethyl, 2-methoxyethyl, cyanomethyl, 2-cyanoethyl, carboxymethyl, 2-carboxymethyl, aminomethyl, methylaminomethyl, ethylaminomethyl, N,N-dimethylaminomethyl, N,N-diethylaminomethyl, N-methyl-N-ethylaminomethyl, 2-aminoethyl, 2-(methylamino)ethyl, 2-(ethylamino)ethyl, 2-(N,N-dimethylamino)ethyl, 2-(N,N-diethylamino)ethyl, 2-(N-methyl-N-ethylamino)ethyl, carbamoylmethyl, N-methylcarbamoylmethyl and N,N-dimethylcarbamoylmethyl;
(ooooo) Q¹ is selected from phenyl, 2-pyridyl, 2-pyrazinyl, 1H-imidazol-2-yl and 3-isoxazolyl, and wherein Q¹ optionally bears 1, 2, or 3 substituents, which may be the same or different, as defined above in (nnnnn);
(ppppp) Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears 1, 2, or 3 substituents, which may be the same or different, as defined above in (nnnnn);
(qqqqq) Q¹ is selected from phenyl, 2-pyridyl, 2-pyrazinyl, 1H-imidazol-2-yl and 3-isoxazolyl, and wherein Q¹ optionally bears 1, 2, or 3 substituents, which may be the same or different, selected from halogeno, hydroxy, amino, N-(1-4C)alkylamino, N, N-di-[(1-4C)alkyl]amino cyano, nitro, (1-4C)alkyl, (1-4C)alkoxy, halogeno-(1-4C)alkyl, hydroxy-(1-4C)alkyl, (1-4C)alkoxy-(1-4C)alkyl and cyano-(1-4C)alkyl;
(rrrrr) Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears 1, 2, or 3 substituents, which may be the same or different, as defined above in (qqqqq);
(sssss) Q¹ is selected from phenyl, 2-pyridyl, 2-pyrazinyl, 1H-imidazol-2-yl and 3-isoxazolyl, and wherein Q¹ optionally bears 1, 2, or 3 substituents, which may be the same or different, selected from halogeno, hydroxy, cyano, nitro, (1-4C)alkyl and (1-4C)alkoxy;
(ttttt) Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears 1, 2, or 3 substituents, which may be the same or different, as defined above in (sssss);
(uuuuu) Q¹ is selected from phenyl, 2-pyridyl, 2-pyrazinyl, 1H-imidazol-2-yl and 3-isoxazolyl, and wherein Q¹ optionally bears 1, 2, or 3 (particularly 1 or 2) substituents, which may be the same or different, selected from fluoro, chloro, cyano, (1-4C)alkyl and (1-4C)alkoxy;
(vvvvv) Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears 1, 2, or 3 (particularly 1 or 2) substituents, which may be the same or different, as defined above in (uuuuu)
(wwwww) Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears 1, 2, or 3 (particularly 1 or 2) substituents, which may be the same or different, selected from fluoro and (1-4C)alkyl (particularly methyl);
(xxxxx) Q¹ is selected from phenyl, 2-pyridyl, 2-pyrazinyl and 3-isoxazolyl,
   and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl, and Y is OCH₂;
(yyyyy) Q¹ is selected from phenyl, 2-pyridyl, 2-pyrazinyl, 1,3-thiazol-4-yl, 1,3-thiazol-5-yl and 3-isoxazolyl, and wherein Q¹ optionally bears one or more substituents (for example 1, 2, or 3), which may be the same or different, as defined above in (xxxxx), and Y is OCH₂;
(zzzzz) Q¹ is selected from phenyl, 2-pyridyl, 2-pyrazinyl and 3-isoxazolyl,
   and wherein Q¹ optionally bears one or more substituents (for example 1, 2, or 3), which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino, and Y is OCH₂;

(aaaaaa) Q¹ is selected from phenyl, 2-pyridyl, 2-pyrazinyl, 1,3-thiazol-4-yl, 1,3-thiazol-5-yl and 3-isoxazolyl, and wherein Q¹ optionally bears one or more substituents (for example 1, 2, or 3), which may be the same or different, as defined above in (zzzzz), and Y is OCH₂;
(bbbbbb) Q¹ is selected from phenyl, 2-pyridyl and 2-pyrazinyl, and wherein Q¹ optionally bears 1 or 2 halogeno substituents, which may be the same or different, (particularly fluoro or chloro, more particularly fluoro), and Y is OCH₂;
(cccccc) Q¹ is 3-fluorophenyl and Y is OCH₂;
(dddddd) Q¹ is 3-isoxazolyl, and wherein Q¹ optionally bears 1 or 2 (1-4C)alkyl substituents (particularly methyl), and Y is OCH₂;
(eeeeee) Q¹ is selected from 3-fluorophenyl, 2-pyridyl and 2-pyrazinyl, and Y is OCH₂;
(ffffff) Q¹ is selected from 1,3-thiazol-4-yl, 1,3-thiazol-5-yl, 5-methyl-isoxazol-3-yl, 3-fluorophenyl, 2-pyridyl and 2-pyrazinyl, and Y is OCH₂;
(gggggg) Q¹ is selected from 2-pyridyl and 2-pyrazinyl and Y is OCH₂;
(hhhhhh) Q¹ is 5-methyl-isoxazol-3-yl and Y is OCH₂;
(iiiiii) Q¹ is 1H-imidazol-2-yl which optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N,N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl, and Y is S;
(jjjjjj) Q¹ is 1H-imidazol-2-yl which optionally bears one or more substituents (for example 1, 2, or 3), which may be the same or different, selected from halogen, hydroxy, cyano, nitro, amino, (1-4C)alkyl, (1-4C)alkoxy, (2-4C)alkanoyl, N-(1-4C)alkylamino and N, N-di-[(1-4C)alkyl]amino, and Y is S;
(kkkkkk) Q¹ is 1-methyl-1H-imidazol-2-yl and Y is S;
(llllll) Q¹ is selected from phenyl and 2-pyridyl,
   and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl, and Y is O;
(mmmmmm) Q¹ is selected from phenyl and 2-pyridyl,
   and wherein Q¹ optionally bears one or more substituents (for example 1, 2, or 3), which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino, and Y is O;
(nnnnnn) Q¹ is selected from phenyl and 2-pyridyl, and wherein Q¹ optionally bears 1 or 2 halogeno substituents, which may be the same or different, (particularly fluoro or chloro, more particularly fluoro), and Y is O;
(oooooo) Q¹ is selected from phenyl and 2-pyridyl and Y is O;
(pppppp) Q¹ is phenyl which optionally bears one or more substituents (for example 1, 2, or 3), which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(qqqqqq) Q¹ is phenyl which optionally bears one or more substituents (for example 1, 2, or 3), which may be the same or different, selected from fluoro, chloro, bromo, cyano, methyl and methoxy;
(rrrrrr) Q¹ is phenyl which bears 1 or 2 substituents, which may be the same or different, selected from halogeno (particularly fluoro and chloro, more particularly fluoro); (ssssss) Q¹ is 3-fluorophenyl;
(tttttt) Q¹ is phenyl;
(uuuuuu) Q¹ is 2-pyridyl which optionally bears one or more substituents (for example 1, 2, or 3), which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(vvvvvv) Q¹ is 2-pyridyl which optionally bears 1 or 2 substituents selected from (1-4C)alkyl;
(wwwwww) Q¹ is 2-pyridyl which optionally bears 1 or 2 substituents, which may be the same or different, selected from fluoro, chloro, bromo, cyano, methyl and methoxy;
(xxxxxx) Q¹ is 2-pyridyl;
(yyyyyy) Q¹ is 2-pyrazinyl which optionally bears one or more substituents (for example 1, 2, or 3), which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(zzzzzz) Q¹ is 2-pyrazinyl which optionally bears 1 or 2 substituents, which may be the same or different, selected from hydroxy, (1-4C)alkyl and (1-4C)alkoxy;

(aaaaaaa) Q¹ is 2-pyrazinyl which optionally bears 1 or 2 substituents, which may be the same or different, selected from fluoro, chloro, bromo, cyano, methyl and methoxy;
(bbbbbbb) Q¹ is 2-pyrazinyl;
(ccccccc) Q¹ is 1H-imidazol-2-yl which optionally bears one or more substituents (for example 1, 2, or 3), which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(ddddddd) Q¹ is 1H-imidazol-2-yl which optionally bears 1 or 2 substituents, which may be the same or different, selected from (1-4C)alkyl;
(eeeeeee) Q¹ is 1H-imidazol-2-yl which bears a (1-4C)alkyl at the 1-postion and optionally bears a further substituent selected from (1-4C)alkyl;
(fffffff) Q¹ is 1H-imidazol-2-yl which bears a substituent at the 1-position selected from methyl and ethyl, and wherein Q¹ optionally bears a further substituent selected from fluoro, chloro and (1-4C)alkyl;
(ggggggg) Q¹ is selected from 1H-imidazol-2-yl and 1-methyl-1H-imidazol-2-yl; (hhhhhhh) Q¹ is 1-methyl-1H-imidazol-2-yl;
(iiiiiii) Q¹ is 3-isoxazolyl which optionally bears 1 or 2 substituents, which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(jjjjjjj) Q¹ is 3-isoxazolyl which optionally bears 1 or 2 substituents, which may be the same or different, selected from (1-4C)alkyl;
(kkkkkkk) Q¹ is 5-methyl-3-isoxazolyl;
(lllllll) Q¹ is selected from 1,3-thiazol-4-yl and 1,3-thiazol-5-yl, and wherein Q¹ optionally bears one or more substituents (for example 1, 2, or 3), which may be the same or different, selected from halogeno, hydroxy, cyano, nitro, amino, (1-4C)alkyl, (1-4C)alkoxy, (2-4C)alkenyl, (2-4C)alkynyl, (2-4C)alkanoyl, N-(1-4C)alkylamino and N, N-di-[(1-4C)alkyl]amino;
(mmmmmmm) Q¹ is selected from 1,3-thiazol-4-yl and 1,3-thiazol-5-yl, and wherein Q¹ optionally bears 1 or 2 substituents selected from (1-4C)alkyl;
(nnnnnnn) Q¹ is selected from 1,3-thiazol-4-yl and 1,3-thiazol-5-yl, and wherein Q¹ optionally bears 1 or 2 substituents, which may be the same or different, selected from fluoro, chloro, bromo, cyano, methyl and methoxy;
(ooooooo) Q¹ is 1,3-thiazol-4-yl;
(ppppppp) Q¹ is 1,3-thiazol-5-yl;
(qqqqqqq) Q¹ is selected from 3-fluorophenyl, 2,3-difluorophenyl, 2-pyridyl, 2-pyrazinyl, 1,3-thiazol-2-yl, 1,3-thiazol-4-yl, 1,3-thiazol-5-yl, 1-methyl-1H-pyrazol-3-yl, 1,5-dimethyl-1H-pyrazol-3-yl, 1-methyl-1H-imidazol-2-yl and 5-methyl-3-isoxazolyl;
(rrrrrrr) Q¹ is selected from 3-fluorophenyl, 2-pyridyl, 2-pyrazinyl, 1-methyl-1H-imidazol-2-yl, 1,3-thiazol-4-yl, 1,3-thiazol-5-yl and 5-methyl-3-isoxazolyl;
(sssssss) Q¹ is selected from 3-fluorophenyl, 2-pyridyl, 2-pyrazinyl, 1-methyl-1H-imidazol-2-yl and 5-methyl-3-isoxazolyl;
(ttttttt) R⁶ is selected from hydrogen, (1-6C)alkoxy and (1-6C)alkoxy-(1-6C)alkoxy, and wherein any CH₂ group attached to 2 carbon atoms or and CH₃ group attached to a carbon atom within a R⁶ substituent optionally bears on each said CH₂ or CH₃ group one or more substituents, which may be the same or different, selected from fluoro and chloro, or optionally bears a hydroxy substituent, for example R⁶ is selected from hydrogen methoxy, ethoxy, isopropyloxy, cyclopropylmethoxy, 2-hydroxyethoxy, 2-fluoroethoxy, 2-methoxyethoxy, 2-ethoxyethoxy, 2,2-difluoroethoxy, 2,2,2-trifluoroethoxy and 3-hydroxy-3-methylbutoxy;
(uuuuuuu) R⁶ is selected from hydrogen, (1-4C)alkoxy and (1-4C)alkoxy(1-4C)alkoxy;
(vvvvvvv) R⁶ is selected from hydrogen, methoxy, ethoxy and 2-methoxyethoxy;
(wwwwwww) R⁶ is (1-4C)alkoxy (particularly methoxy); and (xxxxxxx) R⁶ is hydrogen.

A further aspect of the present invention is a quinazoline derivative of the formula **I**
wherein:
**each of R¹ and R²,** which may be the same or different, is selected from hydrogen, carboxy, cyano, formyl, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl;
**each of R^{1a} and R^{2a},** which may be the same or different, is selected from hydrogen and (1-3C)alkyl;
**each of R³ and R⁴,** which may be the same or different, is selected from (1-3C)alkyl and (2-4C)alkenyl;
and wherein any CH or CH₂ or CH₃ within any of R¹, R^{1a}, R², R^{2a}, R³ and R⁴ optionally bears on each said CH or CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino;
**X** is selected from hydrogen, halogeno, (1-4C)alkyl, (1-4C)alkoxy, (2-4C)alkenyl and (2-4C)alkynyl;
**each R⁵,** which may be the same or different, is selected from halogeno, hydroxy, (1-4C)alkyl, (1-4C)alkoxy, (2-4C)alkenyl and (2-4C)alkynyl;
**Y** is selected from a direct bond, O, S, OC(R⁷)₂, SC(R⁷)₂, SO, SO₂, N(R⁷), CO and N(R⁷)C(R⁷)₂ wherein each R⁷ is, independently, hydrogen or (1-6C)alkyl;
**Q¹** is selected from phenyl, pyridyl, pyrazinyl, 1,3-thiazolyl, 1H-imidazolyl, 1H-pyrazolyl, 1,3-oxazolyl and isoxazolyl,
and wherein Q¹ optionally bears one or more substituents (for example 1, 2 or 3), which may be the same or different, selected from halogeno, cyano, nitro, hydroxy, amino,
   carboxy, carbamoyl, sulfamoyl, formyl, mercapto, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino, and N-(1-6C)alkyl-(1-6C)alkanesulfonylamino, or from a group of the formula:

   -X¹-R⁸
wherein X¹ is a direct bond or is selected from O, CO and N(R⁹), wherein R⁹ is hydrogen or (1-6C)alkyl, and R⁸ is halogeno-(1-6C)alkyl, hydroxy-(1-6C)alkyl, carboxy-(1-6C)alkyl,
(1-6C)alkoxy-(1-6C)alkyl, cyano-(1-6C)alkyl, amino-(1-6C)alkyl, N-(1-6C)alkylamino-(1-6C)alkyl, N,N-di-[(1-6C)alkyl]amino-(1-6C)alkyl, (2-6C)alkanoylamino-(1-6C)alkyl, (1-6C)alkoxycarbonylamino-(1-6C)alkyl, carbamoyl-(1-6C)alkyl, N-(1-6C)alkylcarbamoyl-(1-6C)alkyl, N,N-di-[(1-6C)alkyl]carbamoyl-(1-6C)alkyl, (2-6C)alkanoyl-(1-6C)alkyl or (1-6C)alkoxycarbonyl-(1-6C)alkyl,
and wherein any CH₂ or CH₃ within a substituent on Q¹ optionally bears on each said CH₂ or CH₃ one or more (for example 1, 2, or 3) halogeno or (1-6C)alkyl substituents or a substituent selected from hydroxy, cyano, amino, (1-4C)alkoxy, (1-4C)alkylamino and di-[(1-4C)alkyl]amino;
**R⁶** is selected from hydrogen, (1-6C)alkoxy, (2-6C)alkenyloxy and (2-6C)alkynyloxy, and wherein any CH₂ or CH₃ group within a R⁶ substituent optionally bears on each said CH₂ or CH₃ group one or more halogeno or (1-6C)alkyl substituents, or a substituent selected from hydroxy and (1-6C)alkoxy;
**n** is 0, 1, 2 or 3; or a pharmaceutically acceptable salt thereof.

A further aspect of the present invention is a quinazoline derivative of the formula I
wherein:
**each of R¹, R², R³ and R⁴,** which may be the same or different, is selected from hydrogen and (1-3C)alkyl,
and wherein any CH₂ or CH₃ within any of R¹, R², R³ and R⁴ optionally bears on each said CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkyl]amino;
**R^{1a} and R^{2a}** are hydrogen;
**X** is selected from hydrogen, fluoro, chloro, and methoxy;
**R⁵** is selected from fluoro and chloro;
**Y** is selected from O, S and OCH₂ (particularly S and OCH₂, more particularly OCH₂);
**Q¹** is selected from phenyl, 2-,3- or 4-pyridyl, 2-pyrazinyl, 1,3-thiazol-2-yl, 1,3-thiazol-4-yl, 1,3-thiazol-5-yl, 1H-pyrazol-3-yl, 1H-pyrazol-4-yl, 1H-pyrazol-5-yl, 1H-imidazol-2-yl, 1H-imidazol-4-yl, 1H-imidazol-5-yl, isoxazol-3-yl, isoxazol-4-yl and isoxazol-5-yl,
and wherein Q¹ optionally bears one or more substituents (for example 1, 2 or 3), which may be the same or different selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is selected from hydrogen, (1-4C)alkoxy and (1-4C)alkoxy(1-4C)alkoxy (particularly R⁶ is hydrogen or methoxy, more particularly R⁶ is hydrogen); and
n is 0 or 1 (particularly n is 0);
or a pharmaceutically acceptable salt thereof.

In one aspect of this embodiment of the invention when n is 1, R⁵ may be in an ortho position to the NH group. In another aspect of this embodiment of the invention when n is 1, R⁵ is in the ortho position to the Y-Q¹ group.

According to a further aspect of the invention there is provided a quinazoline derivative of the formula I as hereinbefore defined wherein:
(i) **each of R¹ and R²**, which may be the same or different, is selected from hydrogen and (1-3C)alkyl and **R^{1a} and R^{2a}** are hydrogen, or (ii) **each of R¹ and R^{1a}**, which may be the same or different, is selected from (1-3C)alkyl and **R² and R^{2a}** are hydrogen, or (iii) **R¹ and R^{1a}** are hydrogen and **each of R² and R^{2a}**, which may be the same or different, is selected from (1-3C)alkyl;
**each of R³ and R⁴**, which may be the same or different, is selected from hydrogen, (1-3C)alkyl and (2-4C)alkenyl, and wherein any CH or CH₂ or CH₃ within any of R¹,R^{1a}, R², R^{2a}, R³ and R⁴ optionally bears on each said CH or CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkylamino];
**X** is selected from hydrogen, fluoro, chloro, methyl and methoxy;
**R⁵** is selected from fluoro and chloro;
**Y** is selected from O, S and OCH₂ (particularly S and OCH₂, more particularly OCH₂);
**Q¹** is selected from phenyl, 2-,3- or 4-pyridyl, 2-pyrazinyl, 1,3-thiazol-2-yl, 1,3-thiazol-4-yl, 1,3-thiazol-5-yl, 1H-pyrazol-3-yl, 1H-pyrazol-4-yl, 1H-pyrazol-5-yl, 1H-imidazol-2-yl, 1H-imidazol-4-yl, 1H-imidazol-5-yl, isoxazol-3-yl, isoxazol-4-yl and isoxazol-5-yl,
   and wherein Q¹ optionally bears one or more substituents (for example 1, 2 or 3), which may be the same or different selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is selected from hydrogen, (1-4C)alkoxy and (1-4C)alkoxy(1-4C)alkoxy (particularly R⁶ is hydrogen or methoxy, more particularly R⁶ is hydrogen); and
**n** is 0 or 1 (particularly n is 0); or a pharmaceutically acceptable salt thereof.

In one aspect of this embodiment of the invention when n is 1, R⁵ may be in an ortho position to the NH group. In another aspect of this embodiment of the invention when n is 1, R⁵ is in the ortho position to the Y-Q¹ group.

According to a further aspect of the invention there is provided a quinazoline derivative of the formula I as hereinbefore defined wherein:
**each of R¹ and R²** is selected from hydrogen and methyl, provided that R¹ and R² are not both methyl;
**each of R³ and R⁴,** which may be the same or different, is selected from hydrogen and (1-3C)alkyl;
and wherein any CH₂ or CH₃ within any of R³ and R⁴ which is not attached to a nitrogen atom optionally bears on each said CH₂ or CH₃ one or more (for example 1, 2 or 3) halogeno substituents or a substituent selected from hydroxy, cyano, amino, (1-3C)alkylamino and di-[(1-3C)alkyl]amino;
**R^{1a} and R^{2a}** are hydrogen;
**X** is selected from hydrogen, fluoro, chloro, and methoxy;
**Y** is selected from S and OCH₂;
**Q¹** is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl and isoxazol-3-yl,
and wherein Q¹ optionally bears 1 or 2 substituents, which may be the same or different, selected from selected from halogeno, hydroxy, cyano, nitro, (1-4C)alkyl and (1-4C)alkoxy (particularly Q¹ optionally bears 1 or 2 substituents, which may be the same or different, selected from selected from fluoro and (1-4C)alkyl);
**R⁶** is hydrogen; and
**n** is 0;
or a pharmaceutically acceptable salt thereof.

According to a further aspect of the invention there is provided a quinazoline derivative of the formula I as hereinbefore defined wherein:
(i) **each of R¹ and R²** is selected from hydrogen and methyl, provided that R¹ and R² are not both methyl, and **R^{1a} and R^{2a}** are hydrogen, or (ii) **R¹ and R^{1a}** are methyl and **R² and R^{2a}** are hydrogen, or (iii) **R¹ and R^{1a}** are hydrogen and **R² and R^{2a}** are methyl;
**each of R³ and R⁴**, which may be the same or different, is selected from hydrogen, (1-3C)alkyl and (2-4C)alkenyl; and wherein any CH or CH₂ or CH₃ within any of R³ and R⁴ which is not attached to a nitrogen atom optionally bears on each said CH or CH₂ or CH₃ one or more (for example 1, 2 or 3) halogeno substituents or a substituent selected from hydroxy, cyano, amino, (1-3C)alkylamino and di-[(1-3C)alkyl]amino;
**X** is selected from hydrogen, fluoro, chloro, methyl and methoxy;
**Y** is selected from O, S and OCH₂ (particularly S and OCH₂, more particularly OCH₂);
**Q¹** is selected from phenyl, 2-pyridyl, 2-pyrazinyl, 1,3-thiazol-2-yl, 1,3-thiazol-4-yl, 1,3-thiazol-5-yl, 1H-pyrazol-3-yl, 1H-imidazol-2-yl and isoxazol-3-yl,
and wherein Q¹ optionally bears 1 or 2 substituents, which may be the same or different, selected from selected from halogeno, hydroxy, cyano, nitro, (1-4C)alkyl and (1-4C)alkoxy (particularly Q¹ optionally bears 1 or 2 substituents, which may be the same or different, selected from selected from fluoro and (1-4C)alkyl);
**R⁶** is hydrogen; and
**n** is 0;
or a pharmaceutically acceptable salt thereof.

According to a further aspect of the invention there is provided a quinazoline derivative of the formula I as hereinbefore defined wherein:
**each of R¹, R², R³ and R⁴,** which may be the same or different, is selected from hydrogen and (1-3C)alkyl,
and wherein any CH₂ or CH₃ within any of R¹, R², R³ and R⁴ optionally bears on each said CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkyl]amino;
**R^{1a} and R^{2a}** are hydrogen;
**X** is selected from hydrogen and halogeno;
**Y** is selected from S and OCH₂;
**Q¹** is selected from phenyl, 2-pyridyl, 2-pyrazinyl, 1H-imidazol-2-yl and 3-isoxazolyl,
and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is hydrogen; and
**n** is 0;
or a pharmaceutically acceptable salt thereof.

According to a further aspect of the invention there is provided a quinazoline derivative of the formula I as hereinbefore defined wherein:
(i) **each of R¹ and R²**, which may be the same or different, is selected from hydrogen and (1-3C)alkyl and **R^{1a} and R^{2a}** are hydrogen, or (ii) **each of R¹ and R^{1a}**, which may be the same or different, is selected from (1-3C)alkyl and **R² and R^{2a}** are hydrogen, or (iii) **R¹ and R^{1a}** are hydrogen and each of **R² and R^{2a}**, which may be the same or different, is selected from (1-3C)alkyl;
**each of R³ and R⁴**, which may be the same or different, is selected from hydrogen, (1-3C)alkyl and (2-4C)alkenyl,
and wherein any CH or CH₂ or CH₃ within any of R¹, R^{1a}, R², R^{2a}, R³ and R⁴ optionally bears on each said CH or CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkyl]amino;
**X** is selected from hydrogen, (1-4)alkyl and halogeno;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl,
and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is hydrogen; and
**n** is 0;
or a pharmaceutically acceptable salt thereof.

According to a further aspect of the invention there is provided a quinazoline derivative of the formula I as hereinbefore defined wherein:
(i) **each of R¹ and R²**, which may be the same or different, is selected from hydrogen and (1-3C)alkyl and **R^{1a} and R^{2a}** are hydrogen, or (ii) **each of R¹ and R^{1a},** which may be the same or different, is selected from (1-3C)alkyl and **R²** and **R^{2a}** are hydrogen, or (iii) **R¹ and R^{1a}** are hydrogen and each of **R² and R^{2a}**, which may be the same or different, is selected from (1-3C)alkyl;
**each of R³ and R⁴**, which may be the same or different, is selected from hydrogen, (1-3C)alkyl and (2-4C)alkenyl, and wherein any CH or CH₂ or CH₃ within any of R¹, R^{1a}, R², R^{2a}, R³ and R⁴ optionally bears on each said Ch or CH₂ or CH₃ one or more (for example 1, 2 or 3) halogeno substituents or a substituent selected from hydroxy, cyano, (1-3C)alkoxy, amino, (1-3C)alkylamino and di-[(1-3C)alkyl]amino;
**X** is selected from methyl and chloro;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl,
and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is hydrogen; and
**n** is 0;
or a pharmaceutically acceptable salt thereof.

According to a further aspect of the invention there is provided a quinazoline derivative of the formula I as hereinbefore defined wherein:
**R¹** is (1-3C)alkyl;
**R²** is hydrogen;
**R³** is selected from hydrogen and (1-3C)alkyl;
**R⁴** is selected from hydrogen and (1-3C)alkyl; and wherein any CH₂ or CH₃ within any of R¹, R³ and R⁴ which is not attached to a nitrogen atom optionally bears on each said CH₂ or CH₃ one or more (for example 1, 2 or 3) halogeno substituents or a substituent selected from hydroxy, cyano, amino, (1-3C)alkylamino and di-[(1-3C)alkyl]amino;
**R^{1a} and R^{2a}** are hydrogen;
**X** is selected from hydrogen and chloro;
**Y** is selected from S and OCH₂; and
**Q¹** is selected from phenyl, 2-pyridyl, 2-pyrazinyl, 1H-imidazol-2-yl and 3-isoxazolyl, and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is hydrogen; and
**n** is 0;
or a pharmaceutically acceptable salt thereof.

According to a further aspect of the invention there is provided a quinazoline derivative of the formula I as hereinbefore defined wherein:
(i) **R¹** is (1-3C)alkyl and **R^{1a}**, **R² and R^{2a}** are hydrogen, or (ii) **each of R¹ and R^{1a},** which may be the same or different, is selected from (1-3C)alkyl and **R² and R^{2a}** are hydrogen, or (iii) **R¹ and R^{1a}** are hydrogen and each of **R² and R^{2a}**, which may be the same or different, is selected from (1-3C)alkyl;
**R³** is selected from hydrogen and (1-3C)alkyl;
**R⁴** is selected from hydrogen, (1-3C)alkyl and (2-4C)alkenyl; and wherein any CH or CH₂ or CH₃ within any of R¹, R³ and R⁴ which is not attached to a nitrogen atom optionally bears on each said CH or CH₂ or CH₃ one or more (for example 1, 2 or 3) halogeno substituents or a substituent selected from hydroxy, cyano, amino, (1-3C)alkylamino and di-[(1-3C)alkyl]amino;
**X** is selected from methyl and chloro;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl,
and wherein Q¹ optionally bears one or more substituents (for example 1, 2, or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, amino-(1-4C)alkyl, N-(1-4C)alkylamino-(1-4C)alkyl and N, N-di-[(1-4C)alkyl]amino-(1-4C)alkyl;
**R⁶** is hydrogen; and
**n** is 0;
or a pharmaceutically acceptable salt thereof.
Another embodiment of the present invention is a quinazoline derivative of the formula I wherein:
   **R¹** is methyl
   **R², R^{1a} and R^{2a}** are hydrogen;
   **R³** is selected from hydrogen and methyl;
   **R⁴** is selected from hydrogen, methyl, ethyl and 2-hydroxyethyl;
   **X** is selected from hydrogen and chloro;
   **Y** is selected from S and OCH₂;
   **Q¹** is selected from phenyl, 2-pyridyl, 2-pyrazinyl, 1H-imidazol-2-yl and 3-isoxazolyl, and wherein Q¹ optionally bears one or more substituents (for example 1, 2, or 3), which may be the same or different, selected from halogeno (such as fluoro or chloro) and (1-4C)alkyl (such as methyl);
   **R⁶** is hydrogen; and
      **n** is 0; or a pharmaceutically acceptable salt thereof.

Another embodiment of the present invention is a quinazoline derivative of the formula I wherein:
(i) **R¹** is methyl and **R², R^{1a} and R^{2a}** are hydrogen, or (ii) **R¹ and R^{1a}** are methyl and **R² and R^{2a}** are hydrogen, or (iii) **R¹ and R^{1a}** are hydrogen and **R² and R^{2a}** are methyl;
**R³** is selected from hydrogen, ethyl and methyl;
**R⁴** is selected from hydrogen, methyl, ethyl, propenyl, 2-methoxyethyl and 2-hydroxyethyl;
**X** is selected from methyl and chloro;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl,
   and wherein Q¹ optionally bears one or more substituents (for example 1, 2, or 3), which may be the same or different, selected from halogeno (such as fluoro or chloro) and (1-4C)alkyl (such as methyl);
**R⁶** is hydrogen; and
**n** is 0;
   or a pharmaceutically acceptable salt thereof.

Another embodiment of the present invention is a quinazoline derivative of the formula I wherein:
**each of R¹ and R²**, which may be the same or different, is selected from hydrogen and methyl;
**R³** is selected from hydrogen and methyl;
**R⁴** is selected from hydrogen, methyl, ethyl and 2-hydroxyethyl;
**R^{1a} and R^{2a}** are hydrogen;
**X** is selected from hydrogen and chloro;
**Y** is selected from S and OCH₂;
**Q¹** is selected from phenyl, 2-pyridyl, 2-pyrazinyl, 1H-imidazol-2-yl and 3-isoxazolyl,
and wherein Q¹ optionally bears one or more substituents (for example 1, 2, or 3), which may be the same or different, selected from halogeno (such as fluoro or chloro) and (1-4C)alkyl (such as methyl);
**R⁶** is hydrogen; and
**n** is 0; or a pharmaceutically acceptable salt thereof.

Particular values for Q¹ in this embodiment include, for example, 3-fluorophenyl, 2-pyridyl, 2-pyrazinyl, 1-methyl-1H-imidazol-2-yl and 5-methyl-3-isoxazolyl.

Another embodiment of the present invention is a quinazoline derivative of the formula I wherein:
(i) **each of R¹ and R²,** which may be the same or different, is selected from hydrogen and methyl and **R^{1a} and R^{2a}** are hydrogen, or (ii) **R¹ and R^{1a}** are methyl and **R² and R^{2a}** are hydrogen, or (iii) **R¹ and R^{1a}** are hydrogen and **R² and R^{2a}** are methyl;
**R³** is selected from hydrogen, ethyl and methyl;
**R⁴** is selected from hydrogen, methyl, ethyl, propenyl, 2-methoxyethyl and 2-hydroxyethyl;
**X** is selected from methyl and chloro;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl,
   and wherein Q¹ optionally bears one or more substituents (for example 1, 2, or 3), which may be the same or different, selected from halogeno (such as fluoro or chloro) and (1-4C)alkyl (such as methyl);
**R⁶** is hydrogen; and
**n** is 0; or a pharmaceutically acceptable salt thereof.

Particular values for Q¹ in this embodiment include, for example, 3-fluorophenyl, 2-pyridyl, 2-pyrazinyl, 1-methyl-1H-imidazol-2-yl, 1,3-thiazol-4-yl, 1,3-thiazol-5-yl and 5-methyl-3-isoxazolyl.

Another embodiment of the present invention is a quinazoline derivative of the formula I wherein:
**R¹ and R²** are both hydrogen, or **R¹** is hydrogen and **R²** is methyl, or **R¹** is methyl and **R²** is hydrogen;
**R³** is methyl;
**R⁴** is selected from hydrogen, methyl, ethyl and 2-hydroxyethyl;
**R^{1a} and R^{2a}** are hydrogen;
**X** is selected from hydrogen and chloro;
**Y** is selected from S and OCH₂;
**Q¹** is selected from phenyl, 2-pyridyl, 2-pyrazinyl, 1H-imidazol-2-yl and 3-isoxazolyl, and wherein Q¹ optionally bears 1 or 2 substituents, which may be the same or different, selected from halogeno and (1-4C)alkyl;
**R⁶** is hydrogen; and
**n** is 0; or a pharmaceutically acceptable salt thereof.

Another embodiment of the present invention is a quinazoline derivative of the formula I wherein:
(i) **R¹, R^{1a}, R² and R^{2a}** are hydrogen, or (ii) **R¹, R^{1a} and R^{2a}** are hydrogen and **R²** is methyl, or (iii) **R¹** is methyl and **R^{1a}, R² and R^{2a}** are hydrogen, or (iv) **R¹ and R^{1a}** are methyl and **R² and R^{2a}** are hydrogen, or (v) **R¹ and R^{1a}** are hydrogen and **R² and R^{2a}** are methyl;
**R³** is methyl;
**R⁴** is selected from hydrogen, methyl, ethyl, propenyl, 2-methoxyethyl and 2-hydroxyethyl;
**X** is selected from methyl and chloro;
**Y** is selected from O, S and OCH₂ (particularly S and OCH₂, more particularly OCH₂);
**Q¹** is selected from phenyl, 2-pyridyl, 2-pyrazinyl, 1H-imidazol-2-yl, 1,3-thiazol-4-yl, 1,3-thiazol-5-yl and 3-isoxazolyl, and wherein Q¹ optionally bears 1 or 2 substituents, which may be the same or different, selected from halogeno and (1-4C)alkyl;
**R⁶** is hydrogen; and
**n** is 0; or a pharmaceutically acceptable salt thereof.

In several of those embodiments of the present invention hereinbefore described, R³ and/or R⁴ may be selected from hydrogen. However, in a particular aspect of those embodiments of the invention, neither R³ nor R⁴ is hydrogen.

Another embodiment of the present invention is a quinazoline derivative of the formula I wherein:
**R¹** is selected from hydrogen and methyl;
**R², R^{1a} and R^{2a}** are hydrogen;
**R³** is methyl;
**R⁴** is selected from methyl, ethyl and 2-hydroxyethyl;
**X** is selected from hydrogen and chloro;
**Y** is OCH₂;
**Q¹** is selected from phenyl, 2-pyridyl and 2-pyrazinyl, and wherein Q¹ optionally bears 1 or 2 substituents, which may be the same or different, selected from halogeno (such as fluoro or chloro) and (1-4C)alkyl (such as methyl);
**R⁶** is hydrogen; and
   **n** is 0; or a pharmaceutically acceptable salt thereof.

Another embodiment of the present invention is a quinazoline derivative of the formula I wherein:
(i) **R¹** is selected from hydrogen and methyl and **R², R^{1a} and R^{2a}** are hydrogen, or (ii) **R¹ and R^{1a}** are hydrogen and **R² and R^{2a}** are methyl, or (iii) **R¹ and R^{1a}** are methyl and **R² and R^{2a}** are hydrogen;
**R³** is methyl;
**R⁴** is selected from methyl, ethyl, propenyl, 2-methoxyethyl and 2-hydroxyethyl;
**X** is selected from methyl and chloro;
**Y** is OCH₂;
**Q¹** is selected from phenyl, 2-pyridyl and 2-pyrazinyl, and wherein Q¹ optionally bears 1 or 2 substituents, which may be the same or different, selected from halogeno (such as fluoro or chloro) and (1-4C)alkyl (such as methyl);
**R⁶** is hydrogen; and
   **n** is 0; or a pharmaceutically acceptable salt thereof.

Another embodiment of the present invention is a quinazoline derivative of the formula I wherein:
**R¹ and R²** are both hydrogen, or **R¹** is hydrogen and **R²** is methyl, or **R¹** is methyl and **R²** is hydrogen;
**R³** is methyl;
**R⁴** is selected from methyl, ethyl and 2-hydroxyethyl;
**R^{1a}** and **R^{2a}** are hydrogen;
**X** is selected from hydrogen and chloro;
**Y** is OCH₂;
**Q¹** is phenyl which optionally bears 1 or 2 substituents, which may be the same or different, selected from fluoro or chloro (for example Q¹ is 3-fluorophenyl);
**R⁶** is hydrogen; and
   **n** is 0; or a pharmaceutically acceptable salt thereof.

Another embodiment of the present invention is a quinazoline derivative of the formula I wherein:
(i) **R¹, R^{1a}, R² and R^{2a}** are hydrogen, or (ii) **R¹, R^{1a} and R^{2a}** are hydrogen and **R²** is methyl, or (iii) **R¹** is methyl and **R^{1a}, R²** and **R^{2a}** are hydrogen, or (iv) **R¹ and R^{1a}** are hydrogen and **R² and R^{2a}** are methyl, or (v) **R¹ and R^{1a}** are methyl and **R² and R^{2a}** are hydrogen;
**R³** is methyl;
**R⁴** is selected from methyl, ethyl, propenyl, 2-methoxyethyl and 2-hydroxyethyl;
**X** is selected from methyl and chloro;
**Y** is OCH₂;
**Q¹** is phenyl which optionally bears 1 or 2 substituents, which may be the same or different, selected from fluoro or chloro (for example Q¹ is 3-fluorophenyl);
**R⁶** is hydrogen; and
   **n** is 0; or a pharmaceutically acceptable salt thereof.

Another embodiment of the present invention is a quinazoline derivative of the formula I wherein:
(i) **R¹ and R²** are both hydrogen, **R³ and R⁴** are methyl, and **X** is hydrogen, or
(ii) **R¹ and R²** are both hydrogen, **R³** is methyl, **R⁴** is 2-hydroxyethyl and **X** is chloro, or
(iii) **R¹** is methyl, **R²** is hydrogen, **R³ and R⁴** are methyl, and **X** is chloro, or
(iv) **R¹** is hydrogen, **R²** is methyl, **R³ and R⁴** are methyl, and **X** is chloro, or
(v) **R¹** is methyl, **R²** is hydrogen, **R³ and R⁴** are methyl, and **X** is methoxy, or
(vi) **R¹ and R²** are hydrogen, **R³ and R⁴** are methyl, and **X** is methoxy;
   **R^{1a} and R^{2a}** are hydrogen;
   **Y** is selected from O and OCH₂;
   **Q¹** is phenyl which optionally bears 1 or 2 substituents, which may be the same or different, selected from fluoro or chloro;
   **R⁶** is hydrogen; and
   **n** is 0; or a pharmaceutically acceptable salt thereof.

In particular, in embodiments (i), (ii), (iii) and (iv) above, Q¹ may be 3-fluorophenyl and/or Y may be OCH₂. In embodiments (v) and (vi) above, Q¹ may be phenyl and/or Y may be O.

Another embodiment of the present invention is a quinazoline derivative of the formula I wherein:
(i) **R¹, R^{1a}, R²** and **R^{2a}** are hydrogen, or (ii) **R¹** is methyl and **R^{1a}, R² and R^{2a}** are hydrogen, or (iii) **R²** is methyl and **R¹, R^{1a} and R^{2a}** are hydrogen, or (iv) **R¹** and **R^{1a}** are methyl and **R²** and **R^{2a}** are hydrogen;
**R³** is selected from methyl and ethyl (in particular methyl);
**R⁴** is selected from methyl, ethyl, propenyl, 2-methoxyethyl and 2-hydroxyethyl (in particular methyl and 2-methoxyethyl);
**X** is selected from chloro and methyl;
**Y** is OCH₂;
**Q¹** is selected from 2-pyridyl and 2-pyrazinyl,
   and wherein Q¹ optionally bears 1 or 2 substituents, which may be the same or different, selected from halogeno and (1-4C)alkyl (particularly Q¹ is selected from 2-pyridyl and 2-pyrazinyl);
**R⁶** is hydrogen; and
**n** is 0;
   or a pharmaceutically acceptable salt thereof.

In this embodiment, X is particularly chloro and/or R³ and R⁴ are both methyl.

In this embodiment, particular substituents which are optionally present on Q¹ are selected from fluoro, chloro and (1-4C)alkyl, more particularly fluoro, and methyl.

In this embodiment when Q¹ is 2-pyrazinyl, suitably (ii) **R¹** is methyl and **R^{1a}, R² and R^{2a}** are hydrogen.

In this embodiment when Q¹ is 2-pyridyl, suitably (ii) **R¹** is methyl and **R^{1a}, R² and R^{2a}** are hydrogen or (iii) **R¹ and R^{1a}** are methyl and **R² and R^{2a}** are hydrogen.

In this embodiment when Q¹ is 2-pyridyl, suitably X is chloro.

Another embodiment of the present invention is a quinazoline derivative of the formula I wherein:
**R¹, R^{1a}, R² and R^{2a}** are hydrogen,
**R³** and **R⁴** are methyl;
**X** is selected from chloro and methyl;
**Y** is selected from O and OCH₂;
**Q¹** is selected from 2-pyridyl and 2-pyrazinyl, and wherein Q¹ optionally bears 1 or 2 substituents, which may be the same or different, selected from halogeno and (1-4C)alkyl;
**R⁶** is hydrogen; and
**n** is 0;
or a pharmaceutically acceptable salt thereof.

In this embodiment, in particular X is chloro and/or Y is O and/or Q¹ is 2-pyridyl.

Another embodiment of the present invention is a quinazoline derivative of the formula I wherein:
(i) **R¹, R^{1a}, R² and R^{2a}** are hydrogen, or (ii) **R¹, R^{1a} and R^{2a}** are hydrogen and **R²** is methyl, or (iii) **R¹** is methyl and **R^{1a}, R² and R^{2a}** are hydrogen;
**R³** is methyl;
**R⁴** is selected from methyl, ethyl, propenyl, 2-methoxyethyl and 2-hydroxyethyl (particularly R⁴ is methyl);
**X** is chloro;
**Y** is S;
**Q¹** is 1H-imidazol-2-yl and wherein Q¹ optionally bears 1 or 2 substituents, which may be the same or different, selected from (1-4C)alkyl (particularly Q¹ is 1-methyl-1H-imidazol-2-yl);
**R⁶** is hydrogen; and
**n** is 0;
or a pharmaceutically acceptable salt thereof.

Another embodiment of the present invention is a quinazoline derivative of the formula I wherein:
(i) **R¹, R^{1a}, R² and R^{2a}** are hydrogen, or (ii) **R¹** is methyl and **R^{1a}, R² and R^{2a}** are hydrogen, or (iii) **R¹ and R^{1a}** are hydrogen and **R² and R^{2a}** are methyl;
**R³** is methyl;
**R⁴** is selected from methyl, ethyl, propenyl, 2-methoxyethyl and 2-hydroxyethy (particularly methyl);
**X** is selected from chloro and methyl;
**Y** is OCH₂;
**Q¹** is 1,3-thiazolyl (particularly 1,3-thiazol-4-yl or 1,3-thiazol-5-yl);
**R⁶** is hydrogen; and
**n** is 0;
   or a pharmaceutically acceptable salt thereof.

Another embodiment of the present invention is a quinazoline derivative of the formula I wherein:
(i) **R¹, R^{1a}, R² and R^{2a}** are hydrogen, or (ii) **R¹** is methyl and **R^{1a}, R² and R^{2a}** are hydrogen, or (iii) **R¹ and R^{1a}** are hydrogen and **R² and R^{2a}** are methyl;
**R³** is methyl;
**R⁴** is selected from methyl, ethyl, propenyl, 2-methoxyethyl and 2-hydroxyethyl (particularly methyl);
**X** is selected from chloro, methoxy and methyl;
**Y** is OCH₂;
**Q¹** is 3-isoxazolyl and wherein Q¹ optionally bears 1 or 2 substituents, which may be the same or different, selected from (1-4C)alkyl (particularly Q¹ is 5-methyl-isoxazol-3-yl);
**R⁶** is hydrogen; and
**n** is 0;
   or a pharmaceutically acceptable salt thereof.

Further particular compounds of the invention are, for example, one or more quinazoline derivatives of the formula I selected from:
4-(3-Chloro-4-(2-pyridylmethoxy)anilino)-5-(2dimethylaminoethoxy)quinazoline;
4-(3-Chloro-4-(2-pyridylmethoxy)anilino)-5-(2-dimethylamino-1-methylethoxy)quinazoline;
4-(3-Chloro-4-(1-methyl-1*H*-imidazol-2-ylthio)anilino)-5-(2-dimethylaminoethoxy)quinazoline;
4-(3-Chloro-4-(1-methyl-1*H*-imidazol-2-ylthio)anilino)-5-(2-dimethylamino-2-methylethoxy)quinazoline;
4-(4-(3-Fluorobenzyloxy)anilino)-5-(2-dimethylaminoethoxy)quinazoline;
4-(4-(3-Fluorobenzyloxy)anilino)-5-(2.dimethylamino-1-methylethoxy)quinazoline;
4-(3-Chloro-4-(2-pyrazinylmethoxy)anilino)-5-(2-dimethylaminoethoxy)quinazoline;
4-(3-Chloro-4-(2-pyrazinylmethoxy)anilino)-5-(2-dimethylamino-1-methylethoxy)quinazoline;
4-(3-Chloro-4-(5-methylisoxazol-3-ylmethoxy)anilino)-5-(2-dimethylaminoethoxy)quinazoline;
4-(3-Chloro-4-(5-methylisoxazol-3-ylmethoxy)anilino)-5-(2-dimethylamino-1-methylethoxy)quinazoline;
4-(3-Chloro-4-(3-fluorobenzyloxy)anilino)-5-(2-(N-ethyl-N-methylamino)ethoxy)quinazoline;
4-(3-Chloro-4-(3-fluorobenzyloxy)anilino)-5-(2-dimethylaminoethoxy)quinazoline;
4-(3-Chloro-4-(3-fluorobenzyloxy)anilino)-5-[2-(N-(2-hydroxyethyl)-N-methylamino)ethoxy]quinazoline;
4-(3-Chloro-4-(2-pyridylmethoxy)anilino)- 5-(2-(N-ethyl-N-methylamino)ethoxy)quinazoline;
4-(3-Chloro-4-(2-pyridylmethoxy)anilino)- 5-(2-(N-(2-hydroxyethyl)-N-methylamino)ethoxy)quinazoline;
4-(3-Chloro-4-(3-fluorobenzyloxy)anilino)-5-(2-dimethylamino-2-methylethoxy)quinazoline;
4-(3-Chloro-4-(2-pyridylmethoxy)anilino)-5-(2-dimethylamino-2-methylethoxy)quinazoline; *N*-[3-Chloro-4-(1,3-thiazol-4-ylmethoxy)phenyl]-5-[2-(dimethylamino)ethoxy]quinazolin-4-amine;
*N*-[3-Chloro-4-(pyridin-2-yloxy)phenyl]-5-[2-(dimethylamino)ethoxy]quinazolin-4-amine;
*N*-[3-Chloro-4-(pyrazin-2-ylmethoxy)phenyl]-5-[(1*S*)-2-(dimethylamino)-1-methylethoxy]quinazolin-4-amine;
*N*-{3-Chloro-4-[(3-fluorobenzyl)oxy]phenyl}-5-[(1*S*)-2-(dimethylamino)-1-methylethoxy]quinazolin-4-amine;
*N*-[3-Chloro-4-(pyridin-2-ylmethoxy)phenyl]-5-[(1*R*)-2-(dimethylamino)-1-methylethoxy]quinazolin-4-amine;
*N*-[3-Chloro-4-(1,3-thiazol-4-ylmethoxy)phenyl]-5-[(1*R*)-2-(dimethylamino)-1-methylethoxy]quinazolin-4-amine;
*N*-[3-Chloro-4-(pyrazin-2-ylmethoxy)phenyl]-5-[(1*R*)-2-(dimethylamino)-1-methylethoxy]quinazolin-4-amine;
*N*-{3-Chloro-4-[(3-fluorobenzyl)oxy]phenyl}-5-[(1*R*)-2-(dimethylamino)-1-methylethoxy]quinazolin-4-amine;
*N*-[3-Chloro-4-(pyridin-2-ylmethoxy)phenyl]-5-[2-(dimethylamino)-2-methylpropoxy]quinazolin-4-amine;
*N*-[3-Chloro-4-(1,3-thiazol-4-ylmethoxy)phenyl]-5-[2-(dimethylamino)-2-methylpropoxy]quinazolin-4-amine;
*N*-{3-Chloro-4-[(5-methylisoxazol-3-yl)methoxy]phenyl}-5-[2-(dimethylamino)-2-methylpropoxy]quinazolin-4-amine;
5-[2-(Dimethylamino)ethoxy]-*N*-[3-methyl-4-(pyridin-2-ylmethoxy)phenyl]quinazolin-4-amine;
5-[2-(Dimethylamino)ethoxy]-*N*-[3-methyl-4-(1,3-thiazol-4-ylmethoxy)phenyl]quinazolin-4-amine;
5-[2-(Dimethylamino)ethoxy]-*N*-{3-methyl-4-[(5-methylisoxazol-3-yl)methoxy]phenyl}quinazolin-4-amine;
5-[(1*R*)-2-(Dimethylamino)-1-methylethoxy]-*N*-[3-methyl-4-(pyridin-2-ylmethoxy)phenyl]quinazolin-4-amine;
5-[(1*R*)-2-(Dimethylamino)-l-methylethoxy]-*N*-[3-methyl4-(pyrazin-2-ylmethoxy)phenyl]quinazolin-4-amine;
5-[(1*R*)-2-(dimethylamino)-1-methylethoxy]-*N*-[3-methyl-4-(1,3-thiazol-4-ylmethoxy)phenyl]quinazolin-4-amine;
5-[(1*R*)-2-(Dimethylamino)-1-methylethoxy]-*N*-{3-methyl-4-[(5-methylisoxazol-3-yl)methoxy]phenyl}quinazolin-4-amine;
5-[2-(dimethylamino)-2-methylpropoxy]-*N*-[3-methyl-4-(1,3-thiazol-4-ylmethoxy)phenyl]quinazolin-4-amine;
5-[2-(Dimethylamino)ethoxy]-*N*-{3-methoxy-4-[(5-methylisoxazol-3-yl)methoxy]phenyl}quinazolin-4-amine;
5-[2-(Dimethylamino)ethoxy]-*N*-[3-methoxy-4-(pyrazin-2-ylmethoxy)phenyl]quinazolin-4-amine;
5-[2-(Dimethylamino)ethoxy]-*N*-[3-fluoro-4-(1,3-thiazol-5-ylmethoxy)phenyl]quinazolin-4-amine;
*N*-[3-Chloro-4-(pyridin-2-ylmethoxy)phenyl]-5-[(1*S*)-2-(dimethylamino)-1-methylethoxy]quinazolin-4-amine;
*N*-[3-Chloro-4-(pyridin-2-ylmethoxy)phenyl]-5-{[(2*S*)-2-(dimethylamino)propyl]oxy}quinazolin-4-amine;
*N*-[3-Chloro-4-(pyridin-2-ylmethoxy)phenyl]-5-{[(2*R*)-2-(dimethylamino)propyl]oxy}quinazolin-4-amine;
5-{2-[Allyl(methyl)amino]ethoxy}-*N*-[3-chloro-4-(pyridin-2-ylmethoxy)phenyl]quinazolin-4-amine;
2-[{2-[(4-{[3-Chloro-4-(pyridin-2-ylmethoxy)phenyl]amino}quinazolin-5-yl)oxy]ethyl}(ethyl)amino]ethanol;
*N*-[3-Chloro-4-(pyridin-2-ylmethoxy)phenyl]-5-{(1*S*)-2-[(2-methoxyethyl)(methyl)amino]-1-methylethoxy}quinazolin-4-amine;
*N*-[3-Chloro-4-(pyridin-2-ylmethoxy)phenyl]-5-{(1*R*)-2-[ethyl(methyl)amino]-1-methylethoxy}quinazolin-4-amine;
5-{(1*R*)-2-[Allyl(methyl)amino]-1-methylethoxy}-*N*-[3-chloro-4-(pyridin-2-ylmethoxy)phenyl]quinazolin-4-amine;
5-{(1*S*)-2-[Allyl(methyl)amino]-1-methylethoxy}-*N*-[3-chloro-4-(pyridin-2-ylmethoxy)phenyl]quinazolin-4-amine;
*N*-{3-Chloro-4-[(3-fluorobenzyl)oxy]phenyl}-5-{[(2*S*)-2-(dimethylamino)propyl]oxy}quinazolin-4-amine;
*N*-{3-Chloro-4-[(3-fluorobenzyl)oxy]phenyl}-5-{[(2*R*)-2-(dimethylamino)propyl]oxy}quinazolin-4-amine;
*N*-{3-Chloro-4-[(1-methyl-1*H*-imidazol-2-yl)thio]phenyl}-5-{[(2*S*)-2-(dimethylamino)propyl]oxy}quinazolin-4-amine;
*N*-{3-Chloro-4-[(1-methyl-1*H*-imidazol-2-yl)thio]phenyl}-5-{[(2*R*)-2-(dimethylamino)propyl]oxy}quinazolin-4-amine;
*N-*{3-Chloro-4-[(1-methyl-1*H*-imidazol-2-yl)thio]phenyl}-5-[(1*R*)-2-(dimethylamino)-1-methylethoxy]quinazolin-4-amine;
5-[2-(Dimethylamino)-1-methylethoxy]-*N*-(3-methoxy-4-phenoxyphenyl)quinazolin-4-amine;
5-[2-(Dimethylamino)-1-methylethoxy]-N-(3-methoxy-4-phenoxyphenyl)quinazolin-4-amine; and
*N*-[3-Chloro-4-(pyridin-2-ylmethoxy)phenyl]-5-[2-(dimethylamino)-1,1-dimethylethoxy]quinazolin-4-amine; or a pharmaceutically acceptable acid addition salt thereof.

A quinazoline derivative of the formula I, or a pharmaceutically-acceptable salt thereof, may be prepared by any process known to be applicable to the preparation of chemically-related compounds, for example the processes described in WO96/15118 and WO97/30034. Such processes, when used to prepare a quinazoline derivative of the formula I are provided as a further feature of the invention and are illustrated by the following representative process variants in which, unless otherwise stated, R¹, R^{1a}, R², R^{2a}, R³, R⁴, R⁵, R⁶, X, Y, n and Q¹ have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

Process (a) The reaction, conveniently in the presence of a suitable base, of a quinazoline of the formula **II:** 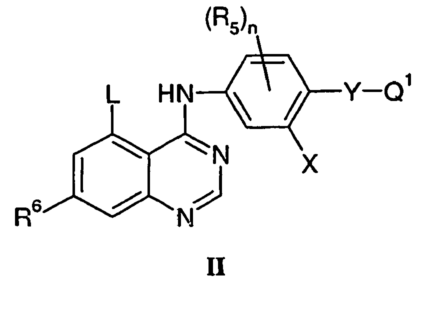 wherein R⁵, R⁶, Q¹, X, Y and n have any of the meanings defined hereinbefore except that any functional group is protected if necessary, and L is a suitable displaceable group, with an alcohol of the formula **III** 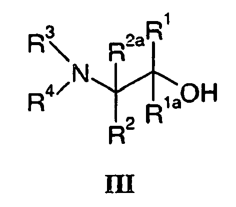 wherein R¹, R^{1a}, R², R^{2a}, R³ and R⁴ have any of the meanings defined hereinbefore except that any functional group is protected if necessary; or

Process (b) for the preparation of those compounds of the formula **I** wherein Y is OC(R⁷)₂, SC(R⁷)₂ or N(R⁷)C(R⁷)₂, the reaction, conveniently in the presence of a suitable base, of a quinazoline of the formula **IV:** 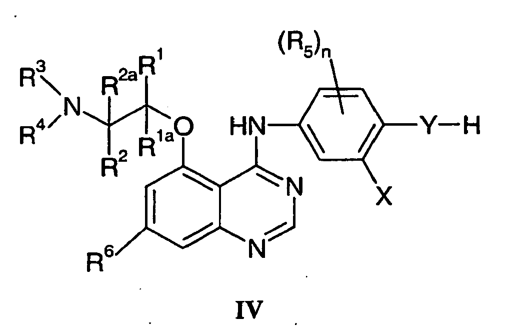 wherein Y is O, S or N(R⁷), and X, R¹, R^{1a}, R², R^{2a}, R³, R⁴, R⁵, R⁶, R⁷ and n have any of the meanings defined hereinbefore except that any functional group is protected if necessary, with a compound of the formula **V:**

Q¹-C(R⁷)₂-L¹ **V**

wherein L¹ is a suitable displaceable group and Q¹ and R⁷ have any of the meanings defined hereinbefore except that any functional group is protected if necessary; or

Process (c) the reaction of a quinazoline of the formula **VI:** 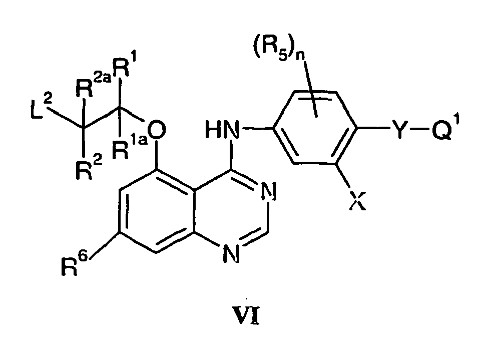 wherein L² is a suitable displaceable group and Q¹, X, Y, R¹, R^{1a}, R², R^{2a}, R⁵, R⁶ and n have any of the meanings defined hereinbefore except that any functional group is protected if necessary, with an amine of the formula **VII:**

NHR³R⁴ **VII**

wherein R³ and R⁴ have any of the meanings defined hereinbefore except that any functional group is protected if necessary; or

Process (d) for the preparation of those compounds of the formula **I** wherein R^{2a} is hydrogen the reductive amination in the presence of a suitable reducing agent of the aldehyde or ketone of the formula **VIII:** 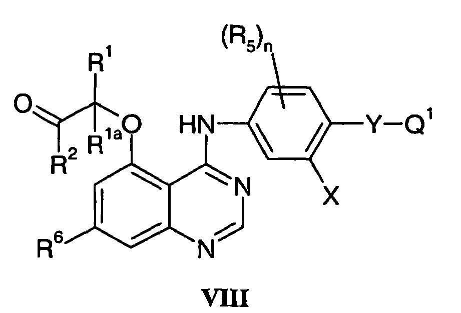 wherein Q¹, X, Y, R¹, R^{1a}, R², R⁵, R⁶ and n have any of the meanings defined hereinbefore except that any functional group is protected if necessary, with an amine of the formula **VII** as hereinbefore defined; or

Process (e) for the preparation of those compounds of the formula **I** wherein Y is O or N(R⁷) and Q¹ is, for example, 2-pyridyl or 4-pyridyl the reaction, in the presence of a suitable catalyst, of a quinazoline of the formula **IV:** 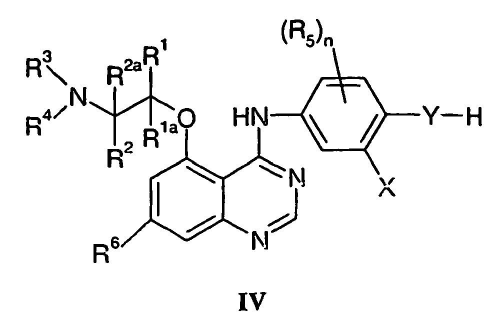 wherein Y is O or N(R⁷) and X, R¹, R^{1a}, R², R^{2a}, R³, R⁴, R⁵, R⁶ and n have any of the meanings defined hereinbefore except that any functional group is protected if necessary, with a suitable heterocycle, for example an amine of the formula IVa or of the formula **IVb:** 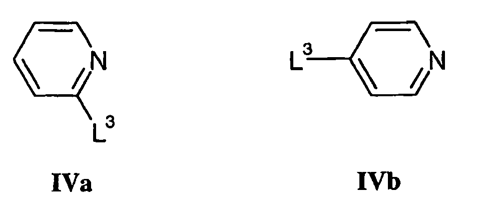 wherein L³ is a suitable displaceable group; or

Process (f) the reaction, conveniently in the presence of a suitable phosphine and a suitable diazo compound, of a quinazoline of the formula **II:** 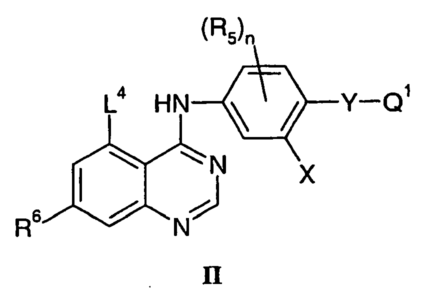 wherein R⁵, R⁶, Q¹, X, Y and n have any of the meanings defined hereinbefore except that any functional group is protected if necessary, and L⁴ is hydroxy, with an alcohol of the formula **III:** 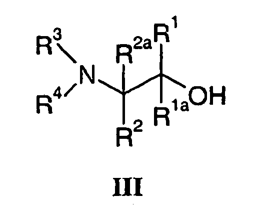 wherein R¹, R^{1a}, R², R^{2a}, R³ and R⁴ have any of the meanings defined hereinbefore except that any functional group is protected if necessary; and thereafter, if necessary:
(i) converting a quinazoline derivative of the formula I into another quinazoline derivative of the formula I;
(ii) removing any protecting group that is present by conventional means;
(iii) forming a pharmaceutically acceptable salt. Specific conditions for the above reactions are as follows:

### Process (a)

A suitable displaceable group L in the quinazoline of formula **II** is for example halogeno or a sulfonyloxy group, for example fluoro, chloro, methylsulfonyloxy or toluene-4-sulfonyloxy group. A particular group L is fluoro or chloro, more particularly fluoro.

A suitable base for the reaction a quinazoline of the formula **II** and the alcohol of the formula **III** includes, for example a strong non-nucleophilic base such as an alkali metal hydride, for example sodium hydride, or an alkali metal amide, for example lithium di-isopropylamide (LDA).

The reaction a quinazoline of the formula **II** and the alcohol of the formula **III** is conveniently carried out in the presence of a suitable inert solvent or diluent, for example a halogenated solvent such as methylene chloride, chloroform or carbon tetrachloride, an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, 10 to 250°C, preferably in the range 40 to 150°C. Conveniently, this reaction may also be performed by heating the reactants in a sealed vessel using a suitable heating apparatus such as a microwave heater.

Conveniently, the reaction a quinazoline of the formula **II** and the alcohol of the formula **III** is performed in the presence of a suitable catalyst, for example a crown ether.

Alcohols of the formula **III** are commercially available compounds or they are known in the literature, or they can be can be prepared by standard processes known in the art.

The quinazoline of the formula **II** may be obtained by conventional procedures. For example, a quinazoline of the formula **IX:** 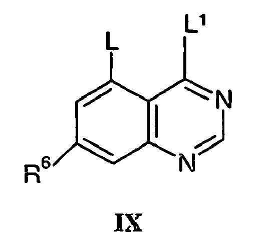 wherein L and L¹ are displaceable groups, and L¹ is more labile than L, may be reacted with a compound of the formula **X:** 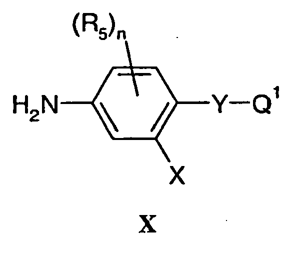 wherein Q¹, R⁵, R⁶, Y, X and n have any of the meanings defined hereinbefore except that any functional group is protected if necessary, whereafter any protecting group that is present is removed by conventional means.

A suitable displaceable group L is as hereinbefore defined, particularly fluoro. A suitable displaceable group L¹ is, for example, a halogeno (particularly chloro), alkoxy, aryloxy, mercapto, alkylthio, arylthio, alkylsulfinyl, arylsulfinyl, alkylsulfonyl, arylsulfonyl or sulfonyloxy group, for example a chloro, bromo, methoxy, phenoxy, pentafluorophenoxy, methylthio, methanesulfonyl, methanesulfonyloxy or toluene-4-sulfonyloxy group.

The reaction is conveniently carried out in the presence of an acid. Suitable acids include, for example hydrogen chloride gas (conveniently dissolved in diethyl ether or dioxane) or hydrochloric acid.

Alternatively, the reaction of the quinazoline of formula **IX** with the compound of formula **X** may be carried out in the presence of a suitable base. A suitable base is, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, for example sodium carbonate, potassium carbonate, cesium carbonate, calcium carbonate, or, for example, an alkali metal hydride, for example sodium hydride.

Alternatively the quinazoline derivative of the formula **IX,** wherein L¹ is halogeno (for example chloro), may be reacted with the compound of the formula **X** in the absence of an acid or a base. In this reaction displacement of the halogeno leaving group L¹ results in the formation of the acid HL¹ in-situ and the autocatalysis of the reaction.

The above reactions are conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The above reactions are conveniently carried out at a temperature in the range, for example, 0 to 250°C, conveniently in the range 40 to 80°C or, preferably, at or near the reflux temperature of the solvent when used.

Conveniently, the above reactions may be performed in the presence of a suitable catalyst, for example a crown ether as described above in relation to the reaction of the quinazoline of the formula **II** and the alcohol of formula **III.**

The quinazoline of the formula **II** wherein Y is OC(R⁷)₂, SC(R⁷)₂ or N(R⁷)C(R⁷)₂ may also be obtained according to Reaction Scheme 1: 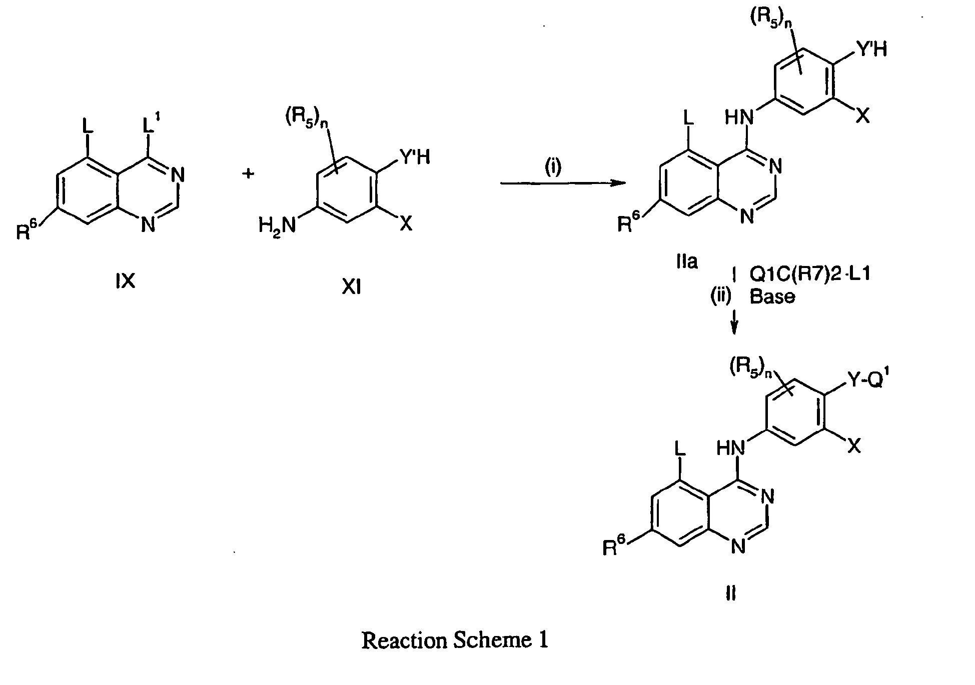 wherein each L¹, which may be the same or different, is a suitable displaceable group, for example chloro, Y' is S, O or N(R⁷) and L is a displaceable group as hereinbefore defined, for example fluoro.
(i) reaction carried out under analogous conditions to those described above in relation to the reaction of the quinazoline of the formula **IX** with the compound of the formula **X** described above.
(ii) reaction carried out under analogous conditions to those used in Process (b) described hereinafter.

The quinazoline of formula **IX** may be obtained using conventional methods, for example when R⁶ is hydrogen, L is fluoro and L¹ is halogeno, 5-fluoro-3,4-dihydroquinazolin-4-one may be reacted with a suitable halogenating agent such as thionyl chloride, phosphoryl chloride or a mixture of carbon tetrachloride and triphenylphosphine. The 5-fluoro-3,4-dihydroquinazoline starting material is commercially available or can be prepared using conventional methods, for example as described in J. Org. Chem. 1952, 17, 164-176.

The compounds of the formulae **XI** and Q¹C(R⁷)₂L¹ (a compound of the formula **V**) are commercially available compounds or they are known in the literature, or they can be can be prepared by standard processes known in the art.

Compounds of the formula **X** are commercially available compounds or they are known in the literature, or they can be can be prepared by standard processes known in the art. For example, the compound of the formula **X** wherein Y is O, S, N(R⁷), OC(R⁷)₂, SC(R⁷)₂ or N(R⁷)C(R⁷)₂ may be prepared in accordance with Reaction Scheme 2: 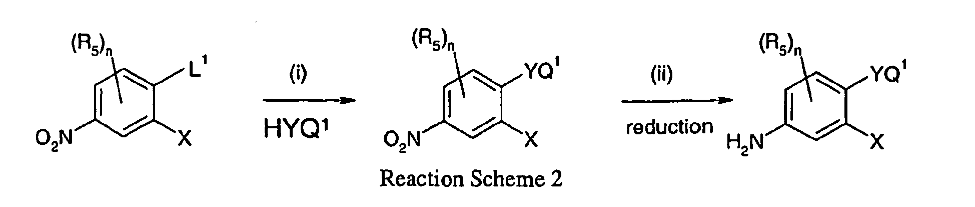 wherein L¹ is a suitable displaceable group as hereinbefore defined (for example halogeno such as chloro) and Q¹, X, R⁵ and n are as hereinbefore defined, except any functional group is protected if necessary, and whereafter any protecting group that is present is removed by conventional means.
(i) The compounds of the formula HYQ¹ are commercially available, or they are known in the literature, or can be prepared using well known processes in the art. For example compounds of the formula Q¹CH₂OH may be prepared using known methods, for example by reduction of the corresponding ester of the formula Q¹COOR, wherein R is, for example (1-6C)alkyl, or benzyl, with a suitable reducing agent, for example sodium borohydride, followed by ester hydrolysis.
(ii) The reduction of the nitro group in step (ii) may be carried out under standard conditions, for example by catalytic hydrogenation over a platinum/carbon, palladium/carbon or nickel catalyst, treatment with a metal such as iron, titanium chloride, tin II chloride or indium, or treatment with another suitable reducing agent such as sodium dithionite.

For example, the compound of the formula **X** wherein Y is OC(R⁷)₂, SC(R⁷)₂ or N(R⁷)C(R⁷)₂ may be prepared in accordance with Reaction Scheme 3: 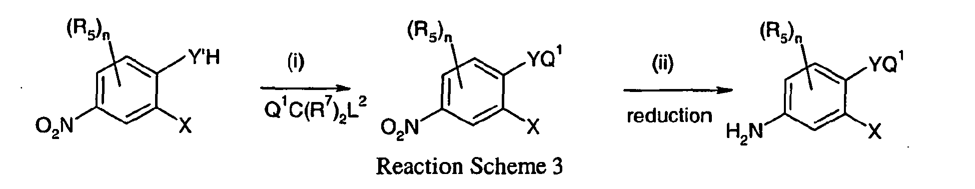 wherein L² is a suitable leaving group, particularly halogeno, for example chloro or bromo, and Y' is O, S or N(R⁷).
(i) analogous conditions to those used in Process (b)
(ii) analogous conditions to those used in Reaction Scheme 2.

Compounds of the formula **X** wherein Y is OC(R⁷)₂ may also be prepared be prepared by coupling the starting nitro phenol in Reaction Scheme 3 with a compound of the formula Q¹C(R⁷)₂OH, conveniently in the presence of a suitable dehydrating agent. A suitable dehydrating agent is, for example, a carbodiimide reagent such as dicyclohexylcarbodiimide or 1-(3-dimethylaminopropyl)-3-ethylcarbodiimide or a mixture of an azo compound such as diethyl or di-tert-butyl azodicarboxylate and a phosphine such as triphenylphosphine. The reaction is conveniently carried out in the presence of a suitable inert solvent or diluent, for example a halogenated solvent such as methylene chloride, chloroform or carbon tetrachloride and at a temperature in the range, for example, 0 to 150°C, preferably at or near ambient temperature.

### Process (b)

A suitable displaceable group, L¹ in the compound of formula **V** is as hereinbefore defined in relation to the displaceable groups in the compound of formula **IX** described above under process (a). Particular displaceable groups include, for example for example halogeno such as chloro, or alkanesulfonyloxy, such as mesyloxy.

The reaction of the quinazoline of the formula **IV** with the compound of formula V is conveniently carried out in an inert solvent such as or a dipolar aprotic solvent for example N,N-dimethylformamide, N,N-dimethylacetamide, N-methylpyrrolidin-2-one or dimethylsulfoxide, or acetonitrile. The reaction is conveniently carried out in the presence of a suitable base. Suitable bases include, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, an alkali or alkaline earth metal carbonate, for example sodium carbonate, potassium carbonate, cesium carbonate or calcium carbonate, or an alkali metal hydride, for example sodium hydride. A particular base is for example an alkali metal carbonate, such as potassium carbonate.

Generally the reaction is suitably performed at a temperature of from -10 to 120°C, conveniently at or near ambient temperature. Conveniently, the reaction a quinazoline of the formula **IV** and the compound of the formula **V** is performed in the presence of a suitable catalyst, for example a crown ether.

The quinazoline of the formula **IV** can be prepared by standard processes known in the art, for example as shown in Reaction Scheme 4:

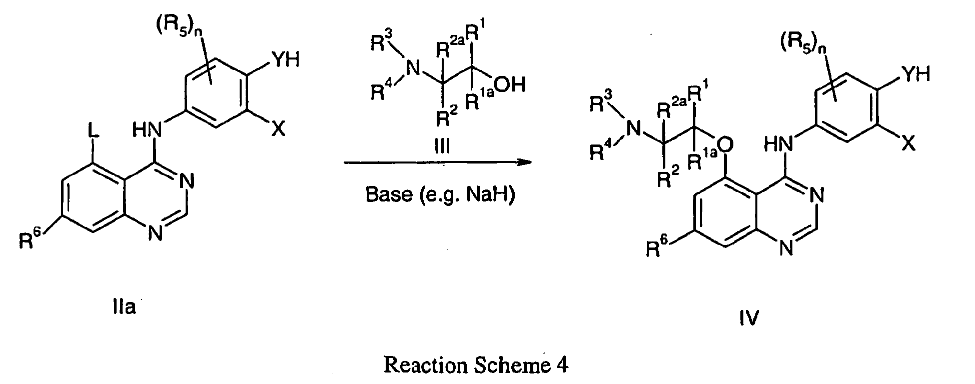

The reaction of the compound of the formula **IIa** with the alcohol of formula **III** may be performed using analogous conditions to those described in Process (a). The compound of formula **IIa** may be prepared using the process described in Reaction Scheme 1.

When R³ and R⁴ are each methyl, the quinazoline of the formula **IV** can, for example, also be prepared by standard processes as shown in Reaction Scheme 4a: 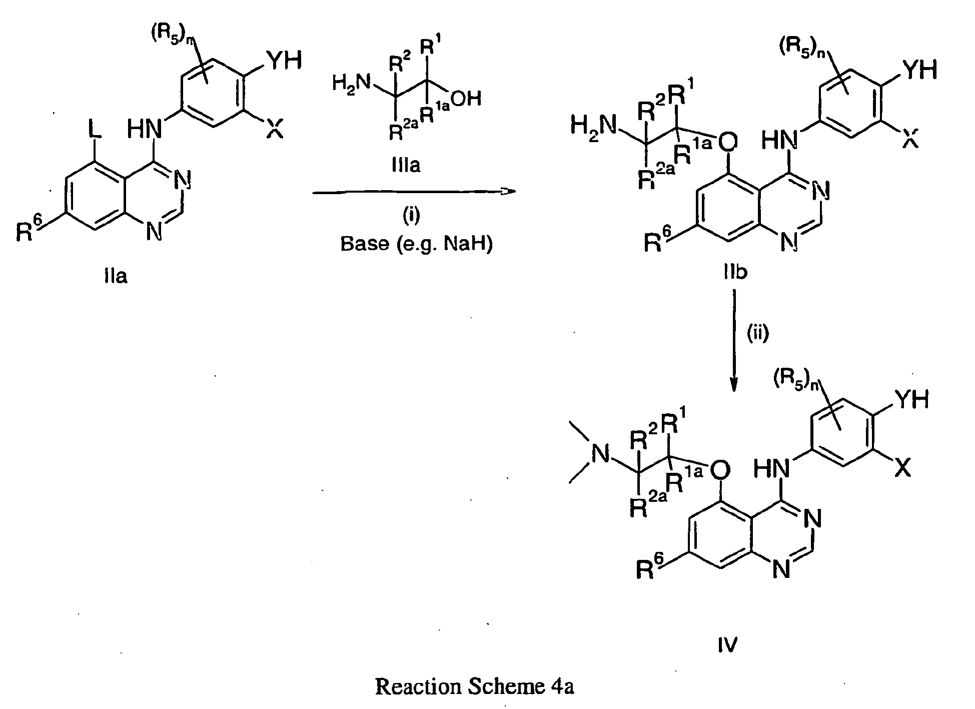
(i) Performed using analogous conditions to those described in Process (a). The compound of formula **IIa** may be prepared using the process described in Reaction Scheme 1. Alcohols of the formula **IIIa** are commercially available compounds or they are known in the literature or they can be prepared by standard processes known in the art.
(ii) Reaction of the compound of the formula **IIb** with formic acid and formaldehyde.
   This reaction is conveniently conducted at a temperature of from 50 to 120°C, particularly of from 80 to 110°C. The reaction may be conducted in the presence of a suitable inert solvent or diluent, for example water. The reaction may alternatively be conducted without a solvent or diluent.

### Process (c)

Suitable displaceable groups represented by L² in the quinazoline of formula **VI** include halogeno, or a sulfonyloxy group, for example chloro or bromo, methane sulfonyloxy or toluenesulfonyloxy. The reaction is suitably performed in the presence of a suitable inert solvent or diluent, for example a solvent(s) or diluent(s) described above in relation to process
(a). Suitably the reaction is carried out at a temperature of, for example 0 to 180°C, particularly 20°C to the reflux temperature of the solvent/diluent. Conveniently the reaction may also be carried out by heating the reactants in a sealed vessel using a suitable heating apparatus such as a microwave heater.

The reaction is conveniently carried out in the presence of a base. Suitable bases include, for example, those described above in relation to process (b) such as cesium carbonate.

Conveniently the reaction may be carried out in the presence of a suitable catalyst, for example an iodine catalyst such as a quaternary ammonium iodide, for example tetra-n-butylammonium iodide.

The quinazoline of the formula **VI** can be prepared by standard processes known in the art, for example when L² is chloro in the quinazoline of formula **VI** Reaction Scheme 5 may be used: 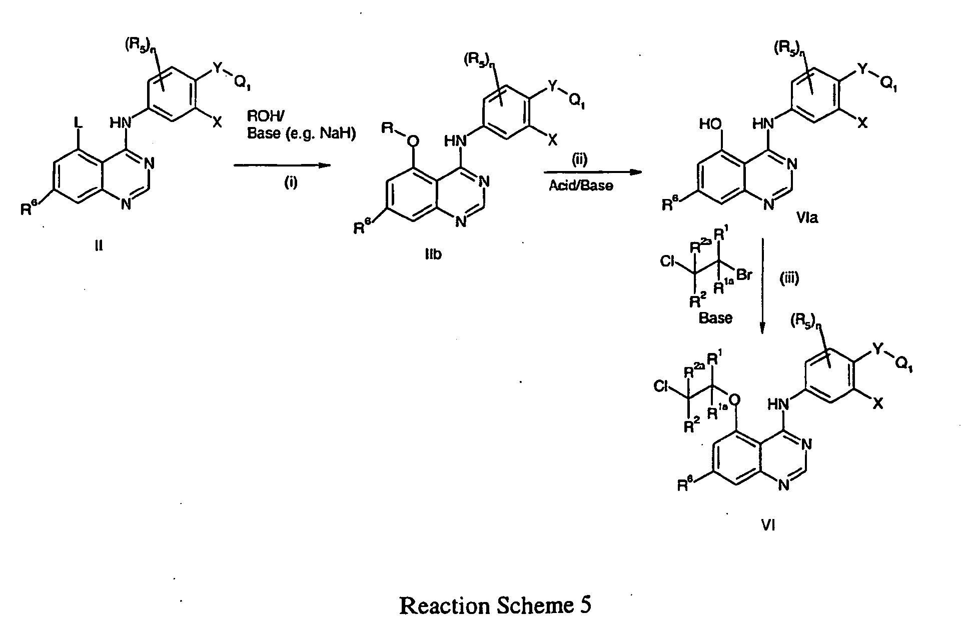
(i) ROH is a suitable alcohol, wherein R is a displaceable group in the compound of formula **IIb.** Suitable groups represented by R include, for example acid or base displaceable groups. For example suitable alcohols of the formula ROH include 4-methoxyphenylmethanol and 2,4-dimethoxyphenylmethanol. The reaction in step (i) may be carried out under analogous conditions to those described for Process (a).
(ii) Cleavage of the R group at the 5-position may be effected by treatment with a suitable acid, for example an inorganic acid or an organic acid, such as trifluoroacetic acid. Alternatively a strong base may be used, for example sodium hydride. The cleavage reaction is suitably carried out at a temperature in the range, for example, from 10 to 150°C, for example from 25 to 80°C.
   The reactions in steps (i) and (ii) are suitably carried out in the presence of a suitable inert solvent or diluent as defined hereinbefore in relation to Process (a).
(iii) Under analogous conditions to those described above for Process (b).

The compound of formula II may be prepared as described in Process (a) above, for example as in Reaction Scheme 1.

### Process (d)

A suitable reducing agent is, for example, a hydride reducing agent, for example an alkali metal aluminium hydride such as lithium aluminium hydride, formic acid or, preferably, an alkali metal borohydride such as sodium borohydride, sodium cyanoborohydride, sodium triethylborohydride, sodium trimethoxyborohydride and sodium trisacetoxyborohydride, or a quaternary ammonium borohydride such as macroporous triethylammonium methylpolystyrene trisacetoxyborohydride. The reaction is conveniently performed in a suitable inert solvent or diluent, for example tetrahydrofuran or diethyl ether for the more powerful reducing agents such as lithium aluminium hydride, and, for example, methylene chloride or a protic solvent such as methanol and ethanol for the less powerful reducing agents such as sodium triacetoxyborohydride and sodium cyanoborohydride. The reaction is conveniently performed at a temperature in the range, for example, 10 to 100°C, conveniently at or near ambient temperature.

An analogous reductive amination to that described above in process (d) may be used to introduce an alkyl or substituted alkyl group onto a primary or secondary amine group in a quinazoline derivative of the formula I by reductive amination with a corresponding ketone in the presence of a suitable reducing agent. For example, for the production of those compounds of the formula I wherein R³ or R⁴ is methyl, the corresponding compound containing an NH or NH₂ group may be reacted with formaldehyde in the presence of a suitable reducing agent as described above.

The quinazoline of formula **VIII** may be prepared using standard processes known in the art, for example according to Reaction Scheme 6: 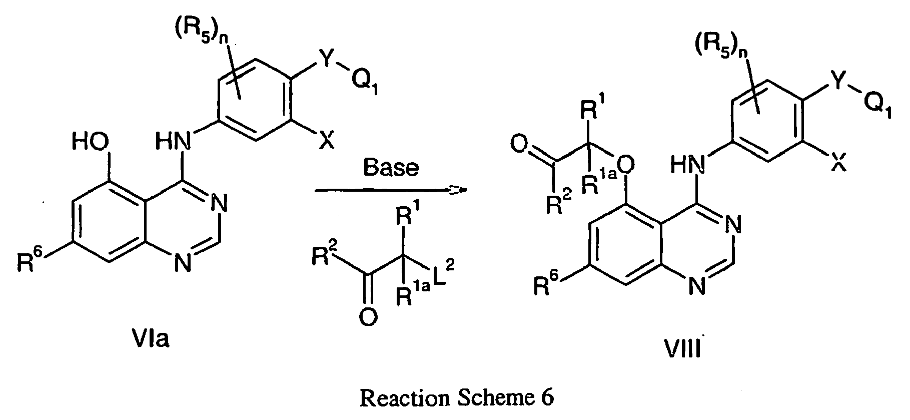
(i) Reaction carried out under analogous conditions to those used in Process (b).
(ii) The compound of formula **VIa** may be prepared according to Reaction Scheme 5.

### Process(e)

A suitable displaceable group, L³ in the compound of formula **IVa** is halogeno, particularly bromo.

A suitable catalyst is a palladium catalyst, for example a catalyst formed in situ by the reaction of bis(dibenzylideneacetone)palladium and (9,9-dimethyl-9H-xanthene-4,5-diyl)bis(diphenylphosphine). The reaction is conveniently performed in the presence of a suitable base, for example cesium carbonate.

The reaction is conveniently performed in a suitable inert solvent or diluent, for example an ether such as tetrahydrofuran or 1,4-dioxane.

Suitably the reaction is carried out at a temperature of, for example 0 to 180°C, particularly 20°C to the reflux temperature of the solvent/diluent. Conveniently the reaction may also be carried out by heating the reactants in a sealed vessel using a suitable heating apparatus such as a microwave heater.

The quinazoline of the formula **IV** can be prepared by standard processes known in the art, for example as shown in Reaction Scheme 4 or Reaction Scheme 4a. Amines of the formula **IVa** are commercially available compounds or they are known in the literature or they can be prepared by standard processes known in the art.

### Process (f)

A suitable phosphine is triphenylphosphine and a suitable diazo compound is di-tert-butyl hydrazodicarboxylate. However, a person skilled in the art using his common general knowledge would readily be able to select different phosphines and/or diazo compounds with which to conduct the reaction.

The reaction is conveniently performed in a suitable inert solvent or diluent, for example dichloromethane. The reaction is conveniently performed at a temperature in the range, for example, 10 to 100°C, conveniently at or near ambient temperature.

The quinazoline of formula **II** may be obtained by conventional procedures, as dicussed above. Alcohols of the formula **III** are commercially available compounds or they are known in the literature or they can be prepared by standard processes known in the art.

The quinazoline derivative of the formula I may be obtained from the above processes in the form of the free base or alternatively it may be obtained in the form of a salt, for example with the acid of the formula H-L¹ or H-L² when L¹ and L² are, for example halogeno such as chloro. When it is desired to obtain the free base from the salt, the salt may be treated with a suitable base, for example, an alkali or alkaline earth metal carbonate or hydroxide, for example sodium carbonate, potassium carbonate, calcium carbonate, sodium hydroxide or potassium hydroxide.

The protecting groups used in the processes above may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the oxidation of alkylthio to alkylsulfinyl or alkylsulfonyl; the substitution of an NH group in Q¹ by the reaction with an optionally substituted alkyl halide.

When a pharmaceutically-acceptable salt of a quinazoline derivative of the formula I is required, for example an acid-addition salt, it may be obtained by, for example, reaction of said quinazoline derivative with a suitable acid using a conventional procedure.

As mentioned hereinbefore some of the compounds according to the present invention may contain one or more chiral centers and may therefore exist as stereoisomers (for example when R¹ and/or R² is (1-3C)alkyl). Stereoisomers may be separated using conventional techniques, e.g. chromatography or fractional crystallisation. The enantiomers may be isolated by separation of a racemate for example by fractional crystallisation, resolution or HPLC. The diastereomers may be isolated by separation by virtue of the different physical properties of the diastereoisomers, for example, by fractional crystallisation, HPLC or flash chromatography. Alternatively particular stereoisomers may be made by chiral synthesis from chiral starting materials under conditions which will not cause racemisation or epimerisation, or by derivatisation, with a chiral reagent. When a specific stereoisomer is isolated it is suitably isolated substantially free for other stereoisomers, for example containing less than 20%, particularly less than 10% and more particularly less than 5% by weight of other stereoisomers.

In the section above relating to the preparation of the compound of formula I, the expression "inert solvent" refers to a solvent which does not react with the starting materials, reagents, intermediates or products in a manner which adversely affects the yield of the desired product.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

### Biological Assays

The inhibitory activities of compounds were assessed in non-cell based protein tyrosine kinase assays as well as in cell based proliferation assays before their *in vivo* activity was assessed in Xenograft studies. **a) Protein Tyrosine Kinase phosphorylation Assays**

This test measures the ability of a test compound to inhibit the phosphorylation of a tyrosine containing polypeptide substrate by an enzyme selected from the EGFR kinase and erbB2 kinase.

Recombinant intracellular fragments of EGFR and erbB2 (accession numbers X00588 and X03363 respectively) were cloned and expressed in the baculovirus/Sf21 system. Lysates were prepared from these cells by treatment with ice-cold lysis buffer (20mM N-2-hydroxyethylpiperizine-N'-2-ethanesulfonic acid (HEPES) pH 7.5, 150mM NaCl, 10% glycerol, 1% Triton X-100, 1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 200µl phosphate buffered saline (PBS) solution and incubated at 4°C overnight). Plates were washed in 50mM HEPES pH 7.4 at room temperature to remove any excess unbound synthetic peptide. EGFR or erbB2 activities were assessed by incubation in peptide coated plates for 20 minutes at room temperature in 100mM HEPES pH 7.4 at room temperature, adenosine trisphosphate (ATP) at Km concentration for the respective enzyme, 10mM MnCl₂, 0.1mM Na₃VO₄, 0.2mM DL-dithiothreitol (DTT), 0.1% Triton X-100 with test compound in DMSO (final concentration of 2.5%). Reactions were terminated by the removal of the liquid components of the assay followed by washing of the plates with PBS-T (phosphate buffered saline with 0.5% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulfonate (6)] diammonium salt crystals (ABTS™ from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values. **b) H16N-2 cell proliferation assay**

This assay measures the ability of a test compound to inhibit heregulin β or EGF driven proliferation of H16N-2 cells. These non-neoplastic eptihelial cells respond in a proliferative manner to stimulation with either EGF or heregulin β (Ram, G.R.and Ethier, S.P.(1996) Cell Growth and Differentiation, 7, 551-561) were isolated human mammary tissue (Band, V. and Sager, R. Tumour progression in breast cancer. In: J. S. Rhim and A. Dritschilo (eds.), Neoplastic Transformation in human Cell Culture, pp 169-178. Clifton, NJ: Humana Press, 1991) and were obtained from the Dana-Farber Cancer Institute, 44 Binney Street, Boston, Massachusetts 02115.

H16N-2 cells were routinely cultured in culture medium (a 1:1 mix of Gibco F12 and Ham's αMEM media containing 1 % foetal calf serum, 10mM HEPES, 1µg/ml Insulin, 12.5ng/ml EGF, 2.8µM Hydrocortisone, 2nM Estradiol 5µM Ascorbic Acid, 10µg/ml Transferrin, 0.1mM Phosphoethanolamine, 15nM Sodium Selenite, 2mM Glutamine, 10nM Tri-iodo-thrynoine, 35µg/ml Bovine pituitary Extract and 0.1mM Ethanolamine) at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.0x10³ cells per well of a 96 well plate in the above media at 37°C in 7.5% CO₂ and allowed to settle for 72 hours.

Following this, the cells were starved of serum for 24 hours upon the addition of starvation medium (a 1:1 mix of Gibco F12 and Ham's αMEM media containing, 10mM HEPES, 2nM Estradiol, 5µM Ascorbic Acid, 10µg/ml Transferrin, 0.1mM Phosphoethanolamine, 15nM Sodium Selenite, 2mM Glutamine, and 0.1mM Ethanolamine) and incubated at 37°C in 7.5% CO₂. The cells were then treated with or without compound at a range of concentrations in dimethylsulfoxide (DMSO) (1 % final) for two hours before the addition of exogenous ligand (at a final concentration of 100ng/ml of heregulin β or 5ng/ml of EGF) and incubation with both ligand and compound in a total volume of 200µl for 4 days at 37°C in 7.5% CO₂. Following the incubation period, cell numbers were determined by addition of 50µl of 3-(4,5-Dimethylthiazol-2-yl)-2,5-diphenyltetrazolium bromide (MTT) (stock 5mg/ml) and incubated at 37°C in a 7.5% CO₂ air incubator for 2 hours. MTT solution was then removed from the cells by aspiration, which were then allowed to air dry and were dissolved upon the addition of 100µl of DMSO.

Absorbance of this solubilised cells was read at 540nm to quantify cell biomass. Inhibition of proliferation was expressed as an IC₅₀ value. This was determined by calculation of the concentration of compound that was required to give 50% inhibition of proliferation. The range of proliferation was calculated from the positive (vehicle plus ligand) and negative (vehicle minus ligand) control values. **c) *In vivo* BT-474 Xenograft assay**

This assay measures the ability of a test compound to inhibit the growth of a BT-474 tumour cell xenograft (human mammary carcinoma obtained from Dr Baselga, Laboratorio Recerca Oncologica, Paseo Vall D'Hebron 119-129, Barcelona 08035, Spain) in Female Swiss athymic mice (Alderley Park, *nu*/*nu* genotype) (Baselga, J. et al. (1998) Cancer Research, 58, 2825-2831).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum*. All procedures were performed on mice of at least 8 weeks of age. BT-474 tumour cell xenografts were established in the hind flank of donor mice by sub-cutaneous injections of 1x10⁷ freshly cultured cells in 100µl of serum free media with 50% Matrigel per animal. On day 14 post-implant, mice were randomised into groups of 10 prior to the treatment with compound or vehicle control that was administered once daily at 0.1ml/kg body weight. Tumour volume was assessed twice weekly by bilateral Vernier calliper measurement, using the formula (length x with) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students *t* test. **d) hERG-encoded Potassium Channel Inhibition Assay**

This assay determines the ability of a test compound to inhibit the tail current flowing through the human ether-a-go-go-related-gene (hERG)-encoded potassium channel.

Human embryonic kidney (HEK) cells expressing the hERG-encoded channel were grown in Minimum Essential Medium Eagle (EMEM; Sigma-Aldrich catalogue number M2279), supplemented with 10% Foetal Calf Serum (Labtech International; product number 4-101-500), 10% M1 serum-free supplement (Egg Technologies; product number 70916) and 0.4 mg/ml Geneticin G418 (Sigma-Aldrich; catalogue number G7034). One or two days before each experiment, the cells were detached from the tissue culture flasks with Accutase (TCS Biologicals) using standard tissue culture methods. They were then put onto glass coverslips resting in wells of a 12 well plate and covered with 2 ml of the growing media.

For each cell recorded, a glass coverslip containing the cells was placed at the bottom of a Perspex chamber containing bath solution (see below) at room temperature (-20 °C). This chamber was fixed to the stage of an inverted, phase-contrast microscope. Immediately after placing the coverslip in the chamber, bath solution was perfused into the chamber from a gravity-fed reservoir for 2 minutes at a rate of - 2 ml/min. After this time, perfusion was stopped.

A patch pipette made from borosilicate glass tubing (GC120F, Harvard Apparatus) using a P-97 micropipette puller (Sutter Instrument Co.) was filled with pipette solution (see hereinafter). The pipette was connected to the headstage of the patch clamp amplifier (Axopatch 200B, Axon Instruments) via a silver/silver chloride wire. The headstage ground was connected to the earth electrode. This consisted of a silver/silver chloride wire embedded in 3% agar made up with 0.85% sodium chloride.

The cell was recorded in the whole cell configuration of the patch clamp technique. Following "break-in", which was done at a holding potential of -80 mV (set by the amplifier), and appropriate adjustment of series resistance and capacitance controls, electrophysiology software (*Clampex*, Axon Instruments) was used to set a holding potential (-80 mV) and to deliver a voltage protocol. This protocol was applied every 15 seconds and consisted of a 1 s step to +40 mV followed by a 1 s step to -50 mV. The current response to each imposed voltage protocol was low pass filtered by the amplifier at 1 kHz. The filtered signal was then acquired, on line, by digitising this analogue signal from the amplifier with an analogue to digital converter. The digitised signal was then captured on a computer running *Clampex* software (Axon Instruments). During the holding potential and the step to + 40 mV the current was sampled at 1 kHz. The sampling rate was then set to 5 kHz for the remainder of the voltage protocol.

The compositions, pH and osmolarity of the bath and pipette solution are tabulated below.

| **Salt** | **Pipette (mM)** | **Bath (mM)** |
|---|---|---|
| NaCl | - | 137 |
| KCl | 130 | 4 |
| MgCl₂ | 1 | 1 |
| CaCl₂ | - | 1.8 |
| HEPES | 10 | 10 |
| glucose | - | 10 |
| Na₂ATP | 5 | - |
| EGTA | 5 | - |

| **Parameter** | **Pipette** | **Bath** |
|---|---|---|
| pH | 7.18 - 7.22 | 7.40 |
| pH adjustment with | 1M KOH | 1M NaOH |
| Osmolarity (mOsm) | 275-285 | 285-295 |

The amplitude of the hERG-encoded potassium channel tail current following the step from +40 mV to -50 mV was recorded on-line by *Clampex* software (Axon Instruments). Following stabilisation of the tail current amplitude, bath solution containing the vehicle for the test substance was applied to the cell. Providing the vehicle application had no significant effect on tail current amplitude, a cumulative concentration effect curve to the compound was then constructed.

The effect of each concentration of test compound was quantified by expressing the tail current amplitude in the presence of a given concentration of test compound as a percentage of that in the presence of vehicle.

Test compound potency (IC₅₀) was determined by fitting the percentage inhibition values making up the concentration-effect to a four parameter Hill equation using a standard data-fitting package. If the level of inhibition seen at the highest test concentration did not exceed 50%, no potency value was produced and a percentage inhibition value at that concentration was quoted. **e) Clone 24 phospho-erbB2 cell assay**

This immunofluorescence end point assay measures the ability of a test compound to inhibit the phosphorylation of erbB2 in a MCF7 (breast carcinoma) derived cell line which was generated by transfecting MCF7 cells with the full length erbB2 gene using standard methods to give a cell line that overexpresses full length wild type erbB2 protein (hereinafter 'Clone 24' cells).

Clone 24 cells were cultured in Growth Medium (phenol red free Dulbecco's modified Eagle's medium (DMEM) containing 10% foetal bovine serum, 2 mM glutamine and 1.2mg/ml G418) in a 7.5% CO₂ air incubator at 37°C. Cells were harvested from T75 stock flasks by washing once in PBS (phosphate buffered saline, pH7.4, Gibco No. 10010-015) and harvested using 2mls of Trypsin (1.25mg/ml) / ethylaminediaminetetraacetic acid (EDTA) (0.8mg/ml) solution. The cells were resuspended in Growth Medium. Cell density was measured using a haemocytometer and viability was calculated using Trypan Blue solution before being further diluted in Growth Medium and seeded at a density of 1x10⁴ cells per well (in 100ul) into clear bottomed 96 well plates (Packard, No. 6005182).

3 days later, Growth Medium was removed from the wells and replaced with 100ul Assay Medium (phenol red free DMEM, 2mM glutamine, 1.2mg/ml G418) either with or without erbB inhibitor compound. Plates were returned to the incubator for 4hours and then 20µl of 20% formaldehyde solution in PBS was added to each well and the plate was left at room temperature for 30 minutes. This fixative solution was removed with a multichannel pipette, 100µl of PBS was added to each well and then removed with a multichannel pipette and then 50µl PBS was added to each well. Plates were then sealed and stored for up to 2 weeks at 4°C.

Immunostaining was performed at room temperature. Wells were washed once with 200µl PBS / Tween 20 (made by adding 1 sachet of PBS / Tween dry powder (Sigma, No. P3563) to 1L of double distilled H₂O) using a plate washer then 200µl Blocking Solution (5% Marvel dried skimmed milk (Nestle) in PBS /Tween 20) was added and incubated for 10 minutes. Blocking Solution was removed using a plate washer and 200µl of 0.5% Triton X-100 / PBS was added to permeabalise the cells. After 10 minutes, the plate was washed with 200µl PBS / Tween 20 and then 200µl Blocking Solution was added once again and incubated for 15 minutes. Following removal of the Blocking Solution with a plate washer, 30µl of rabbit polyclonal anti-phospho ErbB2 IgG antibody (epitope phospho-Tyr 1248, SantaCruz, No. SC-12352-R), diluted 1:250 in Blocking Solution, was added to each well and incubated for 2 hours. Then this primary antibody solution was removed from the wells using a plate washer followed by two 200µl PBS / Tween 20 washes using a plate washer. Then 30µl of Alexa-Fluor 488 goat anti-rabbit IgG secondary antibody (Molecular Probes, No. A-11008), diluted 1:750 in Blocking Solution, was added to each well. From now onwards, wherever possible, plates were protected from light exposure, at this stage by sealing with black backing tape. The plates were incubated for 45 minutes and then the secondary antibody solution was removed from the wells followed by two 200ul PBS / Tween 20 washes using a plate washer. Then 100µl PBS was added to each plate, incubated for 10 minutes and then removed using a plate washer. Then a further 100µl PBS was added to each plate and then, without prolonged incubation, removed using a plate washer. Then 50µl of PBS was added to each well and plates were resealed with black backing tape and stored for up to 2 days at 4°C before analysis.

The Fluorescence signal is each well was measured using an Acumen Explorer Instrument (Acumen Bioscience Ltd.), a plate reader that can be used to rapidly quantitate features of images generated by laser-scanning. The instrument was set to measure the number of fluorescent objects above a pre-set threshold value and this provided a measure of the phosphorylation status of erbB2 protein. Fluorescence dose response data obtained with each compound was exported into a suitable software package (such as Origin) to perform curve fitting analysis. Inhibition of erbB2 phosphorylation was expressed as an IC₅₀ value. This was determined by calculation of the concentration of compound that was required to give 50% inhibition of erbB2 phosphorylation signal.

Although the pharmacological properties of the compounds of the formula I vary with structural change as expected, in general activity possessed by compounds of the formula I, may be demonstrated at the following concentrations or doses in one or more of the above tests (a), (b), (c) and (e):
Test (a):- IC₅₀ in the range, for example, 0.001 - 10 µM;
Test (b):- IC₅₀ in the range, for example, 0.001 - 20 µM;
Test (c):- activity in the range, for example, 1-200 mg/kg/day;
Test (e):- IC₅₀ in the range, for example, 0.001 - 3 µM;
No physiologically unacceptable toxicity was observed in Test (d) at the effective dose for compounds tested of the present invention. Accordingly no untoward toxicological effects are expected when a compound of formula I, or a pharmaceutically-acceptable salt thereof, as defined hereinbefore is administered at the dosage ranges defined hereinafter.

By way of example, the following Table illustrates the activity of representative compounds according to the invention. Column 2 of the Table shows IC₅₀ data for the inhibition of phosphorylation of a tyrosine containing polypeptide substrate by erbB2 kinase in Test (a) described above, column 3 of the Table shows IC₅₀ data for the inhibition of phosphorylation of a tyrosine containing polypeptide substrate by EGFR kinase in Test (a) described above and column 4 of the Table shows IC₅₀ data for the inhibition of phosphorylation of erbB2 in a MCF7 (breast carcinoma) derived cell line in Test (e) described above:

| Example Number | IC₅₀ (µM) Test (a): erbB2 | IC₅₀ (µM) Test (a): EGFR | IC₅₀ (µM) Test (e): erbB2 |
|---|---|---|---|
| 23 | 0.002 | 0.068 | 0.001 |
| 24 | 0.002 | 0.064 | 0.001 |
| 37 | 0.017 | 8.4 | 0.003 |
| 53 | 0.005 | 0.064 | 0.001 |
| 54 | 0.002 | 4.3 | 0.003 |
| 56 | 0.002 | 0.49 | 0.002 |

According to a further aspect of the invention there is provided a pharmaceutical composition which comprises a quinazoline derivative of the formula I, or a pharmaceutically-acceptable thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a quinazoline derivative of the formula I will naturally vary according to the nature and severity of the conditions, the age and sex of the animal or patient and the route of administration, according to well known principles of medicine.

In using a quinazoline derivative of the formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

We have found that the compounds of the present invention possess anti-proliferative properties such as anti-cancer properties that are believed to arise from their erbB2 receptor tyrosine kinase inhibitory activity. Furthermore, certain of the compounds according to the present invention possess substantially better potency against the erbB2 receptor tyrosine kinase, than against other tyrosine kinase enzymes, particularly EGFR tyrosine kinase. Such compounds possess sufficient potency against the erbB2 receptor tyrosine kinase that they may be used in an amount sufficient to inhibit erbB2 receptor tyrosine kinase whilst demonstrating little, or significantly lower, activity against other tyrosine kinases such as EGFR. Such compounds are likely to be useful for the selective inhibition of erbB2 receptor tyrosine kinase and are likely to be useful for the effective treatment of, for example erbB2 driven tumours. Accordingly, the compounds of the present invention are expected to be useful in the treatment of diseases or medical conditions mediated alone or in part by erbB2 receptor tyrosine kinases, i.e. the compounds may be used to produce a erbB2 receptor tyrosine kinase inhibitory effect in a warm-blooded animal in need of such treatment. Thus the compounds of the present invention provide a method for the treatment of malignant cells characterised by inhibition of the erbB2 receptor tyrosine kinase. Particularly the compounds of the invention may be used to produce an anti-proliferative and/or pro-apoptotic and/or anti-invasive effect mediated alone or in part by the inhibition of erbB2 receptor tyrosine kinases. Particularly, the compounds of the present invention are expected to be useful in the prevention or treatment of those tumours that are sensitive to inhibition of the erbB2 receptor tyrosine kinase that are involved in the signal transduction steps which drive proliferation and survival of these tumour cells. Accordingly the compounds of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, erbB2 receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours According to this aspect of the invention there is provided a quinazoline derivative of the formula I, or a pharmaceutically acceptable salt thereof, for use as a medicament.

Thus according to this aspect of the invention there is provided the use of a quinazoline derivative of the formula I, or a pharmaceutically-acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatment may comprise administering to said animal an effective amount of a quinazoline derivative of the formula I, or a pharmaceutically acceptable salt thereof, as hereinbefore defined.

According to a further aspect of the invention there is provided a quinazoline derivative of the formula I, or a pharmaceutically acceptable salt thereof, for use in the production of an anti-proliferative effect in a warm-blooded animal such as man.

According to a further aspect of the invention there is provided the use of a quinazoline derivative of the formula I, or a pharmaceutically-acceptable salt thereof, as defined hereinbefore in the manufacture of a medicament for use in the production of an anti-proliferative effect which effect is produced alone or in part by inhibiting erbB2 receptor tyrosine kinase in a warm-blooded animal such as man.

A method for producing an anti-proliferative effect which effect is produced alone or in part by inhibiting erbB2 receptor tyrosine kinase in a warm-blooded animal, such as man, in need of such treatment may comprise administering to said animal an effective amount of a quinazoline derivative of the formula I, or a pharmaceutically acceptable salt thereof, as hereinbefore defined.

According to a further aspect of the invention there is provided a quinazoline derivative of the formula I, or a pharmaceutically acceptable salt thereof, for use in the production of an anti-proliferative effect which effect is produced alone or in part by inhibiting erbB2 receptor tyrosine kinase in a warm-blooded animal such as man.

According to a further aspect of the present invention there is provided the use of a quinazoline derivative of the formula I, or a pharmaceutically-acceptable salt thereof, as defined hereinbefore in the manufacture of a medicament for use in the treatment of a disease or medical condition (for example a cancer as mentioned herein) mediated alone or in part by erbB2 receptor tyrosine kinase.

A method for treating a disease or medical condition (for example a cancer as mentioned herein) mediated alone or in part by erbB2 receptor tyrosine kinase in a warm-blooded animal, such as man, in need of such treatment, may comprise administering to said animal an effective amount of a quinazoline derivative of the formula I, or a pharmaceutically-acceptable salt thereof, as defined hereinbefore.

According to a further aspect of the invention there is provided a quinazoline derivative of the formula I, or a pharmaceutically acceptable salt thereof, for use in the treatment of a disease or medical condition (for example a cancer as mentioned herein) mediated alone or in part by erbB2 receptor tyrosine kinase.

According to a further aspect of the invention there is provided the use of a quinazoline derivative of the formula I, or a pharmaceutically-acceptable salt thereof, as defined hereinbefore in the manufacture of a medicament for use in the prevention or treatment of those tumours which are sensitive to inhibition of erbB2 receptor tyrosine kinase that is involved in the signal transduction steps which lead to the proliferation of tumour cells.

A method for the prevention or treatment of those tumours which are sensitive to inhibition of erbB2 receptor tyrosine kinase, that is involved in the signal transduction steps which lead to the proliferation and/or survival of tumour cells in a warm-blooded animal, such as man, in need of such treatment, may comprise administering to said animal an effective amount of a quinazoline derivative of the formula I, or a pharmaceutically-acceptable salt thereof, as defined hereinbefore.

According to a further aspect of the invention there is provided a quinazoline derivative of the formula I, or a pharmaceutically acceptable salt thereof, for use in the prevention or treatment of those tumours which are sensitive to inhibition of the erbB2 receptor tyrosine kinase, that is involved in the signal transduction steps which lead to the proliferation and/or survival of tumour cells. According to a further aspect of the invention there is provided the use of a quinazoline derivative of the formula I, or a pharmaceutically-acceptable salt thereof, as defined hereinbefore in the manufacture of a medicament for use in providing a erbB2 receptor tyrosine kinase inhibitory effect.

A method for providing an erbB2 receptor tyrosine kinase inhibitory effect in a warm-blooded animal, such as man, in need of such treatment, may comprise administering to said animal an effective amount of a quinazoline derivative of the formula I, or a pharmaceutically-acceptable salt thereof, as defined hereinbefore.

According to a further aspect of the invention there is provided a quinazoline derivative of the formula I, or a pharmaceutically acceptable salt thereof, for use in providing an erbB2 receptor tyrosine kinase inhibitory effect.

According to a further aspect of the invention there is provided the use of a quinazoline derivative of the formula I, or a pharmaceutically-acceptable salt thereof, as defined hereinbefore in the manufacture of a medicament for use in providing a selective erbB2 kinase inhibitory effect.

A method for providing a selective erbB2 kinase inhibitory effect in a warm-blooded animal, such as man, in need of such treatment, may comprise administering to said animal an effective amount of a quinazoline derivative of the formula I, or a pharmaceutically-acceptable salt thereof, as defined hereinbefore.

According to a further aspect of the invention there is provided a quinazoline derivative of the formula I, or a pharmaceutically acceptable salt thereof, for use in providing a selective erbB2 kinase inhibitory effect.

By "a selective erbB2 kinase inhibitory effect" is meant that the quinazoline derivative of formula I is more potent against erbB2 receptor tyrosine kinase than it is against other kinases. In particular the quinazoline derivative of formula I is more potent against erbB2 receptor kinase than it is against EGFR tyrosine kinase. For example in a cellular assay (such as the H16N-2 assay described herein) the quinazoline derivative of formula I is at least 5 times, preferably at least 10, more preferably at least 100 times more potent against erbB2 receptor tyrosine kinase driven proliferation than it is against EGFR tyrosine kinase driven proliferation, as determined from the relative IC₅₀ values.

According to a further aspect of the present invention there is provided the use of a quinazoline derivative of the formula I, or a pharmaceutically-acceptable salt thereof, as defined hereinbefore in the manufacture of a medicament for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

A method for treating a cancer, for example a cancer selected from selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer in a warm-blooded animal, such as man, in need of such treatment, may comprise administering to said animal an effective amount of a quinazoline derivative of the formula I, or a pharmaceutically-acceptable salt thereof, as defined hereinbefore.

According to a further aspect of the invention there is provided a quinazoline derivative of the formula I, or a pharmaceutically acceptable salt thereof, for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulfan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine/threonine kinase inhibitors, for example other inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a quinazoline derivative of the formula I as defined hereinbefore and an additional anti-tumour agent as defined hereinbefore for the conjoint treatment of cancer.

Although the compounds of the formula I are primarily of value as therapeutic agents for use in warm-blooded animals (including man), they are also useful whenever it is required to inhibit the effects of the erbB2 receptor tyrosine protein kinases. Thus, they are useful as pharmacological standards for use in the development of new biological tests and in the search for new pharmacological agents.

The invention will now be illustrated by the following non-limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulfoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulfur atom have not been resolved;
(xii) where a synthesis is described as being analogous to that described in a previous example the amounts used are the millimolar ratio equivalents to those used in the previous example;
(xiii) all microwave reactions were carried out in a CEM Discover™ microwave synthesisor;
(xiv) preparative high performance liquid chromatography (HPLC) was performed on a Gilson instrument using the following conditions:

| | |
|---|---|
| Column: | 21 mm x 10 cm Hichrom RPB |
| Solvent A: | Water + 0.1% trifluoracetic acid, |
| Solvent B: | Acetonitrile + 0.1% trifluoroacetic acid |
| Flow rate: | 18 ml / min |
| Run time: | 15 minutes with a 10 minute gradient from 5-95% B |
| Wavelength: | 254 nm, bandwidth 10 nm |
| Injection volume | 2.0-4.0 ml; |

(xv) the following abbreviations have been used:
- THF: tetrahydrofuran;
- DMF: *N,N*-dimethylformamide;
- DMA: *N,N*-dimethylacetamide;
- DCM: dichloromethane;
- DMSO: dimethylsulfoxide;
- IPA: isopropyl alcohol; and
- ether: diethyl ether.

### Example 1 4-(3-Chloro-4-(2-pyridylmethoxy)anilino)-5-(2-dimethylaminoethoxy)quinazoline

4-(3-Chloro-4-(2-pyridylmethoxy)anilino)-5-fluoroquinazoline (114 mg), N,N-dimethylethanolamine (30 mg), 60% sodium hydride dispersion in oil (40 mg) and 1,4,7,10,13-pentaoxacyclopentadecane (1 drop) were added to 1,4-dioxane (5 ml) in a 10 ml microwave reaction tube. The reaction tube was sealed and heated in a CEM Discover™ microwave synthesisor for 15 minutes at 150°C. The reaction was cooled, the solution was loaded onto a 10 g silica column and the column was eluted with 5-10% methanol/ethyl acetate. The appropriate fractions were combined and concentrated, and the resulting gum was triturated with ether to give the title compound (59 mg, 66%); NMR spectrum (DMSO-d6) 2.3 (s, 6H), 2.8-2.9 (t, 2H), 4.3-4.4 (t, 2H), 5.25 (s, 2H), 7.1-7.15 (d, 1H), 7.2-7.25 (d, 1H), 7.3-7.4 (m, 2H), 7.55-7.6 (d, 1H), 7.6-7.7 (t, 1H), 7.7-7.8 (dd, 1H), 7.8-7.9 (t, 1H), 7.95 (s, 1H), 8.5 (s, 1H), 8.5-8.6 (d, 1H), 10.3-10.4 (br s, 1H); Mass spectrum MH⁺ 450.2.

The 4-(3-chloro-4-(2-pyridylmethoxy)anilino)-5-fluoroquinazoline used as starting material was obtained as follows:
DMF (0.2 ml) was added to a suspension of 5-fluoro-3,4-dihydro-3H-quinazolin-4-one (1.64 g) in thionyl chloride (10 ml) and the mixture was stirred and heated at 80°C for 6 hours. Volatile material was removed by evaporation and the residue was azeotroped with toluene (20 ml). The resulting solid was added portionwise to a vigorously stirred mixture of saturated sodium bicarbonate (50 ml), crushed ice (50 g) and DCM (50 ml) such that the temperature was kept below 5°C. The organic phase was separated, dried and concentrated to give 4-Chloro-5-fluoroquinazoline (1.82 g, 99%) as a solid which was used without purification; NMR spectrum (CDCl₃) 7.35-7.45 (m, 1H), 7.85-7.95 (m, 2H), 9.0 (s, 1H).

4-Chloro-5-fluoroquinazoline (6.75 g) was added to stirred solution of 3-chloro-4-(2-pyridylmethoxy)aniline (9.27 g) (obtained as described in Example 13 of WO 96/15118) in IPA (200 ml), and the solution was stirred and heated under reflux for 8 hours. The solution was allowed to cool to ambient temperature overnight and the precipitated solid was filtered off, washed with acetone and dried. The solid was added to 50% aqueous methanol (400 ml) and the mixture was heated on a steam bath until all the solid had dissolved. The solution was basified by careful addition of aqueous ammonia (0.880), and the mixture was concentrated to remove methanol. Water (300 ml) was added and the mixture was extracted with DCM (600 ml). The extract was washed with water and saturated brine and dried. The solvent was removed by evaporation to give a solid, which was re-crystallised from a mixture of ethyl acetate, tetrahydrofuran and isohexane to give 4-(3-chloro-4-(2-pyridylmethoxy)anilino)-5-fluoroquinazoline as beige crystals (6.75 g, 48%); NMR spectrum (DMSO-d6) 5.3 (s, 2H), 7.2-7.3 (d, 1H), 7.35-7.5 (m, 2H), 7.5-7.65 (m, 3H), 7.8-7.95 (m, 3H), 8.55 (s, 1H), 8.55-8.6 (d, 1H), 9.1-9.2 (br s, 1H); Mass spectrum MH⁺ 381.4.

### Example 2 4-(3-Chloro-4-(2-pyridylmethoxy)anilino)-5-(2-dimethylamino-1-methylethoxy)quinazoline

The procedure described in Example 1 was repeated using 4-(3-chloro-4-(2-pyridylmethoxy)anilino)-5-fluoroquinazoline and 1-dimethylaminopropan-2-ol to give the title product in (26% yield ); NMR spectrum (DMSO-d6) 1.4-1.5 (d, 3H), 2.15-2.25 (s, 6H), 2.35-2.5 (dd, 1H), 2.85-3.0 (dd, 1H), 4.8-4.95 (m, 1H), 5.3 (s, 2H), 7.1-7.2 (d, 1H), 7.2-7.4 (m, 3H), 7.55-7.7 (m, 2H), 7.7-7.78 (t, 1H), 7.8-7.9 (t, 1H), 7.9-8.0 (d, 1H), 8.5 (s, 1H), 8.55-8.6 (d, 1H), 10.4-10.45 (br s, 1H); Mass spectrum MH⁺ 464.5.

### Example 3 4-(3-Chloro-4-(1-methyl-1H-imidazol-2-ylthio)anilino)-5-(2-dimethylaminoethoxy)quinazoline

4-(3-Chloro-4-(1-methyl-1*H*-imidazol-2-ylthio)anilino)-5-fluoroquinazoline (96 mg), N,N-dimethylethanolamine (49 mg), 60% sodium hydride dispersion in oil (22 mg) and 1,4,7,10,13-pentaoxacyclopentadecane (1 drop) were added to 1,4-dioxane (5 ml) in a 10 ml microwave reaction tube. The reaction tube was sealed and heated in a CEM Discover™ microwave synthesisor for 15 minutes at 140°C. The reaction was cooled and 5% methanol/ethyl acetate (1 ml) and acetic acid (4 drops) were added. The solution was loaded onto a 10 g silica column and the column was eluted with 5-10% methanol/ethyl acetate. The appropriate fractions were combined and concentrated, and the resulting gum was triturated with ether to give the title compound (55 mg, 48%); NMR spectrum (DMSO-d6) 2.3 (s, 6H), 2.8-2.9 (t, 2H), 3.7 (s, 3H), 4.4-4.5 (t, 2H), 6.9-6.95 (d, 1H), 7.15 (s, 1H), 7.15-7.2 (d, 1H), 7.4-7.45 (m, 2H), 7.7-7.8 (t, 1H), 7.8-7.9 (dd, 1H), 8.1 (s, 1H), 8.6 (s, 1H), 10.4-10.5 (br s, 1H); Mass spectrum MH⁺ 455.4.

The 4-(3-chloro-4-(1-methyl-1*H*-imidazol-2-ylthio)anilino)-5-fluoroquinazoline used as starting material was obtained in 72% yield by reacting 4-chloro-5-fluoroquinazoline (obtained as described in Example 1, preparation of starting materials) and 3-chloro-4-(1-methyl-1*H*-imidazol-2-ylthio)aniline (obtained as described in Example 53 of US 4,973,599) using an analogous procedure to that described in the preparation of 4-(3-chloro-4-(2-pyridylmethoxy)anilino)-5-fluoroquinazoline in Example 1; NMR spectrum (DMSO-d6) 3.78 (s, 3H), 6.7-6.75 (d, 1H), 7.3 (br s, 1H), 7.5-7.6 (dd, 1H), 7.65 (s, 1H), 7.7-7.8 (dt, 2H), 7.9-8.0 (m, 1H), 8.2 (d, 1H), 8.75 (s, 1H), 9.3-9.4 (d, 1H); Mass spectrum MH⁺ 386.5.

### Example 4 4-(3-Chloro-4-(1-methyl-1H-imidazol-2-ylthio)anilino)-5-(2-dimethylamino-2-methylethoxy)quinazoline

The procedure described in Example 1 was repeated using 4-(3-chloro-4-(1-methyl-1*H*-imidazol-2-ylthio)anilino)-5-fluoroquinazoline (obtained as described in Example 3, preparation of starting materials) and 2-dimethylaminopropan-1-ol to give the title product (20% yield); NMR spectrum (DMSO-d6) 1.0-1.1 (d, 3H), 2.3 (s, 6H), 3.1-3.2 (m, 1H), 3.7 (s, 3H), 4.15-4.25 (dd, 1H), 4.3-4.4 (dd, 1H), 6.85-6.9 (d, 1H), 7.1 (s, 1H), 7.15-7.2 (d, 1H), 7.35-7.45 (m, 2H), 7.7-7.85 (m, 2H), 8.1 (s, 1H), 8.6 (s, 1H), 10.5-10.6 (br s, 1H); Mass spectrum MH⁺ 469.5.

### Example 5 4-(4-(3-Fluorobenzyloxy)anilino)-5-(2-dimethylaminoethoxy)quinazoline

A mixture of 60% sodium hydride dispersion in oil (25 mg) and N,N-dimethylethanolamine (56 mg) in dry 1,4-dioxane (5 ml) was stirred at room temperature for 5 minutes. 4-(4-(3-Fluorobenzyloxy)anilino)-5-fluoroquinazoline (100 mg) was added and the mixture was heated under reflux overnight. Saturated ammonium chloride (2 ml) was added and volatile material was removed by evaporation. Saturated sodium hydrogen carbonate (10 ml) was added to the residue and the mixture was extracted with DCM (3 x 15 ml). The combined extracts were dried and concentrated and the residue was purified by chromatography, eluting with 5% methanol/ethyl acetate. The appropriate fractions were combined and concentrated to give the title compound (70 mg, 58%); NMR spectrum (CDCl₃) 2.21 (s, 6H), 2.74 (t, 2H), 4.17 (t, 2H), 4.99 (s, 2H), 6.75 (d, 1H), 6.91 (m, 3H), 7.11 (m, 2H), 7.35 (m, 2H), 7.50 (m, 1H), 7.59 (m, 2H), 8.5 (s, 1H), 10.26 (s, 1H); Mass spectrum MH⁺ 433.2.

The 4-(4-(3-fluorobenzyloxy)anilino)-5-fluoroquinazoline used as starting material was obtained as follows:

4-(3-Fluorobenzyloxy)aniline (1.31 g) (obtained using an analogous method to that disclosed in WO98/02434, preparation of intermediates pages 44-45) and 4-chloro-5-fluoroquinazoline (1 g) (prepared as described in Example 1, preparation of starting materials) were suspended in IPA (50 ml) and the mixture was heated under reflux for 15 minutes. The solvent was removed by evaporation and methanol (10 ml) and saturated sodium hydrogen carbonate (50 ml) were added to the residue. The mixture was extracted with ethyl acetate (3 x 50 ml) and the combined extracts were washed with saturated brine (3 x 50 ml) and dried. Volatile material was removed by evaporation to give 4-(4-(3-fluorobenzyloxy)anilino)-5-fluoroquinazoline as a yellow oil which crystallised on standing (1.86 g, 93%); NMR spectrum (DMSO-d6) 5.28 (s, 2H), 7.16 (d, 2H), 7.28 (m, 1H), 7.41 (m, 2H), 7.56 (m, 2H), 7.72 (d, 2H), 7.93 (m, 1H), 8.61 (s, 1H), 9.18 (d, 1H); Mass spectrum MH⁺ 364.2.

### Example 6 4-(4-(3-Fluorobenzyloxy)anilino)-5-(2-dimethylamino-1-methylethoxy)quinazoline

4-(4-(3-Fluorobenzyloxy)anilino)-5-fluoroquinazoline (obtained as described in Example 5) was reacted with 1-dimethylaminopropan-2-ol using the procedure described in Example 5 to give the title product (56% yield); NMR spectrum (DMSO-d6) 1.57 (d, 3H), 2.31 (s, 6H), 2.55 (m, 1H), 3.02 (m, 1H), 5.00 (m, 1H), 5.27 (s, 2H), 7.18 (d, 2H), 7.29 (m, 2H), 7.42 (m, 3H), 7.57 (m, 1H), 7.78 (m, 3H), 8.54 (s, 1H), 10.50 (s, 1H); Mass spectrum MH⁺ 447.2.

### Example 7 4-(3-Chloro-4-(2-pyrazinylmethoxy)anilino)-5-(2-dimethylaminoethoxy)quinazoline

4-(3-Chloro-4-(2-pyazinylmethoxy)anilino)-5-fluoroquinazoline was reacted with and N,N-dimethylethanolamine using the procedure described in Example 5 to give the title product (32% yield); NMR spectrum (CDCl₃) 2.28 (s, 6H), 2.79 (t, 2H), 4.21 (t, 2H), 5.26 (s, 2H), 6.81 (d, 1H), 6.97 (d, 1H), 7.38 (d, 1H), 7.56 (m, 1H), 7.76 m, 1H), 7.88 (m, 1H), 8.50 (s, 2H), 8.56 (s, 1H), 8.92 (s, 1H), 10.36 (bs, 1H; Mass spectrum MH⁺ 451.2.

The 4-(3-chloro-4-(2-pyazinylmethoxy)anilino)-5-fluoroquinazoline used as starting material was obtained as follows:
Methylpyrazine carboxylate (8.5g) was stirred in water (200 ml) and sodium borohydride (11.65 g) was added in one portion, resulting in a vigorous exotherm. The reaction mixture was stirred vigorously for 30 minutes, and then ethanol (80 ml) and saturated potassium carbonate (150 ml) were added. The mixture was stirred for 30 minutes and then extracted with ethyl acetate (5 x 150 ml) and DCM (5 x 150 ml). The combined extracts were dried and concentrated to give pyrazin-2-ylmethanol as a yellow oil (5.43 g, 80%), which was used without purification; NMR spectrum (DMSO-d6) 4.65 (s, 2H), 5.57 (br s, 1H), 8.54 (d, 2H), 8.71 (s, 1H).
Pyrazin-2-ylmethanol (1.5 g) was dissolved in DMA (25 ml) and the solution was cooled to 0°C. 60% Sodium hydride dispersion in oil (0.6 g) was added portionwise and the mixture was stirred for 10 minutes at 0°C. A solution of 3-chloro-4-fluoronitrobenzene (2.18 g) in DMA (25 ml) was added over 15 minutes and the reaction mixture was allowed to warm to room temperature and stirred for 3 hours. Saturated ammonium chloride (100 ml) was added, and the precipitated solid was filtered off and purified by chromatography eluting with 50% ethyl acetate/iso-hexane. The appropriate fractions were concentrated to give 3-Chloro-4-(2-pyrazinylmethoxy)nitrobenzene as a brown solid (1.25 g, 38%); NMR spectrum (DMSO-d6) 5.67 (s, 2H), 7.65 (d, 1H), 8.37 (m, 1H), 8.48 (d, 1H), 8.81 (m, 2H), 8.99 (s, 1H); Mass spectrum MH⁺ 264.1.

A solution of 3-chloro-4-(2-pyrazinylmethoxy)nitrobenzene (1.25 g) in ethyl acetate (100 ml) was catalytically hydrogenated over 10% platinum on carbon (400 mg) at ambient temperature overnight. The reaction mixture was filtered through diatomaceous earth and the filtrate was concentrated to give 3-chloro-4-(2-pyrazinylmethoxy)aniline as a yellow solid (1.03 g, 94%); NMR spectrum (DMSO-d6) 5.09 (br s, 2H), 5.27 (s, 2H), 6.59 (m, 1H), 6.77 (d, 1H), 7.08 (d, 1H), 8.75 (m, 2H), 8.91 (s, 1H); Mass spectrum MH⁺ 236.1.

4-Chloro-5-fluoroquinazoline (obtained as described in Example 1) was reacted with 3-chloro-4-(2-pyrazinylmethoxy)aniline using the procedure described in Example 5 for the preparation of starting materials to give 4-(3-Chloro-4-(2-pyazinylmethoxy)anilino)-5-fluoroquinazoline (76% yield); NMR spectrum (DMSO-d6) 5.51 (s, 2H), 7.45 (d, 1H), 7.55 (m, 1H), 7.74 (m, 2H), 7.96 (m, 1H), 8.04 (d, 1H), 8.67 (s, 1H), 8.77 (m, 1H), 8.81 (m, 1H), 8.98 (d, 1H), 9.28 (d, 1H); Mass spectrum MH⁺ 382.1.

### Example 8 4-(3-Chloro-4-(2-pyrazinylmethoxy)anilino)-5-(2-dimethylamino-1-methylethoxy)quinazoline

The procedure described in Example 5 was repeated but using 4-(3-chloro-4-(2-pyazinylmethoxy)anilino)-5-fluoroquinazoline (obtained as described in Example 7, preparation of starting materials) and 1-dimethylaminopropan-2-ol to give the title product (25% yield); NMR spectrum (CDCl₃) 1.48 (d, 3H), 2.22 (s, 6H), 2.40 (m, 1H), 2.85 (m, 1H), 4.67 (m, 1H), 5.25 (s, 2H), 6.85 (d, 1H), 6.98 (d, 1H), 7.36 (d, 1H), 7.54 (t, 1H), 7.67 (d, 1H), 7.73 (s, 1H), 8.50 (s, 2H), 8.53 (s, 1H), 8.91 (s, 1H), 10.32 (s, 1H); Mass spectrum MH⁺ 465.1.

### Example 9 4-(3-Chloro-4-(5-methylisoxazol-3-ylmethoxy)anilino)-5-(2-dimethylaminoethoxy)quinazoline

The procedure described in Example 5 was repeated using 4-(3-chloro-4-(5-methylisoxazol-3-ylmethoxy)anilino)-5-fluoroquinazoline and N,N-dimethylethanolamine to give the title product (73% yield); NMR spectrum (CDCl₃) 2.34 (s, 6H), 2.44 (s, 3H), 2.84 (t, 2H), 4.26 (t, 2H), 5.19 (s, 2H), 6.20 (s, 1H), 6.85 (d, 1H), 7.04 (d, 1H), 7.44 (d, 1H), 7.60 (m, 1H), 7.84 (m, 2H), 8.61 (s, 1H) 10.40 br s, 1H); Mass spectrum MH⁺ 454.4.

The 4-(3-chloro-4-(5-methylisoxazol-3-ylmethoxy)anilino)-5-fluoroquinazoline used as starting material was obtained as follows:

A mixture of 4-amino-2-chlorophenol (1.2 g), 3-chloromethyl-5-methylisoxazole (1.21 g) potassium carbonate (4.04 g) and 1,4,7,10,13,16-hexaoxacyclooctadecane (100 mg) in DMF (25 ml) was stirred and heated at 60°C overnight. Water (250 ml) was added and the mixture was extracted with ethyl acetate (3 x 200 ml). The combined extracts were washed with saturated brine (3 x 150 ml), dried and concentrated. The residue was purified by chromatography, eluting with 20% ethyl acetate/iso-hexane. The appropriate fractions were combined and concentrated to give 3-Chloro-4-(5-methylisoxazol-3-ylmethoxy)aniline as a pink solid (1.2 g, 60%); NMR spectrum (CDCl₃) 2.35 (s, 3H), 3.44 (br s, 2H), 5.00 (s, 2H), 6.09 (s, 1H), 6.46 (m, 1H), 6.67 (d, 1H), 6.77 (d, 1H); Mass spectrum MH⁺ 239.1.

The procedure described in Example 5, preparation of starting materials, was repeated using 4-chloro-5-fluoroquinazoline (obtained as described in Example 1) and 3-chloro-4-(2-pyrazinylmethoxy)aniline to give 4-(3-Chloro-4-(5-methylisoxazol-3-ylmethoxy)anilino)-5-fluoroquinazoline (71% yield); NMR spectrum (CDCl₃) 2.37 (s, 3H), 5.14 (s, 2H), 6.12 (s, 1H), 7.01 (d, 1H), 7.14 (m, 1H), 7.44 (m, 1H), 7.64 (m, 2H), 7.83 (d, 1H), 8.21 (d, 1H), 8.63 (s, 1H); Mass spectrum MH⁺ 385.1.

### Example 10 4-(3-Chloro-4-(5-methylisoxazol-3-ylmethoxy)anilino)-5-(2-dimethylamino-1-methylethoxy)quinazoline

The procedure described in Example 5 was repeated using 4-(3-chloro-4-(5-methylisoxazol-3-ylmethoxy)anilino)-5-fluoroquinazoline (obtained as described in Example 9.2) and 1-dimethylamino-2-propanol to give the title compound (79% yield); NMR spectrum (CDCl₃) 1.44 (d, 3H), 2.21 (s, 6H), 2.37 (m, 1H), 2.84 (m, 1H), 4.66 (m, 1H), 5.12 (s, 2H), 6.11 (s, 1H), 6.83 (d, 1H), 6.97 (d, 1H), 7.35 (d, 1H), 7.53 (m, 2H), 7.75 (d, 1H), 8.52 (s, 1H), 10.30 (s, 1H); Mass spectrum MH⁺ 468.45.

### Example 11 4-(3-Chloro-4-(3-fluorobenzyloxy)anilino)-5-(2-(N-ethyl-N-methylamino)ethoxy)quinazoline trifluoroacetate

4-(3-Chloro-4-(3-fluorobenzyloxy)anilino)-5-(2-chloroethoxy)quinazoline (100 mg), N-ethylmethylamine (65 mg) and tetra-n-butylammonium iodide (81 mg) were dissolved in 1,4-dioxane (5 ml) in a 10 ml microwave reaction tube. The reaction tube was sealed and heated in a CEM Discover™ microwave synthesisor at 150°C for 15 minutes. Volatile material was removed by evaporation and the residue was partitioned between DCM (10 ml) and water (10 ml). The organic phase was separated, dried and concentrated and the residue was purified by preparative HPLC. The appropriate fractions were combined and concentrated and the resulting gum was triturated with ether to give the title compound (34 mg, 32%); NMR spectrum (DMSO-d6) 1.24 (t, 3H), 2.91 (s, 3H), 3.29 (d, 2H), 3.78 (d, 2H), 4.82 (s, 2H), 5.32 (s, 2H), 7.18 (t, 1H), 7.31-7.38 (m, 4H), 7.47 (m, 2H), 7.59 (dd, 1H), 7.87 (s, 1H), 7.93 (t, 1H), 8.71 (s, 1H), 10.12 (s, 1H); Mass spectrum MH⁺ 481.0.

The 4-(3-chloro-4-(3-fluorobenzyloxy)anilino)-5-(2-chloroethoxy)quinazoline used as starting material was obtained as follows:
Using an analogous procedure to that described for the preparation of 4-(3-chloro-4-(2-pyridylmethoxy)anilino)-5-fluoroquinazoline in Example 1, 4-chloro-5-fluoroquinazoline (obtained as described in Example 1) and 3-chloro-4-(3-fluorobenzyloxy)aniline (obtained using an analogous procedure to that described in WO 98/02434, page 44-45) were reacted to give 4-(3-Chloro-4-(3-fluorobenzyloxy)anilino)-5-fluoroquinazoline in 47% yield; NMR spectrum (DMSO-d6) 5.29 (s, 2H), 7.18 (t, 1H), 7.30 (m, 3H), 7.49 (t, 1H), 7.52 (m, 1H), 7.63 (dd, 1H), 7.82 (m, 1H), 8.06 (m, 1H), 8.81 (s, 1H), 8.92 (s, 1H), 10.32 (s, 1H); Mass spectrum MH⁺ 398.5.

60% Sodium hydride dispersion in oil (738 mg) was added portionwise to 4-(3-chloro-4-(3-fluorobenzyloxy)anilino)-5-fluoroquinazoline (1.75 g) and 4-methoxybenzylalcohol (1.72 ml) in dry DMF (40 ml) under an atmosphere of nitrogen. The mixture was stirred until hydrogen evolution ceased and then heated at 100°C for 3.5 hours. Volatile material was removed by evaporation and the resulting slurry was suspended in ethyl acetate (30 ml) and saturated sodium bicarbonate (30 ml). The insoluble solid was filtered off and washed with water (2 x 30 ml) and ether (30 ml) to give 4-(3-Chloro-4-(3-fluorobenzyloxy)anilino)-5-(4-methoxybenzyloxy)quinazoline as a pale solid (1.9 g, 87%); NMR spectrum (DMSO-d6) 3.80 (s, 3H), 5.24 (s, 2H), 5.34 (s, 2H), 7.05 (d, 2H), 7.17 (d, 1H), 7.29 (d, 1H), 7.38 (m, 2H), 7.44 (m, 2H), 7.58 (d, 1H), 7.62 (d, 2H), 7.78 (t, 1H), 7.88 (t, 1H), 8.54 (s, 1H), 8.59 (d, 1H), 10.10 (s, 1H); Mass spectrum MH⁺ 516.0.

Trifluoroacetic acid (4 ml) was added to a stirred solution of 4-(3-chloro-4-(3-fluorobenzyloxy)anilino)-5-(4-methoxybenzyloxy)quinazoline (3 g) in DCM (3 ml). The mixture was stirred for 45 minutes and then volatile material was removed by evaporation. The resulting solid was suspended in a mixture of methanol (70 ml) and water (30 ml), and the mixture was basified with saturated sodium bicarbonate. The suspension was stirred vigorously for 1hour, and the insoluble solid was filtered off and washed with water (2 x 60 ml) and ether (2 x 40 ml). Trituration with methanol gave 4-(3-Chloro-4-(3-fluorobenzyloxy)anilino)-5-hydroxyquinazoline as a yellow solid (2.24 g, 97%); NMR spectrum (DMSO-d6) 5.27 (s, 2H), 6.78 (d, 1H), 6.81 (d, 1H), 7.18 (t, 1H), 7.24 (d, 1H), 7.31 (m, 2H), 7.45-7.52 (m, 3H), 7.98 (s, 1H), 8.40 (s, 1H); Mass spectrum MH⁺ 396.4.

A mixture of 4-(3-chloro-4-(3-fluorobenzyloxy)anilino)-5-hydroxyquinazoline (2.86 g), 1-bromo-2-chloroethane (1.21 ml) and cesium carbonate (7.08 g) in acetonitrile (150 ml) was heated at 90°C for 3 hours. Volatile material was removed by evaporation and the residue was partitioned between DCM (60 ml) and water (40 ml). The organic phase was separated, washed with water (2 x 40 ml), dried and concentrated. The residual solid was triturated with ether to give 4-(3-Chloro-4-(3-fluorobenzyloxy)anilino)-5-(2-chloroethoxy)quinazoline as a yellow solid (2.3 g, 70%); NMR spectrum (DMSO-d6) 4.43 (t, 2H), 4.71 (t, 2H), 5.37 (s, 2H), 7.30 (m, 2H), 7.39 (d, 1H), 7.43 (m, 2H), 7.50 (d, 1H), 7.57 (m, 1H), 7.76 (dd, 1H), 7.88 (t, 1H), 8.28 (d, 1H), 8.68 (s, 1H), 10.08 (s, 1H); Mass spectrum MH⁺ 58.1.

### Example 12 4-(3-Chloro-4-(3-fluorobenzyloxy)anilino)-5-(2-dimethylaminoethoxy)quinazoline

3-Chloro-4-(3-fluorobenzyloxy)anilino)-5-(2-chloroethoxy)quinazoline (100 mg) (obtained as described in Example 11, preparation of starting materials), 2M dimethylamine in THF (1.1 ml) and tetra-n-butylammonium iodide (81 mg) were dissolved in 1,4-dioxane (5 ml) in a 10 ml microwave reaction tube. The reaction tube was sealed and heated in a CEM Discover™ microwave synthesisor at 150°C for 15 minutes. Volatile material was removed by evaporation and the residue was partitioned between DCM (10 ml) and water (10 ml). The organic phase was separated, dried and concentrated and the residue was purified by preparative HPLC. The appropriate fractions were combined and concentrated and the residue was dissolved in water (5 ml). The solution was neutralised with saturated sodium bicarbonate to precipitate the title compound as a yellow solid (30 mg, 29%); NMR spectrum (DMSO-d6) 2.28 (s, 6H), 2.81 (t, 2H), 4.48 (t, 2H), 5.23 (s, 2H), 7.13 (m. 2H), 7.34 (d, 1H), 7.30 (m, 3H), 7.44 (m, 1H), 7.71 (t, 1H), 7.75 (dd, 1H), 7.96 (d, 1H), 8.50 (s, 1H), 10.32 (s, 1H); Mass spectrum MH⁺ 467.5.

### Example 13 4-(3-Chloro-4-(3-fluorobenzyloxy)anilino)-5-[2-(N-(2-hydroxyethyl)-N-methylamino)ethoxy]quinazoline

The procedure described in Example 12 was repeated using 4-(3-chloro-4-(3-fluorobenzyloxy)anilino)-5-(2-chloroethoxy)quinazoline (obtained as described in Example 11) and N-methylethanolamine to give the title compound in 34% yield; NMR spectrum (DMSO-d6) 2.39 (s, 3H), 2.60 (t, 2H), 2.97 (t, 2H), 3.50 (m, 2H), 3.97 (t, 1H), 4.41 (t, 2H), 5.55 (s, 2H), 7.13 (m, 2H), 7.23 (d, 2H), 7.29 (d, 1H), 7.36 (m, 1H), 7.45 (m, 1H), 7.70 (m, 2H), 8.0 (d, 1H), 8.50 (s, 1H), 10.20 (s, 1H); Mass spectrum MH⁺ 497.6.

### Example 14 4-(3-Chloro-4-(2-pyridylmethoxy)anilino)- 5-(2-(N-ethyl-N-methylamino)ethoxy)quinazoline trifluoroacetate

The procedure described in Example 11 was repeated using 4-(3-chloro-4-(2-pyridylmethoxy)anilino)-5-(2-chloroethoxy)quinazoline and N-ethyl-N-methylamine to give the title compound in 22% yield; NMR spectrum (DMSO-d6) 1.15 (t, 3H), 2.85 (s, 3H), 3.22 (m, 2H), 3.71 (m, 2H), 4.71 (m, 2H), 5.28 (s, 2H), 7.27 (d, 1H), 7.31 (m, 2H), 7.39 (d, 1H), 7.51 (m, 2H), 1.82 (m, 2H), 7.89 (t, 1H), 8.55 (d, 1H), 8.68 (d, 1H) 10.10 (s, 1H); Mass spectrum MH⁺ 464.5.

The 4-(3-chloro-4-(2-pyridylmethoxy)anilino)-5-(2-chloroethoxy)quinazoline used as starting material was obtained as follows:
The procedure described in Example 11, for the preparation of 4-(3-Chloro-4-(3-fluorobenzyloxy)anilino)-5-(4-methoxybenzyloxy)quinazoline, was repeated but using 4-(3-chloro-4-(2-pyridylmethoxy)anilino)-5-fluoroquinazoline (obtained as described in Example
   1) and 4-methoxybenzyl alcohol to give 4-(3-Chloro-4-(2-pyridylmethoxy)anilino)-5-(4-methoxybenzyloxy)quinazoline as a pale white solid in 87% yield; NMR spectrum (DMSO-d6) 3.80 (s, 3H), 5.27 (s, 2H), 5.34 (s, 2H), 7.05 (d, 2H), 7.15 (d, 1H), 7.29 (d, 1H), 7.38 (m, 2H), 7.43 (m, 2H), 7.57 (d, 1H), 7.64 (d, 2H), 7.78 (t, 1H), 7.87 (t, 1H), 8.53 (s, 1H), 8.59 (d, 1H), 10.09 (s, 1H); Mass spectrum MH⁺ 499.4.

The 4-(3-chloro-4-(2-pyridylmethoxy)anilino)-5-(4-methoxybenzyloxy)quinazoline was reacted with trifluoroacetic acid using the procedure described in Example 11 to give 4-(3-Chloro-4-(2-pyridylmethoxy)anilino)-5-hydroxyquinazoline as a yellow solid in 93% yield; NMR spectrum (DMSO-d6) 5.29 (s, 2H), 7.03 (m, 2H), 7.32 (d, 1H), 7.37 (t, 1H), 7.52 (dd, 1H), 7.58 (d, 1H), 7.72 (t, 1H), 7.88 (t, 1H), 7.94 (s, 1H), 8.60 (d, 1H), 8.62 (s, 1H), 12.30 (s, 1H); Mass spectrum MH⁺ 379.2.

4-(3-chloro-4-(2-pyridylmethoxy)anilino)-5-hydroxyquinazoline was reacted with 1-bromo-2-chloroethane using analogous conditions to the reaction described in Example 11, preparation of starting materials to give 4-(3-Chloro-4-(2-pyridylmethoxy)anilino)-5-(2-chloroethoxy)quinazoline as a solid in 48% yield; NMR spectrum (DMSO-d6) 4.28 (t, 2H), 4.60 (t, 2H) 5.29 (s, 2H), 7.18 (d, 1H), 7.27 (d, 1H), 7.38 (m, 2H), 7.60 (d, 1H), 7.65 (dd, 1H), 7.75 (t, 1H), 7.88 (m, 1H), 8.14 (d, 1H), 8.55 (s, 1H), 8.59 (d, 1H), 9.96 (s, 1H); Mass spectrum MH⁺ 441.2.

### Example 15 4-(3-Chloro-4-(2-pyridylmethoxy)anilino)- 5-(3-(N-(2-hydroxyethyl)-N-methylamino)ethoxy)quinazoline trifluoroacetate

The procedure described in Example 11 was repeated using 4-(3-chloro-4-(2-pyridylmethoxy)anilino)-5-(2-chloroethoxy)quinazoline (obtained as described in Example 14) and N-methylethanolamine to give the title compound in 64% yield; NMR spectrum (DMSO-d6) 2.97 (s, 3H), 3.34 (s, 2H), 3.74 (s, 2H), 3.82 (s, 2H), 4.81 (s, 2H), 5.34 (s, 2H), 7.32 (d, 1H), 7.38 (m, 2H), 7.44 (d, 1H), 7.53 (d, 1H), 7.59 (d, 1H), 7.85 (m, 2H), 7.94 (t, 1H), 8.60 (d, 1H), 8.75 (s, 1H), 10.23 (s, 1H); Mass spectrum MH⁺ 480.5.

### Example 16 4-(3-Chloro-4-(3-fluorobenzyloxy)anilino)-5-(2-dimethylamino-2-methylethoxy)quinazoline trifluoroacetate

Macroporous triethylammonium methylpolystyrene trisacetoxyborohydride (2.14 mmol/g; 224 mg), 2M dimethylamine in THF (0.9 ml) and 3A molecular sieves (250 mg) were added to a solution of 4-(3-chloro-4-(3-fluorobenzyloxy)anilino)-5-(acetonyloxy)quinazoline (54 mg) in THF (8 ml) and the mixture was heated at 60°C overnight. A further quantity of 2M dimethylamine in THF (0.9 ml) was added and heating was continued for a further 24 hours. Insoluble material was removed by filtration and the filtrate was concentrated. The residue was purified by preparative HPLC and the appropriate fractions were combined and concentrated to give the title compound as a yellow gum (10 mg, 19%); NMR spectrum (DMSO-d6) 1.41 (d, 3H), 2.85 (s, 6H), 3.06 (m, 1H), 4.56 (m, 1H), 4.80 (m, 1H), 5.34 (s, 2H), 7.21 (m, 1H), 7.32 (m, 3H), 7.43 (m, 3H), 7.51 (dd, 1H), 7.90 (s, 1H), 7.95 (t, 1H), 8.75 (s, 1H), 10.12 (s, 1H); Mass spectrum MH⁺ 481.5.

The 4-(3-chloro-4-(3-fluorobenzyloxy)anilino)-5-(acetonyloxy)quinazoline used as starting material was obtained as follows:
A mixture of 4-(3-chloro-4-(3-fluorobenzyloxy)anilino)-5-hydroxyquinazoline (371 mg) (obtained as described in Example 11, preparation of starting materials), chloroacetone (0.197 ml) and cesium carbonate (92 mg) in acetonitrile (5 ml) was heated at 80°C for 2 hours. Volatile material was removed by evaporation and the residue was partitioned between ethyl acetate (15 ml) and water (15 ml). The organic phase was separated, washed with water (15 ml) and saturated brine (15 ml) and dried. The solution was concentrated to give 4-(3-Chloro-4-(3-fluorobenzyloxy)anilino)-5-(acetonyloxy)quinazoline as a yellow gum (54 mg) which was used without purification or characterisation.

### Example 17 4-(3-Chloro-4-(2-pyridylmethoxy)anilino)-5-(2-dimethylamino-2-methylethoxy)quinazoline

Macroporous triethylammonium methylpolystyrene trisacetoxyborohydride (2.14 mmol/g; 1.65 g), 2M dimethylamine in THF (5.65 ml) and 3A molecular sieves (500 mg) were added to a solution of 4-(3-chloro-4-(2-pyridylmethoxy)anilino)-5-(acetonyloxy)quinazoline (489 mg) in THF (20 ml) and the mixture was heated at 60°C overnight. Further quantities of macroporous triethylammonium methylpolystyrene trisacetoxyborohydride (1.65 g) and 2M dimethylamine in THF (5.65 ml) was added and heating was continued for a further 24 hours. Insoluble material was removed by filtration and the filtrate was concentrated. The residue was purified by preparative HPLC and the appropriate fractions were combined and concentrated. The residue was dissolved in water (10 ml), and the solution was neutralised with saturated sodium bicarbonate and the mixture was extracted with DCM (2 x 20 ml). The organic phase was separated, dried and concentrated to give the title compound as a glassy yellow solid (63 mg, 12%); NMR spectrum (DMSO-d6) 1.15 (d, 3H), 2.36 (s, 6H), 3.29 (m, 1H), 4.20 (t, 1H), 4.48 (dd, 1H), 5.40 (s, 2H), 7.26 (d, 1H), 7.41 (d, 1H), 7.49 (m, 2H), 7.70 (d, 1H), 7.83 (d, 1H), 7.88 (m, 1H), 7.98 (m, 1H), 8.06 (d, 1H), 8.62 (s, 1H), 8.71 (d, 1H); Mass spectrum MH⁺ 464.4.

The 4-(3-chloro-4-(2-pyridylmethoxy)anilino)-5-(acetonyloxy)quinazoline used as starting material was obtained in 93% yield by an analogous procedure to that described in Example 16 (preparation of starting materials), but starting from 4-(3-chloro-4-(2-pyridylmethoxy)anilino)-5-hydroxyquinazoline (obtained as described in Example 14); Mass spectrum MH⁺ 435.4.

### Example 18 N-[3-Chloro-4-(1,3-thiazol-4-ylmethoxy)phenyl]-5-[2-(dimethylamino)ethoxy]quinazolin-4-amine

Potassium carbonate (138 mg, 1.00 mmol) and 18-crown-6 (10 mg) were added to a solution of 2-chloro-4-({5-[2-(dimethylamino)ethoxy]quinazolin-4-yl}amino)phenol (60 mg, 0.17 mmol) in DMA (5 ml). The mixture was briefly sonicated, and a solution of 4-(chloromethyl)-thiazole hydrochloride (36 mg, 0.21 mmol) in DMA (2 ml) was added dropwise. The mixture was heated to 50°C for 16 hours. The solvent was removed *in vacuo*, and the residue was partitioned between DCM (15 ml) and water (15 ml). The DCM layer was loaded onto a silica column; the column was eluted with 2 to 4% (10:1 MeOH / conc. NH_{3(aq)}) in DCM. Evaporation of the appropriate fractions followed by crystallisation from ethyl acetate / *iso*-hexane gave the title compound as a white crystalline solid (55 mg, 71 % yield); NMR spectrum (DMSO-d6); 2.28 (s, 6H), 2.81 (t, 2H), 4.37 (t, 2H), 5.33 (s, 2H), 7.16 (d, 1H), 7.35 (d, 1H), 7.37 (d, 1H), 7.74 (dd, 1H), 7.82 (d, 1H), 7.86 (dd, 1H), 7.97 (d, 1H), 8.53 (s, 1H), 9.15 (d, 1H), 10.50 (s, 1H); Mass spectrum MH⁺ 456.

The 2-chloro-4-({5-[2-(dimethylamino)ethoxy]quinazolin-4-yl}amino)phenol used as starting material was obtained as follows:

4-Chloro-5-fluoroquinazoline (obtained as described in Example 1, preparation of starting materials, 10.00 g, 54.8 mmol) was added to a solution of 4-amino-2-chlorophenol (8.65 g, 60.3 mmol) in *iso*-propanol (200 ml). The mixture was heated to reflux for 2 hours, and then cooled to room temperature. The resulting yellow solid was collected by filtration, and washed with cold *iso*-propanol (2 x 100 ml). The solid was dissolved in a boiling 5:1 mixture of methanol and water (700 ml). Concentrated aqueous ammonia solution (20 ml) was added to the hot solution with vigorous stirring, causing a pale pink solid to precipitate. The mixture was concentrated *in vacuo* to a volume such that all of the methanol had been removed, leaving the crude product as a suspension in aqueous solution. Water (200 ml) was added, and the suspension stood for 16 hours. The solid was collected by filtration, and washed with water (2 x 200 ml). Trituration with hot ethyl acetate gave 2-chloro-4-[(5-fluoroquinazolin-4-yl)amino]phenol as a pale pink solid (13.5 g, 85% yield); NMR spectrum (DMSO-d6); 6.97 (d, 1H), 7.39 (dd, 1H), 7.42 (dd, 1H), 7.59 (d, 1H), 7.73 (d, 1H), 7.81 (ddd, 1H), 8.51 (s, 1H), 9.03 (d, 1H), 10.07 (br. s, 1H); Mass spectrum MH⁺ 290.

*N*,*N*-Dimethylethanolamine (2.61 ml, 26.0 mmol) was added dropwise under nitrogen to a suspension of 60% sodium hydride dispersion (1.04 g, 26 mmol) in anhydrous DMA (75 ml). The mixture was stirred under an atmosphere of nitrogen for 30 minutes until effervescence had ceased. 2-Chloro-4-[(5-fluoroquinazolin-4-yl)amino]phenol (2.90 g, 10.00 mmol) was added, and the mixture heated under an atmosphere of nitrogen to 110°C for 2 hours. The mixture was cooled to ambient temperature, and saturated ammonium chloride solution (10 ml) was added. The mixture was concentrated *in vacuo*, and the residue shaken with a mixture of saturated sodium hydrogen carbonate solution (100 ml) and DCM (100 ml). The resulting precipitate was collected by filtration; the solid was combined with the organic component of the filtrate and evaporated to dryness. The residue was dried at 60°C at 1 mbar pressure for 16 hours. Trituration of the solid with hot ethyl acetate gave the title compound as a pale yellow solid (3.01 g, 84 %); NMR spectrum (DMSO-d6); 2.27 (s, 6H), 2.80 (t, 2H), 4.35 (t, 2H), 6.99 (d, 1H), 7.13 (d, 1H), 7.32 (d, 1H), 7.65 (dd, 1H), 7.72 (dd, 1H), 7.85 (d, 1H), 8.49 (s, 1H), 9.99 (s, 1H), 10.41 (s, 1H); Mass spectrum MH⁺ 359.

### Example 19 N-[3-Chloro-4-(pyridin-2-yloxy)phenyl]-5-[2-(dimethylamino)ethoxy]quinazolin-4-amine

A mixture of 2-chloro-4-({5-[2-(dimethylamino)ethoxy]quinazolin-4-yl}amino)phenol (obtained as described in Example 1, preparation of starting materials, 71 mg, 0.20 mmol), 2-bromopyridine (21 µl, 0.22 mmol), (9,9-dimethyl-9H-xanthene-4,5-diyl)bis(diphenylphosphine) (4 mg, 0.0066 mmol), cesium carbonate (112 mg, 0.35 mmol) and bis(dibenzylideneacetone)palladium (1.3 mg, 0.0022 mmol) in 1,4-dioxane was placed into a 10 ml pressure vial. The vial was capped, and irradiated in a CEM Explorer™ microwave synthesisor at 150°C for 20 minutes. The mixture was concentrated *in vacuo*, and the residue partitioned between DCM (15 ml) and water (15 ml). The aqueous layer was extracted with DCM (15 ml), and the extractions combined with the organic layer. The combined organic fractions were loaded onto a silica column; the column was eluted with 2 to 3% (10:1 MeOH / conc. NH_{3(aq)}) in DCM. Evaporation of the appropriate fractions followed by crystallisation from ethyl acetate / *iso*-hexane gave the title compound as a white crystalline solid (60 mg, 70% yield); NMR spectrum (DMSO-d6); 2.31 (s, 6H), 2.83 (t, 2H), 4.40 (t, 2H), 7.11 (d, 1H), 7.14 (dd, 1H), 7.19 (d, 1H), 7.35 (d, 1H), 7.38 (d, 1H), 7.77 (dd, 1H), 7.88 (ddd, 1H), 7.98 (dd, 1H), 8.13 (dd, 1H), 8.15 (d, 1H), 8.59 (s, 1H), 10.62 (s, 1H); Mass spectrum MH⁺ 437.

### Example 20 N-[3-Chloro-4-(pyrazin-2-ylmethoxy)phenyl]-5-[(1S)-2-(dimethylamino)-1-methylethoxy]quinazolin-4-amine

Methanesulphonyl chloride (171 µl, 2.21 mmol) was added dropwise to a solution of 2-(hydroxymethyl)pyrazine (221 mg, 2.01 mmol) and *N*,*N*-di*iso*-propylethylamine (385 µl, 2.21 mmol) in DCM (10 ml). The mixture was heated to 40°C for 1 hour. The reaction mixture was concentrated *in vacuo* and the residue was dissolved in dry DMA (5 ml). This solution was added to a mixture of 2-chloro-4-({5-[(1*S*)-2-(dimethylamino)-1-methylethoxy]quinazolin-4-yl}amino)phenol (0.5 g, 1.34 mmol), potassium carbonate (0.93 g, 6.7 mmol) and 18-crown-6 (20 mg) in dry DMA (20 ml). The reaction mixture was stirred at ambient temperature overnight. Water (500 ml) was added to the mixture and the resultant precipitate was filtered. This was crystallised from ethyl acetate to give two batches of crystals with a combined weight of 261 mg (42%); NMR spectrum (CDCl₃); 1.46 (d, 3H), 2.23 (s, 6H), 2.40 (dd, 1H), 2.86 (dd, 1H), 4.61-4.74 (m, 1H) 5.25 (s, 2H), 6.85 (d, 1H), 6.98 (d, 1H), 7.37 (d, 1H), 7.55 (t, 1H), 7.68 (d, 1H), 7.73 (s, 1H); 8.50 (s, 2H), 8.54 (s, 1H), 8.92 (s, 1H), 10.32 (s, 1H); Mass spectrum MH⁺ 465.

The 2-chloro-4-({5-[(1*S*)-2-(dimethylamino)-1-methylethoxy]quinazolin-4-yl}amino)phenol used as starting material was obtained as follows: (*S*)-1-*N*,*N*'-Dimethylamino-2-propanol (1.19 g, 9.69 mmol) was added slowly to a suspension of sodium hydride (60% dispersion in mineral oil 388 mg, 9.69 mmol) in dry DMA (20 ml). Upon complete addition, the mixture was stirred for 30 minutes. 2-Chloro-4-[(5-fluoroquinazolin-4-yl)amino]phenol (obtained as described in Example 1, preparation of starting materials, 1.12 g, 3.88 mmol) was added in one portion and the mixture was heated at 110°C for 2 hours. Saturated ammonium chloride solution (5 ml) was added to the cool reaction mixture and stirred for 10 minutes then the DMA was removed *in vacuo*. The residue was partitioned between water (100 ml) and ethyl acetate (100 ml). The ethyl acetate was dried (MgSO₄), pre-absorbed onto silica and chromatographed eluting with 1 to 10% (10:1 MeOH / conc. NH_{3(aq)}) in ethyl acetate to give the title compound as a solid (0.92 g, 64%); NMR spectrum (CDCl₃); 1.45 (d, 3H), 2.19 (s, 6H), 2.39 (dd, 1H), 2.82 (dd, 1H), 4.59-4.72 (m, 1H), 6.84 (d, 1H), 6.91 (d, 1H), 7.31-7.41 (m, 2H), 7.54 (t, 1H), 7.65 (dd, 1H), 8.51 (s, 1H), 10.34 (s, 1H); Mass spectrum MH⁺ 373.

### Example 21 N-{3-Chloro-4-[(3-fluorobenzyl)oxy]phenyl}-5-[(1S)-2-(dimethylamino)-1-methylethoxylauinazolin-4-amine

The procedure described in Example 18 was repeated using 2-chloro-4-({5-[(1*S*)-2-(dimethylamino)-1-methylethoxy]quinazolin-4-yl}amino)phenol (obtained as described in Example 20, preparation of starting materials, 120 mg, 0.32 mmol) and 3-fluorobenzyl chloride (58 mg, 0.40 mmol) to give the title compound as a gum (33 mg, 22%); NMR spectrum (CDCl₃); 1.45 (d, 3H), 2.21 (s, 6H), 2.39 (dd, 1H), 2.84 (dd, 1H), 4.59-4.72 (m, 1H), 5.08 (s, 2H), 6.80-6.99 (m, 3H), 7.10-7.21 (m, 2H), 7.23-7.39 (m, 2H), 7.53 (t, 1H), 7.61 (dd, 1H), 7.70 (d, 1H), 8.52 (s, 1H), 10.30 (s, 1H); Mass spectrum MW⁺ 481.

### Example 22 N-[3-Chloro-4-(pyridin-2-ylmethoxy)phenyl]-5-[(1R)-2-(dimethylamino)-1-methylethoxy]guinazolin-4-amine

2-Chloro-4-({5-[(1*R*)-2-(dimethylamino)-1-methylethoxy]quinazolin-4-yl}amino)phenol (65 mg, 0.176 mmol), potassium carbonate (122 mg, 0.88 mmol), picolyl chloride hydrochloride and 18-crown-6 were stirred in dry DMA (5 ml) overnight at ambient temperature. The mixture was concentrated *in vacuo* and the residue was partitioned between DCM and water. The mixture was filtered through phase-separating paper and the DCM was loaded onto a column and eluted with 2 to 4% (10:1 MeOH / conc. NH_{3(aq)}) in DCM. The required fractions were combined and concentrated to give the title compound as a gum (64 mg, 79%); NMR spectrum (CDCl₃); 1.45 (d, 3H), 2.21 (s, 6H), 2.39 (dd, 1H), 2.84(dd, 1H), 4.59-4.72 (m, 1H), 5.22 (s, 2H), 6.84 (d, 1H), 6.93 (d, 1H), 7.17 (dd, 1H), 7.35 (d, 1H), 7.50-7.62 (m, 3H), 7.63-7.71 (m, 1H), 7.74 (d, 1H), 8.52 (s, 2H), 10.30 (s, 1H); Mass spectrum MH⁺ 464.

The 2-chloro-4-({5-[(1*R*)-2-(dimethylamino)-1-methylethoxy]quinazolin-4-yl}amino)phenol used as starting material was obtained as follows:
(*R*)-1-Aminopropan-2-ol (3.4 ml, 43.25 mmol) was added slowly to a suspension of sodium hydride (60% dispersion in mineral oil 1.73 g, 43.25 mmol) in dry DMA (50 ml). Upon complete addition the mixture was stirred for 30 minutes. 2-Chloro-4-[(5-fluoroquinazolin-4-yl)amino]phenol (obtained as described in Example 1, preparation of starting materials, 5 g, 17.3 mmol) was added in one portion followed by 15-crown-5 (10 mg) and the mixture was heated at 60°C overnight. Saturated ammonium chloride solution (5 ml) was added to the cool reaction mixture and stirred for 10 minutes. The DMA was removed *in vacuo*, water (200 ml) was added to the residue and stirred vigorously for 1 hour. The resultant precipitate was filtered, washed with water (3 x 50 ml) and diethyl ether (2 x 50 ml) to give 4-({5-[(1*R*)-2-amino-1-methylethoxy]quinazolin-4-yl}amino)-2-chlorophenol as a pale green solid (5.53 g, 93%); NMR spectrum (DMSO-d6); 1.39 (d, 3H), 2.88-3.04 (m, 2H), 4.73-4.85 (m, 1H), 6.97 (d, 1H), 7.15 (d, 1H), 7.29 (d, 1H), 7.47 (dd, 1H), 7.68 (t, 1H), 8.06 (d, 1H), 8.45 (s, 1H), 10.50 (br s, 1H); Mass spectrum MH⁺ 345.

4-({5-[(1*R*)-2-Amino-1-methylethoxy]quinazolin-4-yl}amino)-2-chlorophenol (1.4 g, 4.07 mmol), formic acid (3 ml) and aqueous formaldehyde solution (0.5 ml) were heated to 90°C for 3 hours. The mixture was concentrated *in vacuo* and saturated ammonium chloride solution (10 ml) was added to the residue. This was extracted with DCM, dried (MgSO₄) and concentrated *in vacuo*. The residue was purified by chromatography eluting with 10%methanol / ethyl acetate. The solid obtained was triturated with diethyl ether to give the title compound (0.73 g, 48%); NMR spectrum (DMSO-d6); 1.45 (d, 3H), 2.20 (s, 6H), 2.39 (dd, 1H), 2.83 (dd, 1H), 4.59-4.73 (m, 1H), 6.84 (d, 1H), 6.93 (d, 1H), 7.33-7.41 (m, 2H), 7.54 (t, 1H), 7.71 (d, 1H), 8.52 (s, 1H), 10.31 (s, 1H); Mass spectrum MH⁺ 373.

### Example 23 N-[3-Chloro-4-(1,3-thiazol-4-ylmethoxy)phenyl]-5-[(1R)-2-(dimethylamino)-1-methylethoxy]quinazolin-4-amine

The procedure described in Example 22 was repeated using 2-chloro-4-({5-[(1*R*)-2-(dimethylamino)-1-methylethoxy]quinazolin-4-yl}amino)phenol (obtained as described in Example 22, preparation of starting materials, 65 mg, 0.176 mmol) and 4-(chloromethyl)-thiazole hydrochloride (45 mg, 0.264 mmol) to give the title compound as a gum (18 mg, 20%); NMR spectrum (CDCl₃); 1.45 (d, 3H), 2.21 (s, 6H), 2.39 (dd, 1H), 2.85 (dd, 1H), 4.60-4.72 (m, 1H), 5.30 (s, 2H), 6.84 (d, 1H), 6.99 (d, 1H), 7.36 (d, 1H), 7.44 (s, 1H), 7.54 (t, 1H), 7.61 (dd, 1H), 7.73 (dd, 1H), 8.53 (s, 1H), 8.77 (d, 1H), 10.31 (s, 1H); Mass spectrum MH⁺ 470.

### Example 24 N-[3-Chloro-4-(pyrazin-2-ylmethoxy)phenyl]-5-[(1R)-2-(dimethylamino)-1-methylethoxy]quinazolin4-amine

Methanesulphonyl chloride (34 µl, 0.44 mmol) was added dropwise to a solution of 2-(hydroxymethyl)pyrazine (44 mg, 0.40 mmol) and *N*,*N*-di-*iso*-propylethylamine (77 µl, 0.44 mmol) in DCM (10 ml). The mixture was heated to 40°C for 1 hour. The reaction mixture was concentrated *in vacuo* and the residue was dissolved in dry DMA (5 ml). 2-Chloro-4-({5-[(1*R*)-2-(dimethylamino)-1-methylethoxy]quinazolin-4-yl}amino)phenol (obtained as described in Example 22, preparation of starting materials, 100 mg, 0.27 mmol), potassium carbonate (187 mg, 1.35 mmol) and 1S-crown-6 (10 mg) were added to the above solution and stirred vigorously at ambient temperature overnight. The mixture was concentrated *in vacuo* and the residue was partitioned between DCM and water. The mixture was filtered through phase-separating paper and the DCM was loaded onto a column and eluted with 2 to 4% (10:1 MeOH / conc. NH_{3(aq)}) in DCM. The required fractions were combined and concentrated to give the title compound as a solid (38 mg, 30%); NMR spectrum (CDCl₃); 1.46 (d, 3H), 2.21 (s, 6H), 2.39 (dd, 1H), 2.85 (dd, 1H), 4.60-4.72 (m, 1H), 5.25 (s, 2H), 6.85 (d, 1H), 6.97 (d, 1H), 7.36 (d, 1H), 7.54 (t, 1H), 7.67 (dd, 1H), 7.72 (d, 1H), 8.49 (s, 2H), 8.53 (s, 1H), 8.91 (s, 1H), 10.53 (s, 1H); Mass spectrum MH⁺ 465.

### Example 25 N-{3-Chloro-4-[(3-fluorobenzyl)oxy]phenyl}-5-(1R)-2-(dimethylamino)-1-methylethoxy]quinazolin-4-amine

2-Chloro-4-({5-[(1*R*)-2-(dimethylamino)-1-methylethoxy]quinazolin-4-yl}amino)phenol (obtained as described in Example 22, preparation of starting materials, 150 mg, 0.40 mmol), potassium carbonate (276 mg, 2.0 mmol), 3-fluorobenzyl chloride (64 mg, 0.44 mmol) and 18-crown-6 (10 mg) were stirred in DMF (5 ml) at ambient temperature overnight. Water (10 ml) was added and the mixture was extracted with ethyl acetate (3 x 15 ml). The combined organics were dried (MgSO₄), concentrated *in vacuo* and the residue was purified by chromatography using 10% methanol / ethyl acetate as the eluent to give the title compound as a gum (69 mg, 36%); NMR spectrum (CDCl₃); 1.45 (d, 3H), 2.21 (s, 6H), 2.39 (dd, 1H), 2.84 (dd, 1H), 4.59-4.72 (m, 1H), 5.08 (s, 2H), 6.80-6.99 (m, 3H), 7.10-7.21 (m, 2H), 7.23-7.39 (m, 2H), 7.53 (t, 1H), 7.61 (dd, 1H), 7.70 (d, 1H), 8.52 (s, 1H), 10.30 (s, 1H); Mass spectrum MH⁺ 481.

### Example 26 N-[3-Chloro-4-(pyridin-2-ylmethoxy)phenyl]-5-[2-(dimethylamino)-2-methylpropoxy]quinazolin-4-amine

Potassium carbonate (200 mg, 1.45 mmol), 2-chloro-4-({5-[2-(dimethylamino)-2-methylpropoxy]quinazolin-4-yl}amino)phenol (100 mg, 0.26 mmol), and 18-crown-6 (10 mg) were slurried in DMA (5 ml) and sonicated in a sonic cleaning bath for 10 minutes. A solution of picolyl chloride hydrochloride (60 mg, 0.35 mmol) in DMA (5 ml) was added dropwise and the reaction was stirred at room temperature for 2 days. The DMA was removed *in vacuo*, water (5 ml) was added and then the suspension was extracted with DCM (2 x 5 ml). The DCM fraction was purified by chromatography using 2.5 to 5% (10:1 MeOH / conc. NH_{3(aq)}) in DCM as eluent. The appropriate fractions were evaporated, and the residue was crystallised from diethyl ether to give the title compound as a white solid (79 mg, 63%); NMR spectrum (DMSO-d6); 1.16 (s, 6H), 2.21 (s, 6H), 4.16 (s, 2H), 5.25 (s, 2H), 7.09 (d, 1H), 7.27 (d, 1H), 7.33 (d, 1H), 7.35 (dd, 1H), 7.57 (d, 1H), 7.71 (t, 1H), 7.83 (m, 2H), 7.97 (d, 1H), 8.51 (s, 1H), 8.58 (d, 1H), 10.64 (s, 1H); Mass spectrum MH⁺ 478.

The 2-chloro-4-({5-[2-(dimethylamino)-2-methylpropoxy]quinazolin-4-yl}amino)phenol used as starting material was obtained as follows:

A solution of 2-dimethylamino-2-methyl-propan-l-ol (1.2 g, 10.3 mmol) in DMA (15 ml) was added dropwise to a suspension of sodium hydride (60% dispersion in mineral oil, 0.41 g, 10.2 mmol) in DMA (5 ml) under N₂. The reaction was stirred for 30 minutes then 15-crown-5 (50 mg) was added followed by 2-chloro-4-[(5-fluoroquinazolin-4-yl)amino]phenol (obtained as described in Example 1, preparation of starting materials, 1.0 g, 3.4 mmol). The reaction was heated at 110°C for 2 hours. The reaction was cooled, quenched with saturated ammonium chloride solution, and concentrated *in vacuo*. Saturated sodium bicarbonate solution was added and the reaction mixture was extracted with DCM (x 2). Removal of DCM gave a yellow solid which was crystallised from ether to give the title compound as a pale yellow solid (0.87 g, 65%); NMR spectrum (DMSO-d6); 1.13 (s, 6H), 2.20 (s, 6H), 4.14 (s, 2H), 6.97 (d, 1H), 7.07 (d, 1H), 7.31 (d, 1H), 7.64 (dd, 1H), 7.70 (t, 1H), 7.81 (d, 1H), 8.46 (s, 1H), 9.98 (s, 1H), 10.57 (s, 1H); Mass spectrum MH⁺ 387.

### Example 27 N-[3-Chloro-4-(1,3-thiazol-4-ylmethoxy)phenyl]-5-[2-(dimethylamino)-2-methylpropoxy]quinazolin-4-amine

The procedure described in Example 26 was repeated using 2-chloro-4-({5-[2-(dimethylamino)-2-methylpropoxy]quinazolin-4-yl}amino)phenol (obtained as described in Example 26, preparation of starting materials) and 4-(chloromethyl)-thiazole hydrochloride to give the title compound as white crystals in 71% yield; NMR spectrum (DMSO-d6); 1.17 (s, 6H), 2.22 (s, 6H), 4.17 (s, 2H), 5.31 (s, 2H), 7.10 (d, 1H), 7.35 (d, 1H), 7.37 (d, 1H), 7.73 (t, 1H), 7.80 (d, 1H), 7.85 (dd, 1H), 7.94 (d, 1H), 8.52 (s, 1H), 9.14 (d, 1H), 10.64 (s, 1H); Mass spectrum MH⁺ 484.

### Example 28 N-{3-Chloro-4-[(5-methylisoxazol-3-yl)methoxy]phenyl}-5-[2-(dimethylamino)-2-methylpropoxy]quinazolin-4-amine

The procedure described in Example 26 was repeated using 2-chloro-4-({5-[2-(dimethylamino)-2-methylpropoxy]quinazolin-4-yl}amino)phenol (obtained as described in Example 26, preparation of starting materials) and 3-(chloromethyl)-5-methylisoxazole to give the title compound as white crystals in 63% yield; NMR spectrum (DMSO-d6); 1.15 (s, 6H), 2.21 (s, 6H), 2.41 (s, 3H), 4.16 (s, 2H), 5.23 (s, 2H), 6.34 (1H, s), 7.08 (d, 1H), 7.31 (d, 1H), 7.35 (d, 1H), 7.72 (t, 1H), 7.85 (dd, 1H), 7.86 (d, 1H), 8.53 (s, 1H), 10.64 (s, 1H); Mass spectrum MH⁺ 482.

### Example 29 5-[2-(Dimethylamino)ethoxy]-N-[3-methyl-4-(pyridin-2-ylmethoxy)phenyl]quinazolin-4-amine

Potassium carbonate (150 mg, 1.08 mmol), 4-({5-[2-(dimethylamino)ethoxy]quinazolin-4-yl}amino)-2-methylphenol (68 mg, 0.20 mmol), and 18-crown-6 (10 mg) were slurried in DMA (5 ml) and sonicated in a sonic cleaning bath for 10 minutes. A solution of picolyl chloride hydrochloride (43 mg, 0.26 mmol) in DMA (5 ml) was added dropwise and the reaction was stirred at room temperature for 2 days. The DMA was removed *in vacuo*, water (5 ml) was added and then the suspension was extracted with DCM (2 x 5 ml). The DCM fraction was purified by chromatography using 2.5 to 5% (10:1 MeOH / conc. NH_{3(aq)}) in DCM as eluent. The appropriate fractions were evaporated, and the residue was crystallised from ethyl acetate / diethyl ether to give the title compound as a light yellow solid (37 mg, 43%); NMR spectrum (DMSO-d6); 2.14 (s, 6H), 2.16 (s, 3H), 2.79 (t, 2H), 4.33 (t, 2H), 5.19 (s, 2H), 7.01 (d, 1H), 7.11 (d, 1H), 7.30 (d, 1H), 7.34 (t, 1H), 7.50 (s, 1H), 7.55 (d, 1H), 7.70 (m, 2H), 7.84 (t, 1H), 8.42 (s, 1H), 8.57 (d, 1H), 10.36 (s, 1H); Mass spectrum MH⁺ 430.

The 4-({5-[2-(dimethylamino)ethoxy]quinazolin-4-yl}amino)-2-methylphenol used as starting material was obtained as follows:

4-Chloro-5-fluoroquinazoline (obtained as described in Example 1, preparation of starting materials, 6.76 g, 37.0 mmol) was dissolved in *iso*-propanol (200 ml) and 4-amino-2-methylphenol (5.00 g, 40.7 mmol) was added. The mixture was heated under reflux for 2 hours, causing a yellow solid to precipitate. The mixture was cooled to ambient temperature; the solid was collected by filtration. The solid was dissolved in a boiling mixture of methanol (500 ml) and water (100 ml) to give a brown solution. With vigorous stirring, the solution was basified with aqueous ammonia (0.880, 10 ml), causing a light brown solid to precipitate. The mixture was concentrated *in vacuo* to such a volume that all of the methanol had been removed, leaving the product as a suspension in aqueous solution. The suspension was cooled; the solid was collected by filtration, triturated with ethyl acetate and dried over P₂O₅ in a vacuum oven to give 2-methyl-4-[(5-fluoroquinazolin-4-yl)amino]phenol as a light brown solid (8.18 g, 82%); NMR spectrum (DMSO-d6) 3.30 (s, 3H), 6.78 (d, 1H), 7.28 (m, 2H), 7.38 (dd, 1H), 7.57 (d, 1H), 7.78 (m, 1H), 8.43 (s, 1H), 8.88 (d, 1H), 9.22 (s, 1H); Mass spectrum MH⁺ 270.

A solution of *N*,*N*-dimethylethanolamine (1.23 g, 13.8 mmol) in DMA (12 ml) was added dropwise to a suspension of sodium hydride (60% dispersion in mineral oil, 0.55 g, 13.8 mmol) in DMA (25 ml) under N₂. The reaction was stirred for 30 minutes then 15-crown-5 (50 mg) was added followed by 2-methyl-4-[(5-fluoroquinazolin-4-yl)amino]phenol (1.0 g, 3.75 mmol). The reaction was heated at 110°C for 2 hours. The reaction was cooled, quenched with saturated ammonium chloride solution, and concentrated *in vacuo*. Saturated sodium bicarbonate solution was added causing precipitation of a yellow solid which was collected by filtration and crystallised from ethyl acetate to give the title compound as a white solid (0.66 g, 52%); NMR spectrum (DMSO-d6); 2.14 (s, 3H), 2.25 (s, 6H), 2.77 (t, 2H), 4.31 (t, 2H), 6.76 (d, 1H), 7.07 (d, 1H), 7.28 (d, 1H), 7.37 (d, 1H), 7.50 (dd, 1H), 7.66 (t, 1H), 8.40 (s, 1H), 9.15 (s, 1H), 10.25 (s, 1H); Mass spectrum MH⁺ 339.

### Example 30 5-[2-(Dimethylamino)ethoxy]-N-[3-methyl-4-(1,3-thiazol-4-ylmethoxy)phenyl]quinazolin-4-amine

The procedure described in Example 29 was repeated using 4-({5-[2-(dimethylamino)ethoxy]quinazolin-4-yl}amino)-2-methylphenol (obtained as described in Example 29, preparation of starting materials) and 4-(chloromethyl)-thiazole hydrochloride to give the title compound as white crystals in 47% yield; NMR spectrum (DMSO-d6); 2.21 (s, 3H), 2.24 (s, 6H), 2.79 (t, 2H), 4.36 (t, 2H), 5.23 (s, 2H), 7.10 (d, 1H), 7.12 (d, 1H), 7.27 (d, 1H), 7.47 (d, 1H), 7.68 (t, 1H), 7.71 (dd, 1H), 7.76 (d, 1H), 8.44 (s, 1H), 9.15 (d, 1H), 10.36 (s, 1H); Mass spectrum MH⁺ 436.

### Example 31 5-[2-(Dimethylamino)ethoxy]-N-{3-methyl-4-[(5-methylisoxazol-3-yl)methoxy]phenyl}quinazolin-4-amine

The procedure described in Example 29 was repeated using 4-({5-[2-(dimethylamino)ethoxy]quinazolin-4-yl}amino)-2-methylphenol (obtained as described in Example 29, preparation of starting materials) and 3-(chloromethyl)-5-methylisoxazole to give the title compound as white crystals in 61% yield; NMR spectrum (DMSO-d6); 2.19 (s, 3H), 2.23 (s, 6H), 2.41 (s, 3H), 2.78 (t, 2H), 4.35 (t, 2H), 5.14 (s, 2H), 6.35 (s, 1H), 7.05 (d, 1H), 7.11 (d, 1H), 7.28 (d, 1H), 7.48 (d, 1H), 7.70 (m, 2H), 8.43 (s, 1H), 10.36 (s, 1H); Mass spectrum MH⁺ 434.

### Example 32 5-[(1R)-2-(Dimethylamino)-1-methylethoxyl]-N-[3-methyl-4-(pyridin-2-ylmethoxy)phenyl]quinazolin-4-amine

Potassium carbonate (140 mg, 1.0 mmol), 4-({5-[(1*R*)-2-(dimethylamino)-1-methylethoxy]quinazolin-4-yl}amino)-2-methylphenol (60 mg, 0.17 mmol), and 18-crown-6 (10 mg) were slurried in DMA (5 ml) and sonicated in a sonic cleaning bath for 10 minutes. A solution of picolyl chloride hydrochloride (40 mg, 0.24 mmol) in DMA (5 ml) was added dropwise and the reaction was stirred at room temperature for 2 days. The DMA was removed *in vacuo*, water (5 ml) was added and then the suspension was extracted with DCM (2 x 5 ml). The DCM fraction was purified by chromatography using 2.5 to 5% (10:1 MeOH / conc. NH_{3(aq)}) in DCM as eluent. The appropriate fractions were evaporated to give the title compound as a clear gum (28 mg, 37%); NMR spectrum (DMSO-d6); 1.43 (d, 3H), 2.19 (s, 6H), 2.25 (s, 3H), 2.42 (dd, 1H), 2.90 (dd, 1H), 4.85 (m, 1H), 5.19 (s, 2H), 7.01 (d, 1H), 7.17 (d, 1H), 7.26 (d, 1H), 7.35 (dd, 1H), 7.46 (d, 1H), 7.56 (m, 2H), 7.66 (t, 1H), 7.84 (td, 1H), 8.41 (s, 1H), 8.57 (d, 1H), 10.32 (s, 1H); Mass spectrum MH⁺ 444.

The 4-({5-[(1*R*)-2-(dimethylamino)-1-methylethoxy]quinazolin-4-yl}amino)-2-methylphenol used as starting material was obtained as follows:
A solution of (*R*)-(-)-1-amino-propan-2-ol (0.90 g, 12.0 mmol) in DMA (10 ml) was added dropwise to a suspension of sodium hydride (60% dispersion in mineral oil, 0.48 g, 12.0 mmol) in DMA (15 ml) under N₂. The reaction was stirred for 30 minutes then 15-crown-5 (50 mg) was added followed by 2-methyl-4-[(5-fluoroquinazolin-4-yl)amino]phenol (obtained as described in Example 29, preparation of starting materials, 1.00 g, 3.72 mmol). The reaction was heated at 110°C for 3 hours. The reaction was cooled, quenched with saturated ammonium chloride solution, and concentrated *in vacuo*. Saturated sodium bicarbonate solution was added and the reaction mixture was extracted with DCM (x 2). Removal of DCM gave a brown oil which was crystallised from ether to give 4-({5-[(1*R*)-2-amino-1-methylethoxy]quinazolin-4-yl}amino)-2-methylphenol as a brown solid (0.47 g, 39%); NMR spectrum (DMSO-d6); 1.39 (d, 3H), 2.14 (s, 3H), 2.94 (m, 2H), 4.77 (m, 1H), 6.77 (d, 1H), 7.12 (d, 1H), 7.25 (d, 1H), 7.43 (dd, 1H), 7.53 (d, 1H), 7.65 (t, 1H), 8.39 (s, 1H), 9.19 (s, 1H), 10.34 (s, 1H); Mass spectrum MH⁺ 325.

4-({5-[(1*R*)-2-Amino-1-methylethoxy]quinazolin-4-yl}amino)-2-methylphenol (0.40 g, 1.23 mmol) was dissolved in a mixture of formic acid (5 ml) and formaldehyde solution containing 10-15% MeOH (2 ml) and heated to 100°C for 3 hours. The resultant yellow solution was diluted with water (15 ml) and ammonia solution (0.880) was added until the solution was basic. The mixture was extracted with ethyl acetate (x 3) and then purified by chromatography using 2.5 to 5% (10:1 MeOH / conc. NH_{3(aq)}) in DCM as eluent. The appropriate fractions were evaporated to give the title compound as a brown foam (0.24 g, 55%); NMR spectrum (DMSO-d6); 1.43 (d, 3H), 2.15 (s, 3H), 2.18 (s, 6H), 2.41 (dd, 1H), 2.87 (dd, 1H), 4.84 (m, 1H), 6.77 (d, 1H), 7.17 (d, 1H), 7.25 (d, 1H), 7.34 (d, 1H), 7.41 (dd, 1H), 7.67 (t, 1H), 8.38 (s, 1H), 9.16 (s, 1H), 10.24 (s, 1H); Mass spectrum MH⁺ 353.

### Example 33 5-[(1R)-2-(Dimethylamino)-1-methylethoxy]-N-[3-methyl-4-(pyrazin-2-ylmethoxy)phenyl]quinazolin-4-amine

The procedure described in Example 32 was repeated using 4-({5-[(1*R*)-2-(dimethylamino)-1-methylethoxy]quinazolin-4-yl}amino)-2-methylphenol (obtained as described in Example 32, preparation of starting materials) and pyrazin-2-ylmethyl methanesulfonate to give the title compound as white crystals in 48% yield; NMR spectrum (DMSO-d6); 1.42 (d, 3H), 2.17 (s, 6H), 2.24 (s, 3H), 2.42 (dd, 1H), 2.90 (dd, 1H), 4.86 (m, 1H), 5.27 (s, 2H), 7.07 (d, 1H), 7.16 (d, 1H), 7.27 (d, 1H), 7.46 (d, 1H), 7.61 (dd, 1H), 7.66 (t, 1H), 8.41 (s, 1H), 8.63 (d, 1H), 8.66 (d, 1H), 8.83 (s, 1H), 10.34 (s, 1H); Mass spectrum MH⁺ 445.

### Example 34 5-[(1R)-2-(dimethylamino)-1-methylethoxy]-N-[3-methyl-4-(1,3-thiazol-4-yimethoxy)phenyl]quinazolin-4-amine

The procedure described in Example 32 was repeated using 4-({5-[(1*R*)-2-(dimethylamino)-1-methylethoxy]quinazolin-4-yl}amino)-2-methylphenol (obtained as described in Example 32, preparation of starting materials) and 4-(chloromethyl)-thiazole hydrochloride to give the title compound as a clear gum in 33% yield; NMR spectrum (DMSO-d6); 1.43 (d, 3H), 2.19 (s, 6H), 2.22 (s, 3H), 2.42 (dd, 1H), 2.90 (dd, 1H), 4.86 (m, 1H), 5.23 (s, 2H), 7.10 (d, 1H), 7.15 (d,1H), 7.27 (d, 1H), 7.42 (d, 1H), 7.60 (dd, 1H), 7.67 (t, 1H), 7.77 (d, 1H), 8.42 (s, 1H), 9.14 (d, 1H), 10.32 (s, 1H); Mass spectrum MH⁺ 450.

### Example 35 5-[(1R)-2-(Dimethylamino)-1-methylethoxy]-N-{3-methyl-4-[(5-methylisoxazol-3-yl)methoxy]phenyl}quinazolin-4-amine

The procedure described in Example 32 was repeated using 4-({5-[(1*R*)-2-(dimethylamino)-1-methylethoxy]quinazolin-4-yl}amino)-2-methylphenol (obtained as described in Example 32, preparation of starting materials) and 3-(chloromethyl)-5-methylisoxazole to give the title compound as a clear gum in 62% yield; NMR spectrum (DMSO-d6); 1.43 (d, 3H), 2.19 (s, 6H), 2.20 (s, 3H), 2.41 (s, 3H), 2.43 (dd, 1H), 2.91 (dd, 1H), 4.85 (m, 1H), 5.16 (s, 2H), 6.36 (s, 1H), 7.07 (d, 1H), 7.15 (d, 1H), 7.26 (d, 1H), 7.45 (d, 1H), 7.60 (dd, 1H), 7.66 (t, 1H), 8.41 (s, 1H), 10.33 (s, 1H); Mass spectrum MH⁺ 448.

### Example 36 5-[2-(dimethylamino)-2-methylpropoxy]-N-[3-methyl-4-(1,3-thiazol-4-ylmethoxyl)phenyl]quinazolin-4-amine

Potassium carbonate (100 mg, 0.72 mmol), 4-({5-[2-(dimethylamino)-2-methylpropoxy]quinazolin-4-yl}amino)-2-methylphenol (50 mg, 0.14 mmol), and 18-crown-6 (10 mg) were slurried in DMA (5 ml) and sonicated in a sonic cleaning bath for 10 minutes. A solution of 4-(chloromethyl)-thiazole hydrochloride (30 mg, 0.18 mmol) in DMA (5 ml) was added dropwise and the reaction was stirred at room temperature for 2 days. The DMA was removed *in vacuo*, water (5 ml) was added and then the suspension was extracted with DCM (2 x 5 ml). The DCM fraction was purified by chromatography using 2.5 to 5% (10:1 MeOH / conc. NH_{3(aq)}) in DCM as eluent. The appropriate fractions were evaporated, and the residue was crystallised from diethyl ether to give the title compound as a white solid (13 mg, 22%); NMR spectrum (DMSO-d6); 1.14 (s, 6H), 2.19 (s, 6H), 2.21 (s, 3H), 4.13 (s, 2H), 5.23 (s, 2H), 7.07 (d, 1H), 7.12 (d, 1H), 7.30 (d, 1H), 7.47 (d, 1H), 7.68 (t, 1H), 7.72 (dd, 1H), 7.88 (d, 1H), 8.43 (s, 1H), 9.13 (d, 1H), 10.54 (s, 1H); Mass spectrum MH⁺ 464.

The 4-({5-[2-(dimethylamino)-2-methylpropoxy]quinazolin-4-yl}amino)-2-methylphenol used as starting material was obtained as follows:

A solution of 2-dimethylamino-2-methyl-propan-1-ol (0.33 g, 2.82 mmol) in DMA (3 ml) was added dropwise to a suspension of sodium hydride (60% dispersion in mineral oil, 0.15 g, 3.75 mmol) in DMA (5 ml) under N₂. The reaction was stirred for 30 minutes then 15-crown-5 (50 mg) was added followed by 2-methyl-4-[(5-fluoroquinazolin-4-yl)amino]phenol (obtained as described in Example 29, preparation of starting materials, 0.25 g, 0.93 mmol). The reaction was heated at 110°C for 15 hours. The reaction was cooled, quenched with saturated ammonium chloride solution, and concentrated *in vacuo*. Saturated sodium bicarbonate solution was added and the reaction mixture was extracted with DCM (x2). The DCM fraction was purified by chromatography using 4 to 7% (10:1 MeOH / conc. NH_{3(aq)}) in DCM as eluent. The appropriate fractions were evaporated, and the residue was crystallised from ethyl acetate / ether to give the title compound as a yellow solid (0.21 g, 60%); NMR spectrum (DMSO-d6); 1.14 (s, 6H), 2.15 (s, 3H), 2.19 (s, 6H), 4.13 (s, 2H), 6.76 (d, 1H), 7.03 (d, 1H), 7.25 (d, 1H), 7.37 (d, 1H), 7.49 (dd, 1H), 7.66 (t, 1H), 8.40 (s, 1H), 9.17 (s, 1H), 10.46 (s, 1H); Mass spectrum MH⁺ 367.

### Example 37 5-[2-(Dimethylamino)ethoxy]-N-{3-methoxy-4-[(5-methylisoxazol-3-yl)methoxy]phenyl}quinazolin-4-amine

Potassium carbonate (138 mg, 1.00 mmol) and 18-crown-6 (10 mg) were added to a solution of 4-({5-[2-(dimethylamino)ethoxy]quinazolin-4-yl}amino)-2-methoxyphenol (60 mg, 0.17 mmol) in DMA (5 ml). The mixture was briefly sonicated, and a solution of 3-(chloromethyl)-5-methylisoxazole (30 mg, 0.21 mmol) in DMA (2 ml) was added dropwise.
The mixture was heated to 50°C for 16 hours. The solvent was removed *in vacuo*, and the residue was partitioned between DCM (15 ml) and water (15 ml). The DCM layer was loaded onto a silica column; the column was eluted with 2 to 4% (10:1 MeOH / conc. NH_{3(aq)}) in DCM. Evaporation of the appropriate fractions followed by crystallisation from methyl *tert-*butyl ether gave the title compound as a white crystalline solid (38 mg, 50% yield). NMR spectrum (DMSO-d6); 2.25 (s, 6H), 2.43 (s, 3H), 2.80 (t, 2H), 3.81 (s, 3H), 4.37 (t, 2H), 5.12 (s, 2H), 6.34 (s, 1H), 7.09 (d, 1H), 7.15 (d, 1H), 7.21 (dd, 1H), 7.33 (d, 1H), 7.60 (d, 1H), 7.72 (dd, 1H), 8.47 (s, 1H), 10.38 (s, 1H); Mass spectrum MH⁺ 451.

The 4-({5-[2-(Dimethylamino)ethoxy]quinazolin-4-yl}amino)-2-methoxyphenol used as starting material was obtained as follows:

2-Methoxy-4-nitrophenol (1.00 g, 5.92 mmol) was dissolved in methanol (50 ml). The solution was degassed and purged with nitrogen. 10% Platinum on activated carbon (250 mg) was added, and the mixture degassed and purged with hydrogen. The mixture was stirred under a hydrogen atmosphere at atmospheric pressure for 1 hour. The mixture was degassed and purged with nitrogen, and the catalyst removed by filtration. The filtrate was evaporated to dryness to give 4-amino-2-methoxyphenol as a pale pink solid (710 mg).

4-Chloro-5-fluoroquinazoline (obtained as described in Example 1, preparation of starting materials, 800 mg, 4.38 mmol) was added to a solution of 4-amino-2-methoxyphenol (670 mg, 4.82 mmol) in *iso*-propanol (30 ml). The mixture was heated to reflux for 1 hour, and then cooled to room temperature. The resulting yellow solid was collected by filtration, and washed with cold *iso*-propanol (2 x 20 ml). The solid was partitioned between DCM (50 ml) and saturated sodium hydrogen carbonate solution (50 ml). The aqueous layer was extracted with DCM (50 ml), and the extractions combined with the organic layer. The combined DCM fractions were filtered through a silicone-treated filter paper and concentrated to give 4-[(5-fluoroquinazolin-4-yl)amino]-2-methoxyphenol as a yellow-green solid (910 mg, 73% yield). NMR spectrum (DMSO-d6); 3.78 (s, 3H), 6.79 (d, 1H), 7.13 (dd, 1H), 7.30 (d, 1H), 7.40 (dd, 1H), 7.59 (d, 1H), 7.81 (ddd, 1H), 8.50 (s, 1H), 8.89 (s, 1H), 8.94 (d, 1H); Mass spectrum MH⁺ 286.

*N*,*N*-Dimethylethanolamine (2.61 ml, 26.0 mmol) was added dropwise under nitrogen to a suspension of 60% sodium hydride dispersion (1.04 g, 26 mmol) in anhydrous DMA (75 ml). The mixture was stirred under an atmosphere of nitrogen for 30 minutes until effervescence had ceased. 4-[(5-Fluoroquinazolin-4-yl)amino]-2-methoxyphenol (2.00 g, 7.00 mmol) and anhydrous DMA (25 ml) were added, and the mixture heated under an atmosphere of nitrogen to 110°C for 2 hours. The mixture was cooled to ambient temperature, and saturated ammonium chloride solution (10 ml) was added. The mixture was concentrated *in vacuo*, and saturated sodium hydrogen carbonate (100 ml) was added to the residue. The mixture was stirred; the resulting precipitate was collected by filtration and washed with water (2 x 100 ml). The solid was purified by chromatography, eluting with 5% to 8% (10:1 MeOH / conc. NH_{3(aq)}) in ethyl acetate. Evaporation of the appropriate fractions and crystallisation from ethyl acetate gave the title compound as a pale yellow crystalline solid (1.69 g, 68% yield). NMR spectrum (DMSO-d6); 2.24 (s, 6H), 2.79 (t, 2H), 3.80 (s, 3H), 4.35 (t, 2H), 6.79 (d, 1H), 7.09 (dd, 1H), 7.12 (d, 1H), 7.31 (d, 1H), 7.48 (d, 1H), 7.70 (dd, 1H), 8.43 (s, 1H), 8.84 (s, 1H), 10.31 (s, 1H); Mass spectrum MH⁺ 355.

### Example 38 5-[2-(Dimethylamino)ethoxy]-N-(3-methoxy-4-(pyrazin-2-ylmethoxy)phenyl]quinazolin-4-amine

Methanesulphonyl chloride (26 µl, 0.33 mmol) was added dropwise to a solution of 2-(hydroxymethyl)pyrazine (33 mg, 0.30 mmol) and *N*,*N*-di*iso*-propylethylamine (57 µl, 0.33 mmol) in DCM (2 ml). The mixture was heated to 40°C for 2 hours. The solvent was evaporated, and the residue dissolved in DMA (1 ml). This solution was added to a mixture of 4-({5-[2-(dimethylamino)ethoxy]quinazolin-4-yl}amino)-2-methoxyphenol (obtained as described in Example 20, preparation of starting materials, 71 mg, 0.20 mmol), potassium carbonate (138 mg, 1.00 mmol), and 18-crown-6 (20 mg) in DMA (10 ml). The mixture was briefly sonicated, and was stirred at room temperature for 48 hours. The solvent was removed *in vacuo*, and the residue was partitioned between DCM (15 ml) and water (15 ml). The DCM layer was loaded onto a silica column; the column was eluted with 2 to 4% (10:1 MeOH / conc. NH_{3(aq)}) in DCM. Evaporation of the appropriate fractions followed by crystallisation from methyl tert-butyl ether gave the title compound as a white crystalline solid (53 mg, 59% yield). NMR spectrum (DMSO-d6); 2.25 (s, 6H), 2.80 (t, 2H), 3.84 (s, 3H), 4.37 (t, 2H), 5.26 (s, 2H), 7.12 (d, 1H), 7.15 (d, 1H), 7.22 (dd, 1H), 7.33 (d, 1H), 7.62 (d, 1H), 7.72 (dd, 1H), 8.47 (s, 1H), 8.64 (d, 1H), 8.68 (dd, 1H), 8.83 (d, 1H), 10.39 (s, 1H); Mass spectrum MH⁺ 448.

### Example 39 5-[2-(Dimethylamino)ethoxy]-N-[3-fluoro-4-(1,3-thiazol-5-ylmethoxy)phenyl]quinazolin-4-amine

Methanesulphonyl chloride (31 µl, 0.40 mmol) was added dropwise at 0°C to a solution of 5-(hydroxymethyl)-1,3-thiazole (42 mg, 0.36 mmol) and *N,N*-di*iso*propylethylamine (70 µl, 0.40 mmol) in DCM (2 ml). The mixture was allowed to warm to ambient temperature, and was stirred for 2 hours. The solvent was evaporated, and the residue dissolved in DMA (1 ml). This solution was added to a mixture of 4-({5-[2-(dimethylamino)ethoxy]quinazolin-4-yl}amino)-2-fluorophenol (68 mg, 0.20 mmol), potassium carbonate (138 mg, 1.00 mmol), and 18-crown-6 (20 mg) in DMA (10 ml). The mixture was stirred at room temperature for 16 hours. The solvent was removed *in vacuo*, and the residue was partitioned between DCM (15 ml) and water (15 ml). The DCM layer was loaded onto a silica column; the column was eluted with 2 to 4% (10:1 MeOH / conc. NH_{3(aq)}) in DCM. Evaporation of the appropriate fractions followed by crystallisation from ethyl acetate / *iso*-hexane gave the title compound as a white crystalline solid (35 mg, 40% yield); NMR spectrum (DMSO-d6); 2.27 (s, 6H), 2.81 (t, 2H), 4.37 (t, 2H), 5.48 (s, 2H), 7.16 (d, 1H), 7.35 (d, 1H), 7.36 (dd, 1H), 7.57 (dd, 1H), 7.74 (dd, 1H), 7.92 (dd, 1H), 8.02 (s, 1H), 8.53 (s, 1H), 9.14 (s, 1H), 10.51 (s, 1H); Mass spectrum MH⁺ 440.

The 4-({5-[2-(dimethylamino)ethoxy]quinazolin-4-yl}amino)-3-fluorophenol used as starting material was obtained as follows:

4-Chloro-5-fluoroquinazoline (obtained as described in Example 1, preparation of starting materials, 8.95 g, 49.0 mmol) and 4-amino-2-fluorophenol (6.27 g, 49.0 mmol) were heated in *iso*-propanol (150 ml) at 80°C for 1 hour. The mixture was cooled and filtered and the solid was dissolved in a mixture of hot water and methanol. This was basified using aqueous ammonia, stirred for 30 minutes and the resultant precipitate was filtered. Cold diethyl ether (2 x 10 ml) was used to wash the precipitate to give 2-fluoro-4-[(5-fluoroquinazolin-4-yl)amino]phenol as a solid (8.52 g, 64%); NMR spectrum (DMSO-d6); 6.94 (dd, 1H), 7.27 (dd, 1H), 7.41 (dd, 1H), 7.56-7.67 (m, 2H), 7.76-7.87 (m, 1H), 8.52 (s, 1H), 9.02 (d, 1H), 9.73 (s, 1H); Mass spectrum MH⁺ 274.

*N*,*N*'-Dimethylethanolamine (2.02 ml, 20.15 mmol) was added slowly to a suspension of sodium hydride (60% dispersion in mineral oil, 0.81 g, 20.15 mmol) in dry DMA (50 ml) and stirred for 30 minutes. 2-Fluoro-4-[(5-fluoroquinazolin-4-yl)amino]phenol was added in one portion and the mixture was heated to 95°C for 5 hours. The mixture was cooled and saturated ammonium chloride solution (50 ml) was added and the resultant precipitate was filtered to give 4-({5-[2-(dimethylamino)ethoxy]quinazolin-4-yl}amino)-2-fluorophenol as a solid (1.74 g, 56%); NMR spectrum (CDCl₃); 2.32 (s, 6H), 2.85 (t, 2H), 4.28 (t, 2H), 6.87 (d, 1H), 6.98 (dd, 1H), 7.37 (dd, 1H), 7.46 (d, 1H) 7.59-7.66 (m, 1H), 7.73 (dd, 1H), 8.61 (s, 1H), 10.40 (s, 1H); Mass spectrum MH⁺ 343.

### Example 40 N-[3-Chloro-4-(pyridin-2-ylmethoxy)phenyl]-5-[(1S)-2-(dimethylamino)-1-methylethoxy]quinazolin-4-amine

Sodium hydride (60% dispersion in mineral oil, 116 mg, 2.9 mmol) was suspended in 1,4-dioxane (25 ml) to which was added (S)-1-dirnethylamino-2-propanol (0.36 ml, 2.9 mmol) and the mixture stirred for 30 minutes. *N*-[3-Chloro-4-(pyridin-2-ylmethoxy)phenyl]-5-fluoroquinazolin-4-amine (obtained as described in Example 1, preparation of starting materials, 0.5 g, 1.32 mmol) was added in one portion followed by dry DMA (1 ml) and the mixture was heated to 100°C for 60 hours. The solvents were removed *in vacuo* and saturated ammonium chloride solution (10 ml) was added to the residue and stirred. This was basified using saturated sodium hydrogen carbonate and extracted with DCM (3 x 25 ml). The DCM was dried (MgSO₄), concentrated *in vacuo* and the residue purified by chromatography using 15% methanol / ethyl acetate as the eluent to give the title compound as a gum (430 mg, 70%); NMR spectrum (CDCl₃); 1.51 (d, 3H), 2.27 (s, 6H), 2.45 (dd, 1H), 2.90 (dd, 1H), 4.66-4.78 (m, 1H), 5.29 (s, 2H), 6.90 (d, 1H), 7.01 (d, 1H), 7.22 (dd, 1H), 7.42 (d, 1H), 7.54-7.68 (m, 3H), 7.69-7.78 (m, 1H), 7.83 (d, 1H), 8.53-8.64 (m, 2H), 10.39 (s, 1H); Mass spectrum MH⁺ 464.

### Example 41 N-[3-Chloro-4-(pyridin-2-ylmethoxy)phenyl]-5-{[(2S)-2-(dimethylamino)propyl]oxy}quinazolin-4-amine

(2*S*)-2-(Dimethylamino)propan-1-ol (0.064 g, 0.625 mmol) was added to a suspension of sodium hydride (60% dispersion in mineral oil, 0.052 g, 1.30 mmol) in 1,4-dioxan (4 ml) under N₂. The reaction was stirred for 30 minutes then 15-crown-5 (0.100 ml) was added followed by *N*-[3-chloro-4-(pyridin-2-ylmethoxy)phenyl]-5-fluoroquinazolin-4-amine (obtained as described in Example 1, preparation of starting materials, 0.190 g, 0.50 mmol). The reaction was heated in a CEM Explorer™ microwave synthesisor at 140°C for 40 minutes. The reaction was cooled, quenched with acetic acid (2 drops) and partitioned between saturated sodium bicarbonate solution and ethyl acetate. The organic extract was washed with water and concentrated *in vacuo*. The residue was purified by HPLC with MeCN / water as eluent and the fractions containing product concentrated *in vacuo*. The residue was dissolved in methanol (10 ml), cooled to 0°C and treated with ammonium hydroxide solution to give the title compound as a white solid (0.085 g, 37%); NMR spectrum (DMSO-d6); 1.05 (s, 3H), 2.30 (s, 6H), 3.21 (s, 1H), 4.15 (s, 1H), 4.38 (m, 1H), 5.30 (s, 2H), 7.13 (m, 1H), 7.30 (m, 1H), 7.35 (m, 2H), 7.60 (m, 1H), 7.72 (m, 2H), 7.88 (m, 1H), 7.92 (s, 1H), 8.50 (s, 1H), 8.60 (s, 1H), 10.50 (s, 1H); Mass spectrum MH⁺ 464.

The (2*S*)-2-(dimethylamino)propan-1-ol used as starting material was prepared as follows: (S)-Alaninol (6.6 g, 88 mmol) in formic acid (30 ml) and formaldehyde (12 ml) was heated at 95°C for 2 hours and cooled. The solvent was removed *in vacuo*, the residue dissolved in DCM (600 ml) and the solution stirred with polymer-supported sodium bicarbonate (200 g) for 1 hour. The solution was filtered and evaporated *in vacuo*. The residue was purified by distillation (25 mbar, 95<T<110 °C) to give the (2*S*)-2-(dimethylamino)propan-1-ol (4.77 g, 53%); NMR spectrum (DMSO-d6); 0.84 (d, 3H), 2.11 (d, 6H), 2.45 (m, 1H), 3.20 (m, 1H), 3.40 (m, 1H), 4.18 (t, 1H).

### Example 42 N-[3-Chloro-4-(pyridin-2-ylmethoxy)phenyl]-5-{[(2R)-2-(dimethylamino)propyl]oxy}quinazolin-4-amine

The procedure described in Example 41 was repeated using (2*R*)-2-(dimethylamino)propan-1-ol and *N*-[3-chloro-4-(pyridin-2-ylmethoxy)phenyl]-5-fluoroquinazolin-4-amine (obtained as described in Example 1, preparation of starting materials) to give the title compound as white crystals in 16% yield; NMR spectrum (DMSO-d6); 1.04 (s, 3H), 2.25 (s, 6H), 3.15 (m, 1H), 4.10 (m, 1H), 4.37 (m, 1H), 5.30 (s, 2H), 7.14 (d, 1H), 7.34 (m, 3H), 7.59 (d, 1H), 7.75 (m, 2H), 7.90 (m, 2H), 8.50 (s, 1H), 8.60 (d, 1H), 10.60 (m, 1H); Mass spectrum MH⁺ 464.

The (2*R*)-2-(dimethylamino)propan-1-ol used as starting material was prepared using the procedure described in Example 41, preparation of starting materials, using (R)-Alaninol.

### Example 43 5-{2-[Allyl(methyl)amino]ethoxy}-N-[3-chloro-4-(pyridin-2-ylmethoxy)phenyl]quinazolin-4-amine

Tetra-butylammonium iodide (84 mg, 0.22 mmol) and 5-(2-chloroethoxy)-*N*-[3-chloro-4-(pyridin-2-ylmethoxy)phenyl]quinazolin-4-amine (obtained as described in Example 14, preparation of starting materials, 100 mg, 0.22 mmol) were added to *N*-methylallylamine (100 mg, 1.4 mmol) in 1,4-dioxane (4 ml) and the resulting suspension was heated in the CEM Explorer^{™} microwave synthesisor at 150°C for 50 minutes. The solvent was removed *in vacuo* and the solid suspended in DCM (10 ml). The organic layer was washed with saturated sodium hydrogen carbonate solution (10 ml), water (10 ml) and brine (10 ml). The organic layer was dried with MgSO₄ and the solvent evaporated to give an oil. This was purified by preparative HPLC to give the title compound as a solid (63.8 mg, 57%); NMR spectrum (DMSO-d6); 2.20 (s, 3H), 2.80 (t, 2H), 3.00 (d, 2H), 4.30 (t, 2H), 5.00 (d, 1H), 5.10 (d, 1H), 5.20 (s, 2H), 5.60 (m, 1H), 7.10 (d, 1H), 7.20 (d, 1H), 7.30 (m, 2H), 7.50 (d, 1H), 7.70 (m, 2H), 7.80 (t, 1H), 7.90 (d, 1H), 8.40 (s, 1H), 8.50 (d, 1H), 10.30 (s, 1H); Mass spectrum MH⁺ 477.

### Example 44 2-[{2-[(4-{[3-Chloro-4-(pyridin-2-ylmethoxy)phenyl]amino}quinazolin-5-yl)oxy]ethyl}(ethyl)amino]ethanol

The procedure described in Example 43 was repeated using 2-(ethylamino)ethanol and 5-(2-chloroethoxy)-*N*-[3-chloro-4-(pyridin-2-ylmethoxy)phenyl]quinazolin-4-amine (obtained as described in Example 14, preparation of starting materials) to afford the title compound as a solid in 38% yield; NMR spectrum (DMSO-d6) 0.90 (t, 3H), 2.40 (m, 2H), 2.60 (m, 2H), 2.80 (m, 2H), 3.20 (2, H), 4.30 (t, 2H), 5.20 (s, 2H), 7.10 (d, 1H), 7.20 (d, 1H), 7.30 (m, 2H), 7.50 (d, 1H), 7.70 (m, 2H), 7.80 (m, 1H), 7.90 (d, 1H), 8.40 (s, 1H), 8.50 (d, 1H), 10.20 (s, 1H); Mass spectrum MH⁺ 495.

### Example 45 N-[3-Chloro-4-(pridin-2-ylmethoxy)phenyl]-5-{(1S)-2-[(2-methoxyethyl)(methyl)amino]-1-methylethoxy}quinazolin-4-amine dihydrochloride

(*S*)-(-)-Propylene oxide (0.20 ml, 2.86 mmol) and ytterbium triflate (10 mg) were added to a solution of *N*-(2-methoxyethyl)methylamine (87 mg, 1.00 mmol) in 1,4-dioxane (1.0 ml) in a 10 ml pressure vial. The vial was capped and irradiated at 140°C for 20 minutes in a CEM Explorer™ microwave synthesisor. The mixture was concentrated *in vacuo* and the residue dissolved in 1,4-dioxane (2.0 ml) in a 10 ml pressure vial. Sodium hydride (60% dispersion in mineral oil, 25 mg, 0.625 mmol), 15-crown-5 (20 mg) and *N*-[3-chloro-4-(pyridin-2-ylmethoxy)phenyl]-5-fluoroquinazolin-4-amine (obtained as described in Example 1, preparation of starting materials, 97.5 mg, 0.25 mmol) were added. The vial was capped and irradiated at 140°C for 40 minutes in a CEM Explorer™ microwave synthesisor. The solution was cooled to ambient temperature and glacial acetic acid (3 drops) was added. The solution was loaded onto a silica column, which was eluted with 0 to 10% methanol in ethyl acetate. The appropriate fractions were evaporated and the residue was dissolved in ether and treated with hydrogen chloride solution (1M in diethyl ether, 1.0 ml). The mixture was evaporated; precipitation of the residue from ethanol / diethyl ether gave the title compound as a beige solid (30 mg, 22%); NMR spectrum (DMSO-d6 @ 373K); 1.48 (d, 3H), 2.84 (s, 3H), 3.24 (s, 3H), 3.35 (m, 2H), 3.52 (m, 1H), 3.7-3.8 (m, 2H), 3.92 (m, 1H), 5.34 (s, 2H), 5.51 (m, 1H), 7.32 (d, 1H), 7.38 (dd, 1H), 7.55 (d, 1H), 7.57-7.65 (m, 3H), 7.90 (dd, 1H), 7.92 (d, 1H), 7.98 (dd, 1H), 8.60 (d, 1H), 8.70 (s, 1H), 10.40 (br. s, 1H); Mass spectrum MH⁺ 508.

### Example 46 N-[3-Chloro-4-(pyridin-2-ylmethoxy)phenyl]-5-{(1R)-2-[ethyl(methyl)amino]-1-methylethoxy}quinazolin-4-amine dihydrochloride

(R)-(+)-Propylene oxide (0.20 ml, 2.86 mmol) and ytterbium triflate (10 mg) were added to a solution of *N*-ethylmethylamine (35 mg, 0.60 mmol) in 1,4-dioxane (1.0 ml) in a 10 ml pressure vial. The vial was capped and irradiated at 140°C for 20 minutes in a CEM Explorer™ microwave synthesisor. The mixture was concentrated *in vacuo* and the residue dissolved in 1,4-dioxane (2.0 ml) in a 10 ml pressure vial. Sodium hydride (60% dispersion in mineral oil, 25 mg, 0.625 mmol), 15-crown-5 (20 mg) and N-[3-chloro-4-(pyridin-2-ylmethoxy)phenyl]-5-fluoroquinazolin-4-amine (obtained as described in Example 1, preparation of starting materials, 97.5 mg, 0.25 mmol) were added. The vial was capped and irradiated at 140°C for 40 minutes in a CEM Explorer™ microwave synthesisor. The solution was cooled to ambient temperature and glacial acetic acid (3 drops) was added. The solution was loaded onto a silica column, which was eluted with 0 to 10% methanol in ethyl acetate. The appropriate fractions were evaporated; the residue was dissolved in ether and treated with hydrogen chloride solution (1M in diethyl ether, 1.0 ml). The mixture was evaporated; precipitation of the residue from ethanol / diethyl ether gave the title compound as a beige solid (26mg, 18 %); NMR spectrum (DMSO-d6 @373K); 1.30 (t, 3H), 1.49 (d, 3H), 2.80 (s, 3H), 3.20 (m, 2H), 3.47 (m, 1H), 3.87 (m, 1H), 5.33 (s, 2H), 5.50 (m, 1H), 7.31 (d, 1H), 7.38 (dd, 1H), 7.55-7.65 (m, 4H), 7.89 (dd, 1H), 7.90 (m, 2H), 8.60 (d, 1H), 8.70 (s, 1H), 10.40 (br. s, 1H), 11.0-12.0 (br. s, 1H); Mass spectrum MH⁺ 478.

### Example 47 5-{(1R)-2-[Allyl(methyl)amino]-1-methylethoxy}-N-[3-chloro-4-(pyridin-2-ylmethoxy)phenyl]quinazolin-4-amine

(R)-(+)-Propylene oxide (0.20 ml, 2.86 mmol) and ytterbium triflate (10 mg) were added to a solution of *N*-allylmethylamine (42 mg, 0.59 mmol) in 1,4-dioxane (1.0 ml) in a 10 ml pressure vial. The vial was capped and irradiated at 140°C for 20 minutes in a CEM Explorer™ microwave synthesisor. The mixture was concentrated *in vacuo* and the residue dissolved in 1,4-dioxane (2.0 ml) in a 10 ml pressure vial. Sodium hydride (60% dispersion in mineral oil, 25 mg, 0.625 mmol), 15-crown-5 (20 mg) and *N*-[3-chloro-4-(pyridin-2-ylmethoxy)phenyl]-5-fluoroquinazolin-4-amine (obtained as described in Example 1, preparation of starting materials, 97.5 mg, 0.25 mmol) were added. The vial was capped and irradiated at 140°C for 40 minutes in a CEM Explorer™ microwave synthesisor. The solution was cooled to ambient temperature and glacial acetic acid (3 drops) was added. The solution was loaded onto a silica column, which was eluted with 0 to 10% methanol in ethyl acetate. Evaporation of the appropriate fractions gave the title compound as a gum (51mg, 42%); NMR spectrum (DMSO-d6 @373K); 1.49 (d, 3H), 2.25 (s, 3H), 2.50 (dd, 1H), 2.68 (dd, 1H), 3.08 (m, 2H), 4.95 (m, 1H), 5.03 (dd, 1H), 5.10 (dd, 1H), 5.28 (s, 2H), 5.72 (m, 1H), 7.20 (d, 1H), 7.26 (d, 1H), 7.35 (dd, 1H), 7.35 (d, 1H), 7.55-7.60 (m, 2H), 7.70(dd, 1H), 7.83 (ddd, 1H), 7.97 (d, 1H), 8.50 (s, 1H), 8.56 (d, 1H), 10.2 (s, 1H); Mass Spectrum MH⁺ 490.

### Example 48 5-{(1S)-2-[Allyl(methyl)amino]-1-methylethoxy}-N-[3-chloro-4-(pyridin-2-ylmethoxy)phenyl]quinazolin-4-amine

(*S*)-(-)-Propylene oxide (0.20 ml, 2.86 mmol) and ytterbium triflate (10 mg) were added to a solution of *N*-allylmethylamine (42 mg, 0.59 mmol) in 1,4-dioxane (1.0 ml) in a 10 ml pressure vial. The vial was capped and irradiated at 140°C for 20 minutes in a CEM Explorer™ microwave synthesisor. The mixture was concentrated *in vacuo* and the residue dissolved in 1,4-dioxane (2.0 ml) in a 10 ml pressure vial. Sodium hydride (60% dispersion in mineral oil, 25 mg, 0.625 mmol), 15-crown-5 (20 mg) and *N*-[3-chloro-4-(pyridin-2-ylmethoxy)phenyl]-5-fluoroquinazolin-4-amine (obtained as described in Example 1, preparation of starting materials, 97.5 mg, 0.25 mmol) were added. The vial was capped and irradiated at 140°C for 40 minutes in a CEM Explorer™ microwave synthesisor. The solution was cooled to ambient temperature and glacial acetic acid (3 drops) was added. The solvents were evaporated *in vacuo*, and the residue partitioned between DCM (10 ml) and water (10 ml). The organic layer was loaded onto a silica column, which was eluted with 0 to 10% methanol in ethyl acetate. The appropriate fractions were evaporated to give the title compound as a gum (30 mg, 25 %); NMR spectrum (DMSO-d6 @373K); 1.49 (d, 3H), 2.25 (s, 3H), 2.50 (dd, 1H), 2.68 (dd, 1H), 3.08 (m, 2H), 4.95 (m, 1H), 5.03 (dd, 1H), 5.10 (dd, 1H), 5.28 (s, 2H), 5.72 (m, 1H), 7.20 (d, 1H), 7.26 (d, 1H), 7.35 (dd, 1H), 7.35 (d, 1H), 7.55-7.60 (m, 2H), 7.70 (dd, 1H), 7.83 (ddd, 1H), 7.97 (d, 1H), 8.50 (s, 1H), 8.56 (d, 1H), 10.2 (s, 1H); Mass spectrum MH⁺ 490.

### Example 49 N-{3-Chloro-4-[(3-fluorobenzyl)oxy]phenyl}-5-{[(2S)-2-(dimethylamino)propyl]oxy}quinazolin-4-amine

The procedure described in Example 41 was repeated using (2*S*)-2-(dimethylamino)propan-1-ol and *N*-{3-chloro-4-[(3-fluorobenzyl)oxy]phenyl}-5-fluoroquinazolin-4-amine (obtained as described in Example 5, preparation of starting materials) to give the title compound as white crystals in 28% yield; NMR spectrum (DMSO-d6); 1.00 (d, 3H), 2.20 (s, 6H), 3.15 (s, 1H), 4.05 (t, 1H), 4.38 (dd, 1H), 5.25 (s, 2H), 7.15 (m, 2H), 7.30 (m, 4H), 7.45 (m, 1H), 7.74 (m, 2H), 7.90 (s, 1H), 8.50 (s, 1H), 10.58 (s, 1H); Mass spectrum MH⁺ 481.

### Example 50 N-{3-Chloro-4-[(3-fluorobenzyl)oxy]phenyl}-5-{[(2R)-2-(dimethylamino)propyl]oxy}quinazolin-4-amine

The procedure described in Example 41 was repeated using (2R)-2-(dimethylamino)propan-1-ol and *N*-{3-chloro-4-[(3-fluorobenzyl)oxy]phenyl}-5-fluoroquinazolin-4-amine (obtained as described in Example 5, preparation of starting materials) to give the title compound as white crystals in 33% yield; NMR spectrum (DMSO-d6); 1.03 (d, 3H), 2.24 (s, 6H), 3.19 (m, 1H), 4.10 (m, 1H), 4.37 (dd, 1H), 5.24 (s, 2H), 7.15 (d, 2H), 7.30 (m, 4H), 7.45 (m, 2H), 7.70 (m, 2H), 7.90 (d, 1H), 8.50 (s, 1H); Mass spectrum MH⁺ 481.

### Example 51 N-{3-Chloro-4-[(1-methyl-1H-imidazol-2-yl)thio]phenyl}-5-{[(2S)-2-(dimethylamino)propyl]oxy}quinazolin-4-amine

The procedure described in Example 41 was repeated using (2*S*)-2-(dimethylamino)propan-1-ol and *N*-{3-chloro-4-[(1-methyl-1*H*-imidazol-2-yl)thio]phenyl}-5-fluoroquinazolin-4-amine (obtained as described in Example 3, preparation of starting materials) to give the title compound as white crystals in 36% yield; NMR spectrum (DMSO-d6); 1.37 (d, 3H), 2.75 (s, 6H), 3.70 (s, 3H), 3.85 (m, 1H), 4.46 (dd, 1H), 4.55 (m, 1H), 6.85 (d, 1H), 7.10 (s, 1H), 7.20 (d, 1H), 7.40 (m, 2H), 7.65 (dd, 1H), 7.75 (m, 1H), 8.10 (d, 1H), 8.58 (s, 1H); Mass spectrum MH⁺ 469.

### Example 52 N-{3-Chloro-4-[(1-methyl-1H-imidazol-2-yl)thio]phenyl}-5-{[(2R)-2-(dimethylamino)propyl]oxy}quinazolin-4-amine hydrochloride

The procedure described in Example 41 was repeated using (2*R*)-2-(dimethylamino)propan-1-ol and *N*-{3-chloro-4-[(1-methyl-1*H*-imidazol-2-yl)thio]phenyl}-5-fluoroquinazolin-4-amine (obtained as described in Example 3, preparation of starting materials) to give the title compound as white crystals as the hydrochloride salt in 9% yield; NMR spectrum (DMSO-d6); 1.40 (d, 3H), 2.75 (m, 6H), 3.80 (s, 3H), 4.15 (m, 1H), 4.55 (dd, 1H), 4.85 (dd, 1H), 7.00 (d, 1H), 7.49 (d, 1H), 7.58 (m, 2H), 7.68 (dd, 1H), 7.80 (s, 1H), 8.04 (dd, 1H), 8.12 (s, 1H), 8.85 (s, 1H), 10.60 (s, 1H), 11.40 (s, 1H); Mass spectrum MH⁺ 469

### Example 53 N-{3-Chloro-4-[(1-methyl-1H-imidazol-2-yl)thio]phenyl}-5-[(1R)-2-(dimethylamino)-1-methylethoxy]quinazolin-4-amine dihydrochloride

The procedure described in Example 46 was repeated using dimethylamine (2M in 1,4 dioxane), (*R*)-(+)-propylene oxide and *N*-{3-chloro-4-[(1-methyl-1*H*-imidazol-2-yl)thio]phenyl}-5-fluoroquinazolin-4-amine (obtained as described in Example 3, preparation of starting materials) to give the title compound as a yellow solid (25%); NMR spectrum (DMSO-d6 @373K); 1.50 (d, 3H), 2.88 (s, 6H), 3.55 (d, 1H), 3.80 (s, 3H), 3.87 (dd, 1H), 5.52 (m,1H), 7.10 (d, 1H), 7.40 (d, 1H), 7.58 (d, 1H), 7.60-7.65 (m, 2H), 7.72 (d, 1H), 7.98 (dd,1H), 8.21 (d, 1H), 8.75 (s, 1H), 10.40 (br. s, 1H), 11.5 (br. s, 1H); Mass spectrum MH⁺ 469.

### Example 54 5-[2-(Dimethylamino)-1-methylethoxy]-N-(3-methoxy-4-phenoxyphenyl)quinazolin-4-amine

Sodium hydride (111 mg, 2.8 mmol of a 60% dispersion in mineral oil) was added to 1-(dimethylamino)propan-2-ol (114 mg, 1.1 mmol) in DMF (2 ml). Once the effervescence had subsided, 5-fluoro-*N*-(3-methoxy-4-phenoxyphenyl)quinazolin-4-amine (200 mg, 0.55 mmol) was added and the reaction was heated at 120°C for 2 hours. The DMF was removed *in vacuo*, ethyl acetate (10 ml) was added and the mixture sonicated using a sonic cleaning bath to achieve a fine suspension. Water (10 ml) was added and the organic layer separated. This was washed with brine (10 ml), diatomaceous earth was added to the organic layer and the solvent was removed *in vacuo* to enable dry loading of residue onto a column (SiO₂). A gradient of 0-5% 7N methanolic ammonia in DCM, evaporation of relevant fractions and trituration with diethyl ether of the resultant oil afforded the title compound as a beige solid (34 mg, 14%); NMR spectrum (DMSO-d6) 1.50 (d, 3H), 2.20 (s, 6H) 2.40 (m, 1H), 2.90 (dd, 1H), 3.80 (s, 3H), 4.90 (m, 1H), 6.80 (d, 2H), 7.00 (m, 2H), 7.20 (d, 1H), 7.30 (m, 4H), 7.70 (dd, 2H), 8.50 (s, 1H), 10.40 (br s, 1H); Mass spectrum MH⁺ 445.

The 5-fluoro-*N*-(3-methoxy-4-phenoxyphenyl)quinazolin-4-amine used as starting material was obtained as follows:
Potassium carbonate (12.65 g, 0.09 mol) was added to a solution of phenol (5.06 g, 0.05 mol) in DMF (200 ml) to give a white, cloudy suspension. 2-bromo-5-nitroanisole (15.00 g, 0.065 mol) was added portionwise over 20 minutes to give a progressively darker solution. The resulting mixture was heated at 80°C for 36 hours. DMF was removed *in vacuo*, water (200 ml) was added and the aqueous layer was extracted with ethyl acetate (x 3). The organics were combined, dried with MgSO₄, filtered and evaporated to give a brown oil, which crystallized on standing. This was purified by chromatography using 3 : 7 DCM :
   isohexane as eluent. The relevant fractions were combined and evaporated to afford 2-methoxy-4-nitro-1-phenoxybenzene as a yellow oil (8.21 g, 62%); NMR spectrum (DMSO-d6) 3.90 (s, 3H), 7.00 (m, 3H), 7.20 (m, 1H), 7.40 (m, 2H), 7.80 (dd, 1H), 7.90 (d, 1H).

2-Methoxy-4-nitro-1-phenoxybenzene (4.00 g, 16 mmol) was dissolved in ethyl acetate : ethanol (9:1) (200 ml). 10% Pd/C (0.40 g) was added and the resulting solution was stirred under a hydrogen balloon for 3 hours. The suspension was filtered through diatomaceous earth and the filter pad washed several times with more ethanol. The solvent was removed *in vacuo* to give 3-methoxy-4-phenoxyaniline as a pink solid (3.36 g, 96%); NMR spectrum (DMSO-d6) 3.60 (s, 3H), 5.00 (s, 2H), 6.10 (dd, 1H), 6.40 (d, 1H), 6.70 (m, 3H), 6.90 (t, 1H), 7.20 (t, 2H); Mass spectrum MH⁺ 216.

4-Chloro-5-fluoroquinazoline (obtained as described in Example 1, preparation of starting materials, 0.85 g, 4.6 mmol) was added portionwise to a solution of 3-methoxy-4-phenoxyaniline (1.00 g, 4.6 mmol) and di-*iso*-propylethylamine (0.82 ml, 4.6 mmol) in isopropyl alcohol (50 ml). The resulting orange solution was heated at 80°C for 18 hours. The isopropyl alcohol was removed *in vacuo* to a minimum volume and the flask cooled in an ice bath. The precipitated product was removed by filtration and subsequently washed with cold isopropyl alcohol and then diethyl ether to afford the title compound as a beige solid (1.22 g, 73%); NMR spectrum (DMSO-d6); 3.75 (s, 3H), 6.80 (d, 2H), 7.00 (m, 2H), 7.30 (m, 2H), 7.50 (m, 2H), 7.60 (m, 2H), 7.80 (m, 1H), 8.60 (s, 1H), 9.20 (d, 1H); Mass spectrum MH⁺ 362.

### Example 55 5-[2-(Dimethylamino)-1-methylethoxy]-N-(3-methoxy-4-phenoxyphenyl)quinazolin-4-amine

The procedure described in Example 54 was repeated using 2-(dimethylamino)ethanol and 5-fluoro-*N*-(3-methoxy-4-phenoxyphenyl)quinazolin-4-amine (obtained as described in Example 54, preparation of starting materials) to give the title compound as a beige solid in 50% yield; NMR spectrum (DMSO-d6) 2.20 (s, 6H), 2.80 (t, 2H), 3.80 (s, 3H), 4.40 (t, 2H), 6.80 (d, 2H), 7.00 (m, 2H), 7.20 (d, 1H), 7.30 (m, 4H), 7.70 (t, 1H), 7.80 (m, 1H), 8.50 (s, 1H), 10.40 (br s, 1H); Mass spectrum MH⁺ 431.

### Example 56 N-[3-Chloro-4-(pyridin-2-ylmethoxy)phenyl]-5-[2-(dimethylamino)-1,1-dimethylethoxy]quinazolin-4-amine

Di-*tert*-butyl hydrazodicarboxylate (64 mg, 0.28 mmol) was added to a mixture of 4-(3-chloro-4-(2-pyridylmethoxy)anilino)-5-hydroxyquinazoline (prepared as described in Example 14, preparation of starting materials, 70 mg, 0.19 mmol), 2-(dimethylamino)-2-methylpropan-1-ol (33 mg, 0.28 mmol) and triphenylphosphine (73 mg, 0.28 mmol) in anhydrous DCM (15 ml). The mixture was stirred at ambient temperature. At intervals of 1 hour and 2 hours, further charges of 2-(dimethylamino)-2-methylpropan-1-ol (33 mg, 0.28 mmol), triphenylphosphine (73 mg, 0.28 mmol) and di-*tert*-butyl hydrazodicarboxylate (64 mg, 0.28 mmol) were made. After 3 hours, the reaction mixture was loaded onto an SCX column. The column was eluted with 20% MeOH in DCM, then with 20 % (7N NH₃ in MeOH) in DCM. The product containing fractions were combined and evaporated. The residue was purified by chromatography, eluting with 1% to 3% (10:1 MeOH / conc. NH_{3(aq)}) in DCM. Evaporation of the appropriate fractions and crystallisation of the residue from ethyl acetate / *iso*-hexane gave the title compound as a white crystalline solid (40 mg, 45% yield); NMR spectrum (DMSO-d6); 1.45 (s, 6H), 2.18 (s, 6H), 2.69 (s, 2H), 5.31 (s, 2H), 7.29 (d, 1H), 7.31 (d, 1H), 7.38 (dd, 1H), 7.45 (d, 1H), 7.46 (dd, 1H), 7.59 (d, 1H), 7.71 (dd, 1H), 7.75 (d, 1H), 7.89 (ddd, 1H), 8.40 (s, 1H), 8.60 (d, 1H), 10.76 (s, 1H); Mass spectrum MH⁺ 478.

## Claims

1. A quinazoline derivative of the formula I: 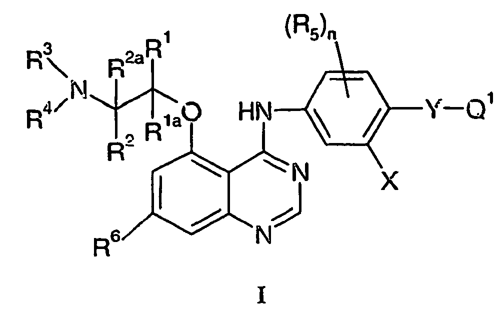 wherein:
**each of R¹ and R²,** which may be the same or different, is selected from hydrogen, carboxy, cyano, formyl, (1-3C)alkyl, (2-3C)alkanoyl, (1-3C)alkoxycarbonyl, carbamoyl, N-(1-3C)alkylcarbamoyl and N, N-di-[(1-3C)alkyl]carbamoyl;
**each of R^{1a} and R^{2a},** which may be the same or different, is selected from hydrogen and (1-3C)alkyl;
**each of R³ and R⁴,** which may be the same or different, is selected from hydrogen, (1-3C)alkyl and (2-4C) alkenyl;
and wherein any CH or CH₂ or CH₃ within any of R¹, R^{1a}, R², R^{2a}, R³ and R⁴ optionally bears on each said CH or CH₂ or CH₃ one or more (for example 1, 2 or 3) halogeno substituents or a substituent selected from hydroxy, cyano, (1-3C)alkoxy, amino, (2-3C)alkanoyl, (1-3C)alkylamino and di-[(1-3C)alkyl]amino;
**X** is selected from hydrogen, halogeno, (1-4C)alkyl, (1-4C)alkoxy, (2-4C)alkenyl and (2-4C)alkynyl;
**each R⁵,** which may be the same or different, is selected from halogeno, hydroxy, (1-4C)alkyl, (1-4C)alkoxy, (2-4C)alkenyl and (2-4C)alkynyl;
**Y** is selected from a direct bond, O, S, OC(R⁷)₂, SC(R⁷)₂, SO, SO₂, N(R⁷), CO and N(R⁷)C(R⁷)₂ wherein each R⁷ is, independently, hydrogen or (1-6C)alkyl;
**Q¹** is selected from phenyl, pyridyl, pyrazinyl, 1,3-thiazolyl, 1H-imidazolyl, 1H-pyrazolyl, 1,3-oxazolyl and isoxazolyl,
and wherein Q¹ optionally bears one or more substituents (for example 1, 2 or 3), which may be the same or different, selected from halogeno, cyano, nitro, hydroxy, amino, carboxy, carbamoyl, sulfamoyl, formyl, mercapto, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino, and N-(1-6C)alkyl-(1-6C)alkanesulfonylamino, or from a group of the formula:
-X¹-R⁸
wherein X¹ is a direct bond or is selected from O, CO and N(R⁹), wherein R⁹ is hydrogen or (1-6C)alkyl, and R⁸ is halogeno-(1-6C)alkyl, hydroxy-(1-6C)alkyl, carboxy-(1-6C)alkyl, (1-6C)alkoxy-(1-6C)alkyl, cyano-(1-6C)alkyl, amino-(1-6C)alkyl, N-(1-6C)alkylamino-(1-6C)alkyl, N,N-di-[(1-6C)alkyl]amino-(1-6C)alkyl, (2-6C)alkanoylamino-(1-6C)alkyl, (1-6C)alkoxycarbonylamino-(1-6C)alkyl, carbamoyl-(1-6C)alkyl, N-(1-6C)alkylcarbamoyl-(1-6C)alkyl, N,N-di-[(1-6C)alkyl]carbamoyl-(1-6C)alkyl, (2-6C)alkanoyl-(1-6C)alkyl or (1-6C)alkoxycarbonyl-(1-6C)alkyl,
and wherein any CH₂ or CH₃ within a substituent on Q¹ optionally bears on each said CH₂ or CH₃ one oR more (for example 1, 2, or 3) halogeno or (1-6C)alkyl substituents or a substituent selected from hydroxy, cyano, amino, (1-4C)alkoxy, (1-4C)alkylamino and di-[(1-4C)alkyl]amino;
**R⁶** is selected from hydrogen, (1-6C)alkoxy, (2-6C)alkenyloxy and (2-6C)alkynyloxy, and wherein any CH₂ or CH₃ group within a R⁶ substituent optionally bears on each said CH₂ or CH₃ group one or more halogeno or (1-6C)alkyl substituents, or a substituent selected from hydroxy and (1-6C)alkoxy;
**n** is 0, 1, 2 or 3; or a pharmaceutically acceptable salt thereof.

2. A quinazoline derivative of the formula I as defined in claim 1, wherein R¹ is selected from hydrogen, methyl and ethyl, R² is selected from hydrogen, carboxy, cyano, methyl, ethyl, acetyl, methoxycarbonyl, carbamoyl, N-methylcarbamoyl and N, N-di-methylcarbamoyl, and R^{1a} and R^{2a} are each hydrogen.

3. A quinazoline derivative of the formula I as defined in claim 1, wherein R² is selected from hydrogen, methyl and ethyl, R¹ is selected from hydrogen, carboxy, cyano, methyl, ethyl, acetyl, methoxycarbonyl, carbamoyl, N-methylcarbamoyl and N, N-di-methylcarbamoyl, and R^{1a} and R^{2a} are each hydrogen.

4. A quinazoline derivative of the formula I as defined in claim 1, wherein R¹ and R^{1a} are each hydrogen, R² is selected from hydrogen, carboxy, cyano, methyl, ethyl, acetyl, methoxycarbonyl, carbamoyl, N-methylcarbamoyl and N, N-di-methylcarbamoyl, and R^{2a} is selected from hydrogen and (1-3C)alkyl.

5. A quinazoline derivative of the formula I as defined in claim 1, wherein R² and R^{2a} are each hydrogen, R¹ is selected from hydrogen, carboxy, cyano, methyl, ethyl, acetyl, methoxycarbonyl, carbamoyl, N-methylcarbamoyl and N, N-di-methylcarbamoyl, and R^{1a} is selected from hydrogen and (1-3C)alkyl.

6. A quinazoline derivative of the formula I as defined in any one of claims 1, 2, 3 and 5,
wherein R¹ is methyl, and R², R^{1a} and R^{2a} are each hydrogen.

7. A quinazoline derivative of the formula I as defined in any one of claims 1 to 4,
wherein R² is methyl and R¹, R^{1a} and R^{2a} are each hydrogen.

8. A quinazoline derivative of the formula I as defined in claim 1 or claim 5, wherein R¹ and R^{1a} are each methyl and R² and R^{2a} are each hydrogen.

9. A quinazoline derivative of the formula I as defined in claim 1 or claim 4, wherein R² and R^{2a} are each methyl and R¹ and R^{1a} are each hydrogen.

10. A quinazoline derivative of the formula I as defined in any one of the preceding claims, wherein each of R³ and R⁴, which may be the same or different, is selected from (1-3C)alkyl, wherein any CH or CH₂ or CH₃ within any of R³ and R⁴ optionally bears on each said CH or CH₂ or CH₃ one or more substituents selected from hydroxy and (1-3C)alkoxy.

11. A quinazoline derivative of the formula I as defined in any one of claims 1 to 9,
wherein each of R³ and R⁴, which may be the same or different, is selected from hydrogen, methyl, ethyl, propenyl, 2-methoxyethyl and 2-hydroxyethyl.

12. A quinazoline derivative of the formula I as defined in claim 11, wherein each of R³ and R⁴, which may be the same or different, is selected from methyl, ethyl, propenyl, 2-methoxyethyl and 2-hydroxyethyl.

13. A quinazoline derivative of the formula I as defined in claim 11 or claim 12, wherein R³ is methyl and R⁴ is selected from methyl, ethyl, 2-hydroxyethyl, 2-methoxyethyl and propenyl.

14. A quinazoline derivative of the formula I as defined in any one of claims 10 to 13,
wherein R³ and R⁴ are each methyl.

15. A quinazoline derivative of the formula I as defined in any one of claims 10 to 12,
wherein R³ is ethyl and R⁴ is 2-hydroxyethyl.

16. A quinazoline derivative of the formula I as defined in any one of the preceding claims, wherein X is selected from hydrogen, halogeno, (1-4C)akyl and (1-4C)alkoxy.

17. A quinazoline derivative of the formula I as defined in claim 16, wherein X is selected from hydrogen, fluoro, chloro, methyl and methoxy.

18. A quinazoline derivative of the formula I as defined in claim 16 or claim 17, wherein X is selected from methyl and chloro.

19. A quinazoline derivative of the formula I as defined in claim 18, wherein X is chloro.

20. A quinazoline derivative of the formula I as defined in claim 18, wherein X is methyl.

21. A quinazoline derivative of the formula I as defined in any one of the preceding claims, wherein Y is selected from O, S and OC(R⁷)₂ wherein each R⁷ is, independently, hydrogen or (1-4C)alkyl.

22. A quinazoline derivative of the formula I as defined in claim 21, wherein Y is selected from O, S and OCH₂.

23. A quinazoline derivative of the formula I as defined in claim 21 or claim 22, wherein YisO.

24. A quinazoline derivative of the formula I as defined in claim 21 or claim 22, wherein Y is S.

25. A quinazoline derivative of the formula I as defined in claim 21 or claim 22, wherein Y is OCH₂.

26. A quinazoline derivative of the formula I as defined in any one of the preceding claims, wherein n is 0.

27. A quinazoline derivative of the formula I as defined in any one of the preceding claims, wherein Q¹ is selected from phenyl, 2-pyridyl, 2-pyrazinyl, 1,3-thiazol-4-yl, 1,3-thiazol-5-yl, 1H-imidazol-2-yl and isoxazol-3-yl, and wherein Q¹ optionally bears one or more substituents, which may be the same or different, as defined in claim 1.

28. A quinazoline derivative of the formula I as defined in claim 27, wherein Q¹ is selected from phenyl, 2-pyridyl, 2-pyrazinyl, 1,3-thiazol-4-yl, 1,3-thiazol-5-yl, 1H-imidazol-2-yl and 3-isoxazolyl, and wherein Q¹ optionally bears one or more substituents, which may be the same or different, selected from fluoro and (1-4C)alkyl.

29. A quinazoline derivative of the formula I as defined in claim 27 or claim 28, wherein Q¹ is selected from 3-fluorophenyl, 2-pyridyl, 2-pyrazinyl, 1-methyl-1H-imidazol-2-yl, 1,3-thiazol-4-yl, 1,3-thiazol-5-yl and 5-methyl-3-isoxazolyl.

30. A quinazoline derivative of the formula I as defined in any one of the preceding claims, wherein R⁶ is hydrogen.

31. A quinazoline derivative according to claim 1 selected from one or more of the following:
4-(3-Chloro-4-(2-pyridylmethoxy)anilino)-5-(2dimethylaminoethoxy)quinazoline;
4-(3-Chloro-4-(2-pyridylmethoxy)anilino)-5-(2-dimethylamino-1-methylethoxy)quinazoline;
4-(3-Chloro-4-(1-methyl-1*H*-imidazol-2-ylthio)anilino)-5-(2-dimethylaminoethoxy)quinazoline;
4-(3-Chloro-4-(1-methyl-1*H*-imidazol-2-ylthio)anilino)-5-(2-dimethylamino-2-methylethoxy)quinazoline;
4-(4-(3-Fluorobenzyloxy)anilino)-5-(2-dimethylaminoethoxy)quinazoline;
4-(4-(3-Fluorobenzyloxy)anilino)-5-(2-dimethylamino-1-methylethoxy)quinazoline;
4-(3-Chloro-4-(2-pyrazinylmethoxy)anilino)-5-(2-dimethylaminoethoxy)quinazoline;
4-(3-Chloro-4-(2-pyrazinylmethoxy)anilino)-5-(2-dimethylamino-1-methylethoxy)quinazoline;
4-(3-Chloro-4-(5-methylisoxazol-3-ylmethoxy)anilino)-5-(2-dimethylaminoethoxy)quinazoline;
4-(3-Chloro-4-(5-methylisoxazol-3-ylmethoxy)anilino)-5-(2-dimethylamino-1-methylethoxy)quinazoline;
4-(3-Chloro-4-(3-fluorobenzyloxy)anilino)-5-(2-(N-ethyl-N-methylamino)ethoxy)quinazoline;
4-(3-Chloro-4-(3-fluorobenzyloxy)anilino)-5-(2-dimethylaminoethoxy)quinazoline;
4-(3-Chloro-4-(3-fluorobenzyloxy)anilino)-5-[2-(N-(2-hydroxyethyl)-N-methylamino)ethoxy]quinazoline;
4-(3-Chloro-4-(2-pyridylmethoxy)anilino)- 5-(2-(N-ethyl-N-methylamino)ethoxy)quinazoline;
4-(3-Chloro-4-(2-pyridylmethoxy)anilino)- 5-(2-(N-(2-hydroxyethyl)-N-methylamino)ethoxy)quinazoline;
4-(3-Chloro-4-(3-fluorobenzyloxy)anilino)-5-(2-dimethylamino-2-methylethoxy)quinazoline;
4-(3-Chloro-4-(2-pyridylmethoxy)anilino)-5-(2-dimethylamino-2-methylethoxy)quinazoline;
*N*-[3-Chloro-4-(1,3-thiazol-4-ylmethoxy)phenyl]-5-[2-(dimethylamino)ethoxy]quinazolin-4-amine;
*N*-[3-Chloro-4-(pyridin-2-yloxy)phenyl]-5-[2-(dimethylamino)ethoxy]quinazolin-4-amine;
*N*-[3-Chloro-4-(pyrazin-2-ylmethoxy)phenyl]-5-[(1*S*)-2-(dimethylamino)-1-methylethoxy]quinazolin-4-amine;
*N*-{3-Chloro-4-[(3-fluorobenzyl)oxy]phenyl}-5-[(1*S*)-2-(dimethylamino)-1-methylethoxy]quinazolin-4-amine;
*N*-[3-Chloro-4-(pyridin-2-ylmethoxy)phenyl]-5-[(1*R*)-2-(dimethylamino)-1-methylethoxy]quinazolin-4-amine;
*N*-[3-Chloro-4-(1,3-thiazol-4-ylmethoxy)phenyl]-5-[(1*R*)-2-(dimethylamino)-1-methylethoxy]quinazolin-4-amine;
*N*-[3-Chloro-4-(pyrazin-2-ylmethoxy)phenyl]-5-[(1*R*)-2-(dimethylamino)-1-methylethoxy]quinazolin-4-amine;
*N*-{3-Chloro-4-[(3-fluorobenzyl)oxy]phenyl}-5-[(1*R*)-2-(dimethylamino)-1-methylethoxy]quinazolin-4-amine;
*N*-[3-Chloro-4-(pyridin-2-ylmethoxy)phenyl]-5-[2-(dimethylamino)-2-methylpropoxy]quinazolin-4-amine;
*N*-[3-Chloro-4-(1,3-thiazol-4-ylmethoxy)phenyl]-5-[2-(dimethylamino)-2-methylpropoxy]quinazolin-4-amine;
*N*-{3-Chloro-4-[(5-methylisoxazol-3-yl)methoxy]phenyl}-5-[2-(dimethylamino)-2-methylpropoxy]quinazolin-4-amine;
5-[2-(Dimethylamino)ethoxy]-*N*-[3-methyl4-(pyridin-2-ylmethoxy)phenyl]quinazolin-4-amine;
5-[2-(Dimethylamino)ethoxy]-*N*-[3-methyl-4-(1,3-thiazol-4-ylmethoxy)phenyl]quinazolin-4-amine;
5-[2-(Dimethylamino)ethoxy]-*N*-{3-methyl-4-[(5-methylisoxazol-3-yl)methoxy]phenyl}quinazolin-4-amine;
5-[(1*R*)-2-(Dimethylamino)-1-methylethoxy]-*N*-[3-methyl-4-(pyridin-2-ylmethoxy)phenyl]quinazolin-4-amine;
5-[(1*R*)-2-(Dimethylamino)-1-methylethoxy]-*N*-[3-methyl-4-(pyrazin-2-ylmethoxy)phenyl]quinazolin-4-amine;
5-[(1*R*)-2-(dimethylamino)-1-methylethoxy]-*N*-[3-methyl-4-(1,3-thiazol-4-ylmethoxy)phenyl]quinazolin-4-amine;
5-[(1*R*)-2-(Dimethylamino)-1-methylethoxy]-*N*-{3-methyl-4-[(5-methylisoxazol-3-yl)methoxy]phenyl}quinazolin-4-amine;
5-[2-(dimethylamino)-2-methylpropoxy]-*N*-[3-methyl-4-(1,3-thiazol-4-ylmethoxy)phenyl]quinazolin-4-amine;
5-[2-(Dimethylamino)ethoxy]-*N*-{3-methoxy-4-[(5-methylisoxazol-3-yl)methoxy]phenyl}quinazolin-4-amine;
5-[2-(Dimethylamino)ethoxy]-*N*-[3-methoxy-4-(pyrazin-2-ylmethoxy)phenyl]quinazolin-4-amine;
5-[2-(Dimethylamino)ethoxy]-*N*-[3-fluoro-4-(1,3-thiazol-5-ylmethoxy)phenyl]quinazolin-4-amine;
*N*-[3-Chloro-4-(pyridin-2-ylmethoxy)phenyl]-5-[(1*S*)-2-(dimethylamino)-1-methylethoxy]quinazolin-4-amine;
*N*-[3-Chloro-4-(pyridin-2-ylmethoxy)phenyl]-5-{[(2*S*)-2-(dimethylamino)propyl]oxy}quinazolin-4-amine;
*N*-[3-Chloro-4-(pyridin-2-ylmethoxy)phenyl]-5-{[(2*R*)-2-(dimethylamino)propyl]oxy}quinazolin-4-amine;
5-{2-[Allyl(methyl)amino]ethoxy}-*N*-[3-chloro-4-(pyridin-2-ylmethoxy)phenyl]quinazolin-4-amine;
2-[{2-[(4-{[3-Chloro-4-(pyridin-2-ylmethoxy)phenyl]amino}quinazolin-5-yl)oxy]ethyl}(ethyl)amino]ethanol;
*N*-[3-Chloro-4-(pyridin-2-ylmethoxy)phenyl]-5-{(1*S*)-2-[(2-methoxyethyl)(methyl)amino]-1-methylethoxy}quinazolin-4-amine;
*N*-[3-Chloro-4-(pyridin-2-ylmethoxy)phenyl]-5-{(1*R*)-2-[ethyl(methyl)amino]-1-methylethoxy}quinazolin-4-amine;
5-{(1*R*)-2-[Allyl(methyl)amino]-1-methylethoxy}-*N*-[3-chloro-4-(pyridin-2-ylmethoxy)phenyl]quinazolin-4-amine;
5-{(1*S*)-2-[Allyl(methyl)amino]-1-methylethoxy}-*N*-[3-chloro-4-(pyridin-2-ylmethoxy)phenyl]quinazolin-4-amine;
*N*-{3-Chloro-4-[(3-fluorobenzyl)oxy]phenyl}-5-{[(2*S*)-2-(dimethylamino)propyl]oxy}quinazolin-4-amine;
*N*-{3-Chloro-4-[(3-fluorobenzyl)oxy]phenyl}-5-{[(2*R*)-2-(dimethylamino)propyl]oxy}quinazolin-4-amine;
*N*-{3-Chloro-4-[(1-methyl-1*H*-imidazol-2-yl)thio]phenyl}-5-{[(2*S*)-2-(dimethylamino)propyl]oxy}quinazolin-4-amine;
*N*-{3-Chloro-4-[(1-methyl-1*H*-imidazol-2-yl)thio]phenyl}-5-{[(2*R*)-2-(dimethylamino)propyl]oxy}quinazolin-4-amine;
*N*-{3-Chloro-4-[(1-methyl-1*H*-imidazol-2-yl)thio]phenyl}-5-[(1*R*)-2-(dimethylamino)-1-methylethoxy]quinazolin-4-amine;
5-[2-(Dimethylamino)-1-methylethoxy]-*N*-(3-methoxy-4-phenoxyphenyl)quinazolin-4-amine;
5-[2-(Dimethylamino)-1-methylethoxy]-N-(3-methoxy-4-phenoxyphenyl)quinazolin-4-amine; and
*N*-[3-Chloro-4-(pyridin-2-ylmethoxy)phenyl]-5-[2-(dimethylamino)-1,1-dimethylethoxy]quinazolin-4-amine;
or a pharmaceutically acceptable salt thereof.

32. A pharmaceutical composition which comprises a quinazoline derivative of the formula I, or a pharmaceutically acceptable salt thereof, as defined in any one of claims 1 to 31 in association with a pharmaceutically-acceptable diluent or carrier.

33. A quinazoline derivative of the formula I, or a pharmaceutically-acceptable salt thereof, as defined in any one of claims 1 to 31 for use as a medicament.

34. A quinazoline derivative of the formula I, or a pharmaceutically acceptable salt thereof, as defined in any one of claims 1 to 31 for use in the production of an anti-proliferative effect which effect is produced alone or in part by inhibiting erbB2 receptor tyrosine kinase in a warm-blooded animal such as man.

35. A quinazoline derivative of the formula I, or a pharmaceutically acceptable salt thereof, as defined in any one of claims 1 to 31 for use in the production of an erbB2 receptor tyrosine kinase inhibitory effect in a warm-blooded animal such as man.

36. A quinazoline derivative of the formula I, or a pharmaceutically acceptable salt thereof, as defined in any one of claims 1 to 31 for use in the production of a selective erbB2 receptor tyrosine kinase inhibitory effect in a warm-blooded animal such as man.

37. A process for the preparation of a quinazoline derivative of the formula I, or a pharmaceutically acceptable salt thereof, as defined in claim 1 which comprises:
(a) the reaction, conveniently in the presence of a suitable base, of a quinazoline of the formula **II:** 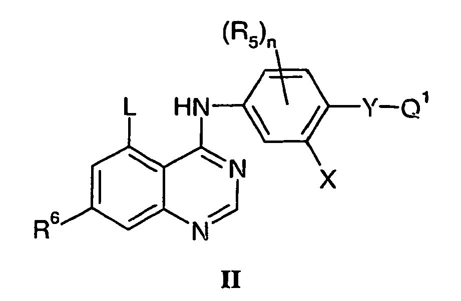 wherein R⁵, R⁶, Q¹, X, Y and n are as defined in claim 1 except that any functional group is protected if necessary, and L is a displaceable group, with an alcohol of the formula **III** 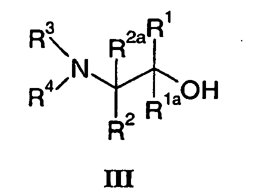 wherein R¹, R^{1a}, R², R^{2a} ,R³ and R⁴ are as defined in claim 1 except that any functional group is protected if necessary; or
(b) for the preparation of those compounds of the formula **I** wherein Y is OC(R⁷)₂, SC(R⁷)₂ or N(R⁷)C(R⁷)₂, the reaction, conveniently in the presence of a suitable base, of a quinazoline of the formula **IV:** 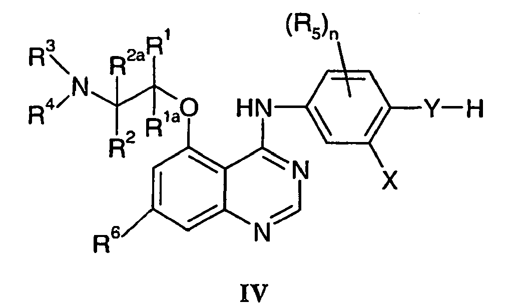 wherein Y is O, S or N(R⁷), and X, R¹, R^{1a}, R², R^{2a}, R³, R⁴, R⁵, R⁶, R⁷ and n are as defined in claim 1 except that any functional group is protected if necessary, with a compound of the formula **V:**
Q¹-C(R⁷)₂-L¹**V**
wherein L¹ is a suitable displaceable group and Q¹ and R⁷ are as defined in claim 1 except that any functional group is protected if necessary; or
(c) the reaction of a quinazoline of the formula **VI:** 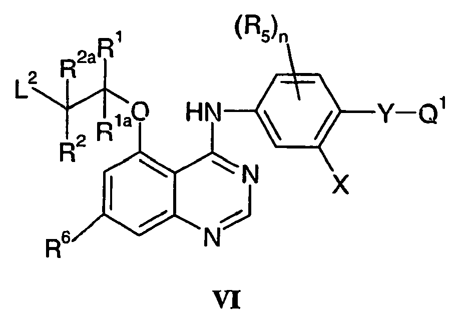 wherein L² is a suitable displaceable group and Q¹, X, Y, R¹, R^{1a}, R², R^{2a}, R⁵, R⁶ and n are as defined in claim 1 except that any functional group is protected if necessary, with an amine of the formula **VII:**
NHR³R⁴ **VII**
wherein R³ and R⁴ are as defined in claim 1 except that any functional group is protected if necessary; or
(d) for the preparation of those compounds of the formula **I** wherein R^{2a} is hydrogen, the reductive amination in the presence of a suitable reducing agent of the aldehyde or ketone of the formula **VIII:** 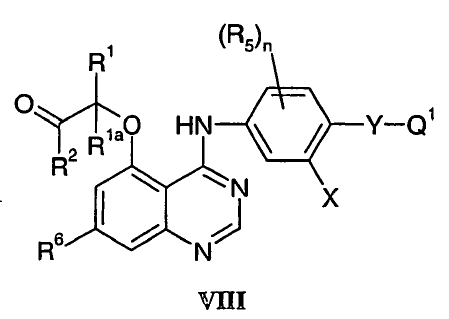 wherein Q¹, X, Y, R¹, R^{1a}, R², R⁵, R⁶ and n are as defined in claim 1 except that any functional group is protected if necessary, with an amine of the formula **VII:**
NHR³R⁴ **VII**
wherein R³ and R⁴ are as defined in claim 1 except that any functional group is protected if necessary; or
(e) for the preparation of those compounds of the formula **I** wherein Y is O or N(R⁷) and Q¹ is 2-pyridyl or 4-pyridyl the reaction, in the presence of a suitable catalyst, of a quinazoline of the formula **IV:** 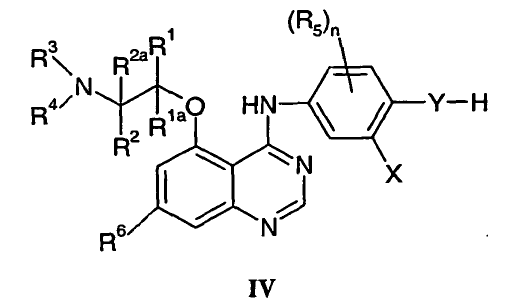 wherein Y is O or N(R⁷) and X, R¹, R^{1a}, R², R^{2a}, R³, R⁴, R⁵, R⁶ and n are as defined in claim 1 except that any functional group is protected if necessary, with an amine of the formula **IVa** or of the formula **IVb:** 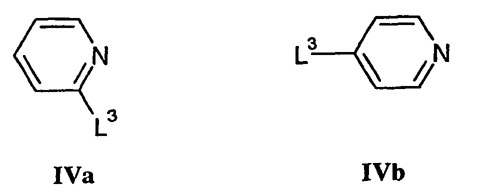 wherein L³ is a suitable displaceable group; or
(f) the reaction, conveniently in the presence of a suitable phosphine and a suitable diazo compound, of a quinazoline of the formula **II:** 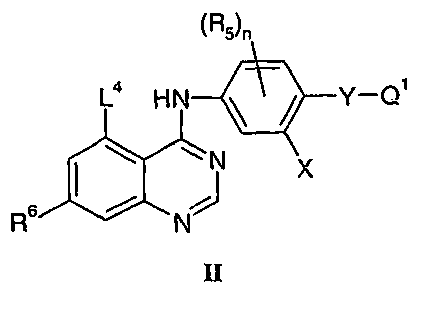 wherein R⁵, R⁶, Q¹, X, Y and n are as defined in claim 1 except that any functional group is protected if necessary, and L⁴ is hydroxy, with an alcohol of the formula **III:** 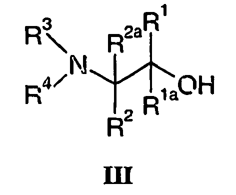 wherein R¹, R^{1a}, R², R^{2a}, R³ and R⁴ are as defined in claim 1 except that any functional group is protected if necessary; and thereafter, if necessary:
(i) converting a quinazoline derivative of the formula I into another quinazoline derivative of the formula I;
(ii) removing any protecting group that is present by conventional means;
(iii) forming a pharmaceutically acceptable salt.

## Patentansprüche

1. Chinazolinderivat der Formel I: 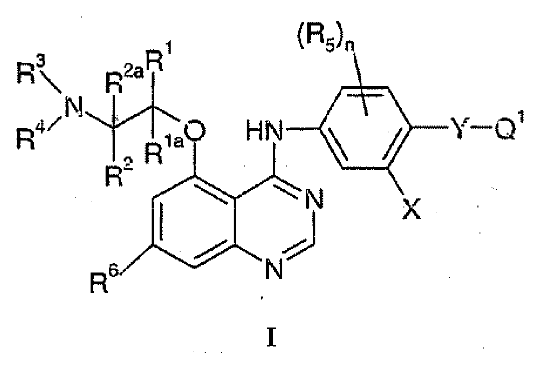 worin:
R¹ und R² gleich oder verschieden sein können und jeweils unter Wasserstoff, Carboxy, Cyano, Formyl, C₁₋₃-Alkyl, C₂₋₃-Alkanoyl, C₁₋₃-Alkoxycarbonyl, Carbamoyl, N-C₁₋₃-Alkylcarbamoyl und N,N-Di(C₁₋₃-alkyl)carbamoyl ausgewählt sind;
R^{1a} und R^{2a} gleich oder verschieden sein können und jeweils unter Wasserstoff und C₁₋₃-Alkyl ausgewählt sind;
R³ und R⁴ gleich oder verschieden sein können und jeweils unter Wasserstoff, C₁₋₃-Alkyl und C₂₋₄-Alkenyl ausgewählt sind; und worin jedes CH oder CH₂ oder CH₃ in R¹, R^{1a}, R², R^{2a}, R³ und R⁴ gegebenenfalls an jedem CH oder CH₂ oder CH₃ einen oder mehrere (beispielsweise 1, 2 oder 3) Halogensubstituenten oder einen unter Hydroxy, Cyano, C₁₋₃-Alkoxy, Amino, C₂₋₃-Alkanoyl, C₁₋₃-Alkylamino und Di(C₁₋₃-alkyl)amino ausgewählten Substituenten trägt;
X unter Wasserstoff, Halogen, C₁₋₄-Alkyl, C₁₋₄-Alkoxy, C₂₋₄-Alkenyl und C₂₋₄-Alkinyl ausgewählt ist;
R⁵ jeweils gleich oder verschieden sein kann und unter Halogen, Hydroxy, C₁₋₄-Alkyl, C₁₋₄-Alkoxy, C₂₋₄-Alkenyl und C₂₋₄-Alkinyl ausgewählt ist;
Y unter einer direkten Bindung, O, S, OC(R⁷)₂, SC(R⁷)₂, SO, SO₂, N(R⁷), CO und N(R⁷)C(R⁷)₂, worin R⁷ jeweils unabhängig voneinander für Wasserstoff oder C₁₋₆-Alkyl steht, ausgewählt ist;
Q¹ unter Phenyl, Pyridyl, Pyrazinyl, 1,3-Thiazolyl, 1H-Imidazolyl, 1H-Pyrazolyl, 1,3-Oxazolyl und
Isoxazolyl ausgewählt ist, und worin Q¹ gegebenenfalls einen oder mehrere Substituenten (beispielsweise 1, 2 oder 3) trägt, die gleich oder verschieden sind und unter Halogen, Cyano, Nitro, Hydroxy, Amino, Carboxy, Carbamoyl, Sulfamoyl, Formyl, Mercapto, C₁₋₆-Alkyl, C₂₋₈-Alkenyl, C₂₋₈-Alkinyl, C₁₋₆-Alkoxy, C₂₋₆-Alkenyloxy, C₂₋₆-Alkinyloxy, C₁₋₆-Alkylthio, C₁₋₆-Alkylsulfinyl, C₁₋₆-Alkylsulfonyl, C₁₋₆-Alkylamino, Di(C₁₋₆-alkyl)amino, C₁₋₆-Alkoxycarbonyl, N-C₁₋₆-Alkylcarbamoyl, N,N-Di(C₁₋₆-alkyl)carbamoyl, C₂₋₆
Alkanoyl, C₂₋₆-Alkanoyloxy, C₂₋₆-Alkanoylamino, N
C₁₋₆-Alkyl-C₂₋₆-alkanoylamino, C₃₋₆-Alkenoylamino, N-C₁₋₆-Alkyl-C₃₋₆-alkenoylamino, C₃₋₆-Alkinoylamino, N-C₁₋₆-Alkyl-C₃₋₆-alkinoylamino, N-C₁₋₆-Alkylsulfamoyl, N,N-Di(C₁₋₆-alkyl)sulfamoyl, C₁₋₆-Alkansulfonylamino und N-C₁₋₆-Alkyl-C₁₋₆-alkansulfonylamino oder einer Gruppe der Formel:
-X¹-R⁸
worin X¹ für eine direkte Bindung steht oder unter O, CO und N(R⁹), worin R⁹ Wasserstoff oder C₁₋₆-Alkyl bedeutet, ausgewählt ist und R⁸ für Halogen-C₁₋₆-alkyl, Hydroxy-C₁₋₆-alkyl, Carboxy-C₁₋₆-alkyl, C₁₋₆-Alkoxy-C₁₋₆-alkyl, Cyano-C₁₋₆-alkyl, Amino-C₁₋₆-alkyl, N-C₁₋₆-Alkylamino-C₁₋₆-alkyl, N,N-Di(C₁₋₆-alkyl)amino-C₁₋₆-alkyl, C₂₋₆-Alkanoylamino-C₁₋₆-alkyl, C₁₋₆-Alkoxycarbonylamino-C₁₋₆-alkyl, Carbamoyl-C₁₋₆-alkyl, N-C₁₋₆-Alkylcarbamoyl-C₁₋₆-alkyl, N,N-Di(C₁₋₆-alkyl)carbamoyl-C₁₋₆-alkyl, C₂₋₆-Alkanoyl-C₁₋₆-alkyl oder C₁₋₆-Alkoxycarbonyl-C₁₋₆-alkyl steht, ausgewählt sind; und worin jedes CH₂ oder CH₃ in einem Substituenten an Q¹ gegebenenfalls an jedem CH₂ oder CH₃ einen oder mehrere (beispielsweise 1, 2 oder 3) Halogen-oder C₁₋₆-Alkylsubstituenten oder einen unter Hydroxy, Cyano, Amino, C₁₋₄-Alkoxy, C₁₋₄-Alkylamino und Di(C₁-₄-alkyl)amino ausgewählten Substituenten trägt;
R⁶ unter Wasserstoff, C₁₋₆-Alkoxy, C₂₋₆-Alkenyloxy und C₂₋₆-Alkinyloxy ausgewählt ist, und worin jede CH₂- oder CH₃-Gruppe in einem Substituenten R⁶ gegebenenfalls an jeder CH₂- oder CH₃-Gruppe einen oder mehrere Halogen- oder C₁₋₆-Alkylsubstituenten oder einen unter Hydroxy und C₁₋₆-Alkoxy ausgewählten Substituenten trägt; n für 0, 1, 2 oder 3 steht; oder ein pharmazeutisch annehmbares Salz davon.

2. Chinazolinderivat der Formel I nach Anspruch 1,
worin R¹ unter Wasserstoff, Methyl und Ethyl ausgewählt ist, R² unter Wasserstoff, Carboxy, Cyano, Methyl, Ethyl, Acetyl, Methoxycarbonyl, Carbamoyl, N-Methylcarbamoyl und N,N-Dimethyl-carbamoyl ausgewählt ist und R^{1a} und R^{2a} jeweils für Wasserstoff stehen.

3. Chinazolinderivat der Formel I nach Anspruch 1,
worin R² unter Wasserstoff, Methyl und Ethyl ausgewählt ist, R¹ unter Wasserstoff, Carboxy, Cyano, Methyl, Ethyl, Acetyl, Methoxycarbonyl, Carbamoyl, N-Methylcarbamoyl und N,N-Dimethyl-carbamoyl ausgewählt ist und R^{1a} und R^{2a} jeweils für Wasserstoff stehen.

4. Chinazolinderivat der Formel I nach Anspruch 1,
worin R¹ und R^{1a} jeweils für Wasserstoff stehen, R² unter Wasserstoff, Carboxy, Cyano, Methyl, Ethyl, Acetyl, Methoxycarbonyl, Carbamoyl, N-Methylcarbamoyl und N,N-Dimethylcarbamoyl ausgewählt ist und R^{2a} unter Wasserstoff und C₁₋₃-Alkyl ausgewählt ist.

5. Chinazolinderivat der Formel I nach Anspruch 1,
worin R² und R^{2a} jeweils für Wasserstoff stehen, R¹ unter Wasserstoff, Carboxy, Cyano, Methyl, Ethyl, Acetyl, Methoxycarbonyl, Carbamoyl, N-Methylcarbamoyl und N,N-Dimethylcarbamoyl ausgewählt ist und R^{1a} unter Wasserstoff und C₁₋₃-Alkyl ausgewählt ist.

6. Chinazolinderivat der Formel I nach einem der Ansprüche 1, 2, 3 und 5, worin R¹ für Methyl steht und R², R^{1a} und R^{2a} jeweils für Wasserstoff stehen.

7. Chinazolinderivat der Formel I nach einem der Ansprüche 1 bis 4, worin R² für Methyl steht und R¹, R^{1a} und R^{2a} jeweils für Wasserstoff stehen.

8. Chinazolinderivat der Formel I nach Anspruch 1 oder Anspruch 5, worin R¹ und R^{1a} jeweils für Methyl stehen und R² und R^{2a} jeweils für Wasserstoff stehen.

9. Chinazolinderivat der Formel I nach Anspruch 1 oder Anspruch 4, worin R² und R^{2a} jeweils für Methyl stehen und R¹ und R^{1a} jeweils für Wasserstoff stehen.

10. Chinazolinderivat der Formel I nach einem der vorhergehenden Ansprüche, worin R³ und R⁴ gleich oder verschieden sein können und jeweils unter C₁₋₃-Alkyl ausgewählt sind, worin jedes CH oder CH₂ oder CH₃ in R³ und R⁴ gegebenenfalls an jedem CH oder CH₂ oder CH₃ einen oder mehrere unter Hydroxy und C₁₋₃-Alkoxy ausgewählte Substituenten trägt.

11. Chinazolinderivat der Formel I nach einem der Ansprüche 1 bis 9, worin R³ und R⁴ gleich oder verschieden sein können und jeweils unter Wasserstoff, Methyl, Ethyl, Propenyl, 2-Methoxyethyl und 2-Hydroxyethyl ausgewählt sind.

12. Chinazolinderivat der Formel I nach Anspruch 11,
worin R³ und R⁴ gleich oder verschieden sein können und jeweils unter Methyl, Ethyl, Propenyl, 2-Methoxyethyl und 2-Hydroxyethyl ausgewählt sind.

13. Chinazolinderivat der Formel I nach Anspruch 11 oder Anspruch 12, worin R³ für Methyl steht und R⁴ unter Methyl, Ethyl, Propenyl, 2-Methoxyethyl und 2-Hydroxyethyl ausgewählt ist.

14. Chinazolinderivat der Formel I nach einem der Ansprüche 10 bis 13, worin R³ und R⁴ jeweils für Methyl stehen.

15. Chinazolinderivat der Formel I nach einem der Ansprüche 10 bis 12, worin R³ für Ethyl steht und R⁴ für 2-Hydroxyethyl steht.

16. Chinazolinderivat der Formel I nach einem der vorhergehenden Ansprüche, worin X unter Wasserstoff, Halogen, C₁₋₄-Alkyl und C₁₋₄-Alkoxy ausgewählt ist.

17. Chinazolinderivat der Formel I nach Anspruch 16,
worin X unter Wasserstoff, Fluor, Chlor, Methyl und Methoxy ausgewählt ist.

18. Chinazolinderivat der Formel I nach Anspruch 16 oder Anspruch 17, worin X unter Methyl und Chlor ausgewählt ist.

19. Chinazolinderivat der Formel I nach Anspruch 18,
worin X für Chlor steht.

20. Chinazolinderivat der Formel I nach Anspruch 18,
worin X für Methyl steht.

21. Chinazolinderivat der Formel I nach einem der vorhergehenden Ansprüche, worin Y unter 0, S und OC(R⁷)₂, worin R⁷ jeweils unabhängig voneinander für Wasserstoff oder C₁₋₄-Alkyl steht, ausgewählt ist.

22. Chinazolinderivat der Formel I nach Anspruch 21,
worin Y unter 0, S und OCH₃ ausgewählt ist.

23. Chinazolinderivat der Formel I nach Anspruch 21 oder Anspruch 22, worin Y für O steht.

24. Chinazolinderivat der Formel I nach Anspruch 21 oder Anspruch 22, worin Y für S steht.

25. Chinazolinderivat der Formel I nach Anspruch 21 oder Anspruch 22, worin Y für OCH₂ steht.

26. Chinazolinderivat der Formel I nach einem der vorhergehenden Ansprüche, worin n für 0 steht.

27. Chinazolinderivat der Formel I nach einem der vorhergehenden Ansprüche, worin Q¹ unter Phenyl, 2-Pyridyl, 2-Pyrazinyl, 1,3-Thiazol-4-yl, 1,3-Thiazol-5-yl, 1H-Imidazol-2-yl und Isoxazol-3-yl ausgewählt ist und worin Q¹ gegebenenfalls einen oder mehrere Substituenten, die gleich oder verschieden sein können und wie in Anspruch 1 definiert sind, trägt.

28. Chinazolinderivat der Formel I nach Anspruch 27,
worin Q¹ unter Phenyl, 2-Pyridyl, 2-Pyrazinyl, 1,3-Thiazol-4-yl, 1,3-Thiazol-5-yl, 1H-Imidazol-2-yl und Isoxazol-3-yl ausgewählt ist und worin Q¹ gegebenenfalls einen oder mehrere Substituenten, die gleich oder verschieden sein können und unter Fluor und C₁₋₄-Alkyl ausgewählt sind, trägt.

29. Chinazolinderivat der Formel I nach Anspruch 27 oder Anspruch 28, worin Q¹ unter 3-Fluorphenyl, 2-Pyridyl, 2-Pyrazinyl, 1-Methyl-1H-imidazol-2-yl, 1,3-Thiazol-4-yl, 1,3-Thiazol-5-yl und 5-Methyl-3-isoxazolyl ausgewählt ist.

30. Chinazolinderivat der Formel I nach einem der vorhergehenden Ansprüche, worin R⁶ für Wasserstoff steht.

31. Chinazolinderivat nach Anspruch 1, ausgewählt unter einem oder mehreren der folgenden:
4-(3-Chlor-4-(2-pyridylmethoxy)anilino)-5-(2-dimethylaminoethoxy)chinazolin;
4-(3-Chlor-4-(2-pyridylmethoxy)anilino)-5-(2-dimethylamino-1-methylethoxy)chinazolin;
4-(3-Chlor-4-(1-methyl-1H-imidazol-2-ylthio)-anilino)-5-(2-dimethylaminoethoxy)chinazolin;
4-(3-Chlor-4-(1-methyl-1H-imidazol-2-ylthio)-anilino)-5-(2-dimethylamino-2-methylethoxy)-chinazolin;
4-(4-(3-Fluorbenzyloxy)anilino)-5-(2-dimethyl-aminoethoxy)chinazolin;
4-(4-(3-Fluorbenzyloxy)anilino)-5-(2-dimethylamino-1-methylethoxy)chinazolin;
4-(3-Chlor-4-(2-pyrazinylmethoxy)anilino)-5-(2-dimethylaminoethoxy)chinazolin;
4-(3-Chlor-4-(2-pyrazinylmethoxy)anilino)-5-(2-dimethylamino-1-methylethoxy)chinazolin;
4-(3-Chlor-4-(5-methylisoxazol-3-ylmethoxy)-anilino)-5-(2-dimethylaminoethoxy)chinazolin;
4-(3-Chlor-4-(5-methylisoxazol-3-ylmethoxy)-anilino)-5-(2-dimethylamino-1-methylethoxy)-chinazolin;
4-(3-Chlor-4-(3-fluorbenzyloxy)anilino)-5-(2-(N-ethyl-N-methylamino)ethoxy)chinazolin;
4-(3-Chlor-4-(3-fluorbenzyloxy)anilino)-5-(2-dimethylaminoethoxy)chinazolin;
4-(3-Chlor-4-(3-fluorbenzyloxy)anilino)-5-[2-(N-(2-hydroxyethyl)-N-methylamino)ethoxy]chinazolin;
4-(3-Chlor-4-(2-pyridylmethoxy)anilino)-5-(2-(N-ethyl-N-methylamino)ethoxy)chinazolin;
4-(3-Chlor-4-(2-pyridylmethoxy)anilino)-5-(2-(N-(2-hydroxyethyl)-N-methylamino)ethoxy)chinazolin;
4-(3-Chlor-4-(3-fluorbenzyloxy)anilino)-5-(2-dimethylamino-2-methylethoxy)chinazolin;
4-(3-Chlor-4-(2-pyridylmethoxy)anilino)-5-(2-dimethylamino-2-methylethoxy)chinazolin;
*N*-[3-Chlor-4-(1,3-thiazol-4-ylmethoxy)phenyl]-5-[2-(dimethylamino)ethoxy]chinazolin-4-amin;
*N*-[3-Chlor-4-(pyridin-2-yloxy)phenyl]-5-[2-(dimethylamino)ethoxy]chinazolin-4-amin;
*N*-[3-Chlor-4-(pyrazin-2-ylmethoxy)phenyl]-5-[(1*S*)-2-(dimethylamino)-1-methylethoxy]chinazolin-4-amin;
*N*-[3-Chlor-4-[(3-fluorbenzyl)oxy]phenyl]-5-[(1*S*)-2-(dimethylamino)-1-methylethoxy]chinazolin-4-amin;
*N*-[3-Chlor-4-(pyridin-2-ylmethoxy)phenyl]-5-[(1*R*)-2-(dimethylamino)-1-methylethoxy]chinazolin-4-amin;
*N*-[3-Chlor-4-(1,3-thiazol-4-ylmethoxy)phenyl]-5-[(1*R*)-2-(dimethylamino)-1-methylethoxy]chinazolin-4-amin;
*N*-[3-Chlor-4-(pyrazin-2-ylmethoxy)phenyl]-5-[(1*R*)-2-(dimethylamino)-1-methylethoxy]chinazolin-4-amin;
*N*-[3-Chlor-4-[(3-fluorbenzyl)oxy]phenyl]-5-[(1*R*)-2-(dimethylamino)-1-methylethoxy]chinazolin-4-amin;
*N*-[3-Chlor-4-(pyridin-2-ylmethoxy)phenyl]-5-[2-(dimethylamino)-2-methylpropoxy]chinazolin-4-amin;
*N*-[3-Chlor-4-(1,3-thiazol-4-ylmethoxy)phenyl]-5-[2-(dimethylamino)-2-methylpropoxy]chinazolin-4-amin;
*N*-[3-Chlor-4-[(5-methylisoxazol-3-yl)methoxy]-phenyl]-5-[2-(dimethylamino)-2-methylpropoxy]-chinazolin-4-amin;
5-[2-(Dimethylamino)ethoxy]-*N*-[3-methyl-4-(pyridin-2-ylmethoxy)phenyl]chinazolin-4-amin;
5-[2-(Dimethylamino)ethoxy]-*N*-[3-methyl-4-(1,3-thiazol-4-ylmethoxy)phenyl]chinazolin-4-amin;
5-[2-(Dimethylamino)ethoxy]-*N*-[3-methyl-4-[(5-methylisoxazol-3-yl)methoxy]phenyl]chinazolin-4-amin;
5-[(1*R*)-2-(Dimethylamino)-1-methylethoxy]-*N*-[3-methyl-4-(pyridin-2-ylmethoxy)phenyl]chinazolin-4-amin;
5-[(1*R*)-2-(Dimethylamino)-1-methylethoxy]-*N*-[3-methyl-4-(pyrazin-2-ylmethoxy)phenyl]chinazolin-4-amin;
5-[(1*R*)-2-(Dimethylamino)-1-methylethoxy]-*N*-[3-methyl-4-(1,3-thiazol-4-ylmethoxy)phenyl]-chinazolin-4-amin;
5-[(1*R*)-2-(Dimethylamino)-1-methylethoxy]-*N*-[3-methyl-4-[(5-methylisoxazol-3-yl)methoxy]phenyl]-chinazolin-4-amin;
5-[2-(Dimethylamino)-2-methylpropoxy]-*N*-[3-methyl-4-(1,3-thiazol-4-ylmethoxy)phenyl]chinazolin-4-amin;
5-[2-(Dimethylamino)ethoxy]-*N*-[3-methoxy-4-[(5-methylisoxazol-3-yl)methoxy]phenyl]chinazolin-4-amin;
5-[2-(Dimethylamino)ethoxy]-*N*-[3-methoxy-4-(pyrazin-2-ylmethoxy)phenyl]chinazolin-4-amin;
5-[2-(Dimethylamino)ethoxy]-*N*-[3-fluor-4-(1,3-thiazol-5-ylmethoxy)phenyl]chinazolin-4-amin;
*N*-[3-Chlor-4-(pyridin-2-ylmethoxy)phenyl]-5-[(1*S*)-2-(dimethylamino)-1-methylethoxy]chinazolin-4-amin;
*N*-[3-Chlor-4-(pyridin-2-ylmethoxy)phenyl]-5-{[(2*S*)-2-(dimethylamino)propyl]oxy}chinazolin-4-amin;
*N*-[3-Chlor-4-(pyridin-2-ylmethoxy)phenyl]-5-{[(2*R*)-2-(dimethylamino)propyl]oxy}chinazolin-4-amin;
5-{2-[Allyl(methyl)amino]ethoxy}-*N*-[3-chlor-4-(pyridin-2-ylmethoxy)phenyl]chinazolin-4-amin; 2-[{2-[(4-{[3-Chlor-4-(pyridin-2-ylmethoxy)-phenyl]amino}chinazolin-5-yl)oxy]ethyl}(ethyl)-amino]ethanol;
*N*-[3-Chlor-4-(pyridin-2-ylmethoxy)phenyl]-5-{(1*S*)-2-[(2-methoxyethyl(methyl)amino]-1-methylethoxy}-chinazolin-4-amin;
*N*-[3-Chlor-4-(pyridin-2-ylmethoxy)phenyl]-5-{(1*R*)-2-[ethyl(methyl)amino]-1-methylethoxy}chinazolin-4-amin;
5-{(1*R*)-2-[Allyl(methyl)amino]-1-methylethoxy}-*N-*[3-chlor-4-(pyridin-2-ylmethoxy)phenyl]chinazolin-4-amin;
5-{(1*S*)-2-[Allyl(methyl)amino]-1-methylethoxy}-*N-*[3-chlor-4-(pyridin-2-ylmethoxy)phenyl]chinazolin-4-amin;
*N*-[3-Chlor-4-[(3-fluorbenzyl)oxy]phenyl]-5-{[(2*S*)-2-(dimethylamino)propyl]oxy}chinazolin-4-amin;
*N*-[3-Chlor-4-[(3-fluorbenzyl)oxy]phenyl]-5-{[(2*R*)-2-(dimethylamino)propyl]oxy}chinazolin-4-amin;
*N*-[3-Chlor-4-[(1-methyl-1*H*-imidazol-2-yl)thio]-phenyl]-5-{[(2*S*)-2-(dimethylamino)propyl]oxy}-chinazolin-4-amin;
*N*-[3-Chlor-4-[(1-methyl-1*H*-imidazol-2-yl)thio]-phenyl]-5-{[(2*R*)-2-(dimethylamino)propyl]oxy}-chinazolin-4-amin;
*N*-[3-Chlor-4-[(1-methyl-1*H*-imidazol-2-yl)thio]-phenyl]-5-[(1*R*)-2-(dimethylamino)-1-methylethoxy]-chinazolin-4-amin;
5-[2-(Dimethylamino)-1-methylethoxy]-*N*-(3-methoxy-4-phenoxyphenyl)chinazolin-4-amin;
5-[2-(Dimethylamino)-1-methylethoxy]-*N*-(3-methoxy-4-phenoxyphenyl)chinazolin-4-amin und
*N*-[3-Chlor-4-(pyridin-2-ylmethoxy)phenyl]-5-[2-(dimethylamino)-1,1-dimethylethoxy]chinazolin-4-amin;
oder ein pharmazeutisch annehmbares Salz davon.

32. Pharmazeutische Zusammensetzung, die ein Chinazolinderivat der Formel I oder ein pharmazeutisch annehmbares Salz davon nach einem der Ansprüche 1 bis 31 zusammen mit einem pharmazeutisch annehmbaren Verdünnungsmittel oder Träger enthält.

33. Chinazolinderivat der Formel I oder ein pharmazeutisch annehmbares Salz davon nach einem der Ansprüche 1 bis 31 zur Verwendung als Arzneimittel.

34. Chinazolinderivat der Formel I oder ein pharmazeutisch annehmbares Salz davon nach einem der Ansprüche 1 bis 31 zur Verwendung bei der Hervorrufung einer antiproliferativen Wirkung, welche alleine oder zum Teil durch Inhibierung von erbB2-Rezeptor-Tyrosinkinase hervorgerufen wird, bei einem Warmblüter wie dem Menschen.

35. Chinazolinderivat der Formel I oder ein pharmazeutisch annehmbares Salz davon nach einem der Ansprüche 1 bis 31 zur Verwendung bei der Hervorrufung einer Hemmwirkung gegenüber erbB2-Rezeptor-Tyrosinkinase bei einem Warmblüter wie dem Menschen.

36. Chinazolinderivat der Formel I oder ein pharmazeutisch annehmbares Salz davon nach einem der Ansprüche 1 bis 31 zur Verwendung bei der Hervorrufung einer selektiven Hemmwirkung gegenüber erbB2-Rezeptor-Tyrosinkinase bei einem Warmblüter wie dem Menschen.

37. Verfahren zur Herstellung eines Chinazolinderivats der Formel I oder eines pharmazeutisch annehmbaren Salzes davon nach Anspruch 1, bei dem man:
(a) ein Chinazolin der Formel II: 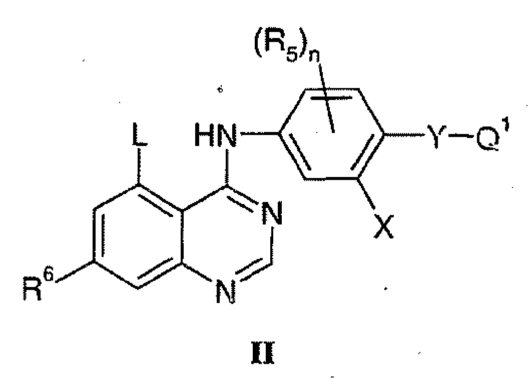 worin R⁵, R⁶, Q¹, X, Y und n wie in Anspruch 1 definiert sind, wobei jedoch jede funktionelle Gruppe gegebenenfalls geschützt ist und L für eine verdrängbare Gruppe steht, mit einem Alkohol der Formel III 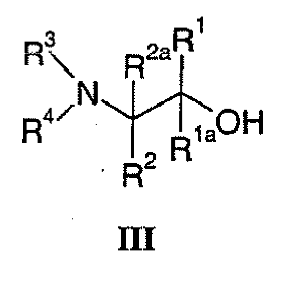 worin R¹, R^{1a}, R², R^{2a}, R³ und R⁴ wie in Anspruch 1 definiert sind, wobei jedoch jede funktionelle Gruppe gegebenenfalls geschützt ist, umsetzt, zweckmäßigerweise in Gegenwart einer geeigneten Base; oder
(b) zur Herstellung derjenigen Verbindungen der Formel I, worin Y für OC(R⁷)₂, SC(R⁷)₂ oder N(R⁷)C(R⁷)₂ steht, ein Chinazolin der Formel IV: 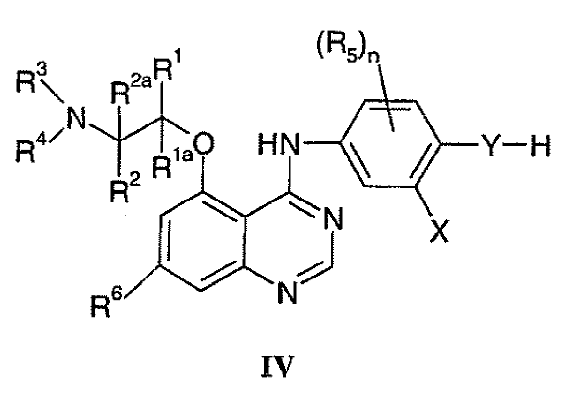 worin Y für 0, S oder N(R⁷) steht und X, R¹, R^{1a}, R², R^{2a}, R³, R⁴, R⁵, R⁶, R⁷ und n wie in Anspruch 1 definiert sind, wobei jedoch jede funktionelle Gruppe gegebenenfalls geschützt ist, mit einer Verbindung der Formel V:
Q¹-C(R⁷)₂-L¹ V
worin L¹ für eine geeignete verdrängbare Gruppe steht und Q¹ und R⁷ wie in Anspruch 1 definiert sind, wobei jedoch jede funktionelle Gruppe gegebenenfalls geschützt ist, umsetzt, zweckmäßigerweise in Gegenwart einer geeigneten Base; oder
(c) ein Chinazolin der Formel VI: 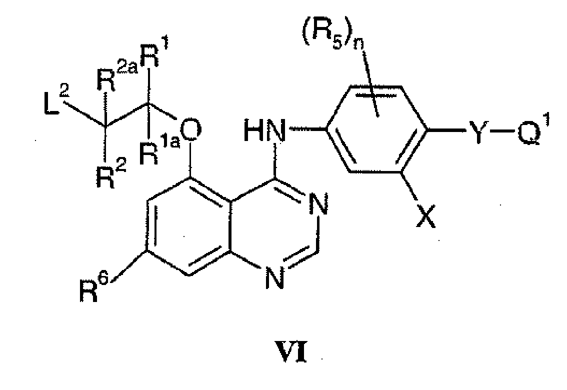 worin L² für eine geeignete verdrängbare Gruppe steht und Q¹, X, Y, R¹, R^{1a}, R², R^{2a}, R⁵, R⁶ und n wie in Anspruch 1 definiert sind, wobei jedoch jede funktionelle Gruppe gegebenenfalls geschützt ist, mit einem Amin der Formel VII:
NHR³R⁴ VII
worin R³ und R⁴ wie in Anspruch 1 definiert sind, wobei jedoch jede funktionelle Gruppe gegebenenfalls geschützt ist, umsetzt; oder
(d) zur Herstellung derjenigen Verbindungen der Formel I, worin R^{2a} für Wasserstoff steht, den Aldehyd bzw. das Keton der Formel VIII: 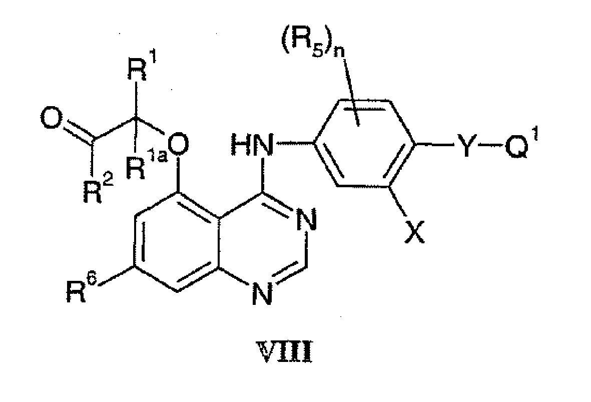 worin Q¹, X, Y, R¹, R^{1a}, R², R⁵, R⁶ und n wie in Anspruch 1 definiert sind, wobei jedoch jede funktionelle Gruppe gegebenenfalls geschützt ist, in Gegenwart eines Reduktionsmittels mit einem Amin der Formel VII:
NHR³R⁴ VII
worin R³ und R⁴ wie in Anspruch 1 definiert sind, wobei jedoch jede funktionelle Gruppe gegebenenfalls geschützt ist, reduktiv aminiert; oder
(e) zur Herstellung derjenigen Verbindungen der Formel I, worin Y für 0 oder N(R⁷) steht und Q¹ für 2-Pyridyl oder 4-Pyridyl steht, ein Chinazolin der Formel IV: 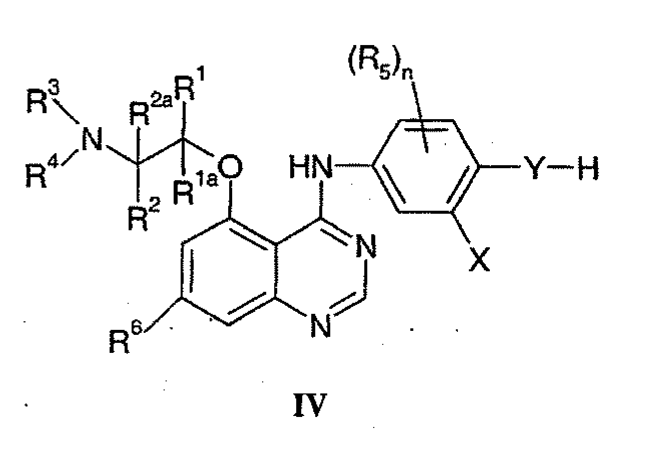 worin Y für O oder N(R⁷) steht und X, R¹, R^{1a}, R², R^{2a}, R³, R⁴, R⁵, R⁶ und n wie in Anspruch 1 definiert sind, wobei jedoch jede funktionelle Gruppe gegebenenfalls geschützt ist, in Gegenwart eines geeigneten Katalysators mit einem Amin der Formel IVa oder der Formel IVb: 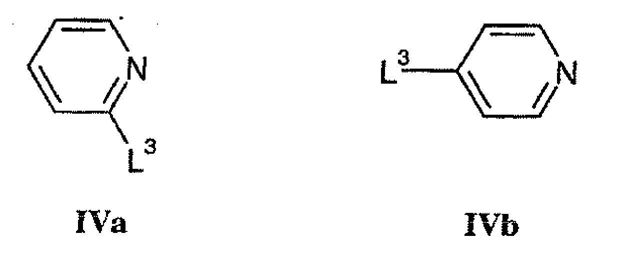 worin L³ für eine geeignete verdrängbare Gruppe steht, umsetzt; oder
(f) ein Chinazolin der Formel II: 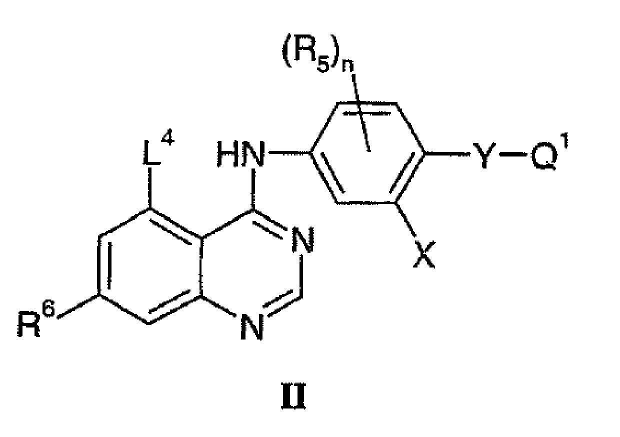 worin R⁵, R⁶, Q¹, X, Y und n wie in Anspruch 1 definiert sind, wobei jedoch jede funktionelle Gruppe gegebenenfalls geschützt ist, und L⁴ für Hydroxy steht, mit einem Alkohol der Formel III: 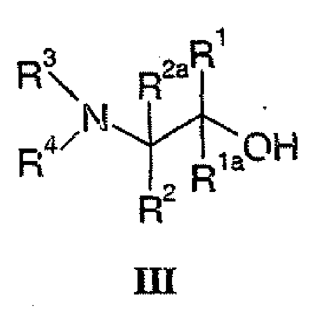 worin R¹, R^{1a}, R², R^{2a}, R³ und R⁴ wie in Anspruch 1 definiert sind, wobei jedoch jede funktionelle Gruppe gegebenenfalls geschützt ist, umsetzt, zweckmäßigerweise in Gegenwart eines geeigneten Phosphins und einer geeigneten Diazoverbindung; und danach gegebenenfalls:
(i) ein Chinazolinderivat der Formel I in ein anderes Chinazolinderivat der Formel I umwandelt;
(ii) jede vorhandene Schutzgruppe mit herkömmlichen Mitteln abspaltet;
(iii) ein pharmazeutisch annehmbares Salz bildet.

## Revendications

1. Dérivé de la quinazoline de formule I : 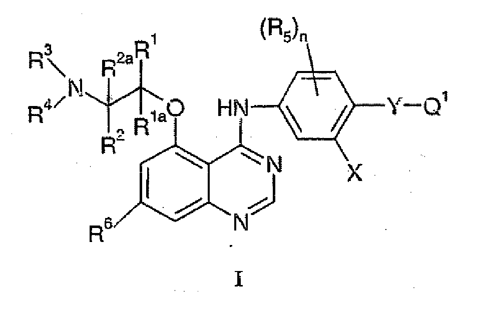 dans laquelle :
**chacun de R¹ et R²,** qui peuvent être identiques ou différents, est choisi parmi hydrogène, carboxy, cyano, formyle, (1-3C)alkyle, (2-3C)alcanoyle, (1-3C)alcoxycarbonyle, carbamoyle, N-(1-3C)alkylcarbamoyle et N,N-di-[(1-3C)alkyl]carbamoyle ;
**chacun de R^{1a} et R^{2a},** qui peuvent être identiques ou
différents, est choisi parmi hydrogène et (1-3C)alkyle ;
**chacun de R³ et R⁴,** qui peuvent être identiques ou différents, est choisi parmi hydrogène, (1-3C)alkyle et (2-4C)alcényle ;
et où tout CH ou CH₂ ou CH₃ dans l'un quelconque de R¹, R^{1a}, R², R^{2a}, R³ et R⁴ porte éventuellement sur chaque dit CH ou CH₂ ou CH₃ un ou plusieurs (par exemple 1, 2 ou 3) substituants halogéno ou un substituant choisi parmi hydroxy, cyano, (1-3C)alcoxy, amino, (2-3C)alcanoyle, (1-3C)alkylamino et di-[(1-3C)alkyl]amino ; **X** est choisi parmi hydrogène, halogéno, (1-4C)alkyle, (1-4C)alcoxy, (2-4C)alcényle et (2-4C)alcynyle ;
**chaque R⁵,** qui peut être identique ou différent, est choisi parmi halogéno, hydroxy, (1-4C)alkyle, (1-4C)alcoxy, (2-4C)alcényle et (2-4C)alcynyle ; **Y** est choisi parmi une liaison directe, 0, S, OC(R⁷)₂, SC(R⁷)₂, SO, SO₂, N(R⁷), CO et N(R⁷)C(R⁷)₂
où chaque R⁷ est, indépendamment, hydrogène ou (1-6C)alkyle ;
**Q¹** est choisi parmi phényle, pyridyle, pyrazinyle, 1,3-thiazolyle, 1H-imidazolyle, 1H-pyrazolyle, 1,3-oxazolyle et isoxazolyle, et où Q¹ porte éventuellement un ou plusieurs substituants (par exemple 1, 2 ou 3), qui peuvent être identiques ou différents, choisis parmi halogéno, cyano, nitro, hydroxy, amino, carboxy, carbamoyle, sulfamoyle, formyle, mercapto, (1-6C)alkyle, (2-8C)alcényle, (2-8C)alcynyle, (1-6C) alcoxy, (2-6C) alcényloxy, (2-6C) alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di-[(1-6C)alkyl)carbamoyle, (2-6C)alcanoyle, (2-6C)alcanoyloxy, (2-6C)alcanoylamino, N-(1-6C)alkyl-(2-6C)alcanoylamino, (3-6C)alcénoylamino, N-(1-6C)alkyl-(3-6C)alcénoylamino, (3-6C)alcynoylamino, N-(1-6C)alkyl-(3-6C)alcynoylamino, N-(1-6C)alkylsulfamoyle, N,N-di-[(1-6C)alkyl]sulfamoyle, (1-6C)alcanesulfonylamino et N-(1-6C)alkyl-(1-6C)alcanesulfonylamino, ou parmi un groupement de formule :
-X¹-R⁸
dans laquelle X¹ est une liaison directe ou est choisi parmi 0, CO et N(R⁹), où R⁹ est hydrogène ou (1-6C)alkyle, et R⁸ est halogéno-(1-6C)alkyle, hydroxy-(1-6C)alkyle, carboxy-(1-6C)alkyle, (1-6C)alcoxy-(1-6C)alkyle, cyano-(1-6C)alkyle, amino-(1-6C)alkyle, N-(1-6C)alkylamino-(1-6C)alkyle, N,N-di-[(1-6C)alkyl]amino-(1-6C)alkyle, (2-6C)alcanoylamino-(1-6C)alkyle, (1-6C)alcoxycarbonylamino-(1-6C)alkyle, carbamoyl-(1-6C)alkyle, N-(1-6C)alkylcarbamoyl-(1-6C)alkyle, N,N-di-[(1-6C)alkyl]carbamoyl-(1-6C)alkyle, (2-6C)alcanoyl-(1-6C)alkyle, ou (1-6C)alcoxycarbonyl-(1-6C)alkyle, et où tout CH₂ ou CH₃ dans un substituant sur Q¹ porte éventuellement sur chaque dit CH₂ ou CH₃ un
ou plusieurs (par exemple 1, 2 ou 3) substituants halogéno ou (1-6C)alkyle ou un substituant choisi parmi hydroxy, cyano, amino, (1-4C)alcoxy, (1-4C)alkylamino et di-[(1-4C)alkyl]amino ; **R⁶** est choisi parmi hydrogène, (1-6C)alcoxy, (2-6C)alcényloxy et (2-6C)alcynyloxy, et où tout groupement CH₂ ou CH₃ dans un substituant R⁶ porte éventuellement sur chaque dit groupement CH₂ ou CH₃ un ou plusieurs substituants halogéno ou (1-6C)alkyle, ou un substituant choisi parmi hydroxy et (1-6C) alcoxy ;
**n** est 0, 1, 2 ou 3 ;
ou un sel pharmaceutiquement acceptable de celui-ci .

2. Dérivé de la quinazoline de formule I tel que défini dans la revendication 1, dans laquelle R¹ est choisi parmi hydrogène, méthyle et éthyle, R² est choisi parmi hydrogène, carboxy, cyano, méthyle, éthyle, acétyle, méthoxycarbonyle, carbamoyle, N-méthylcarbamoyle et N,N-diméthylcarbamoyle et R^{1a} et R^{2a} sont chacun hydrogène.

3. Dérivé de la quinazoline de formule I tel que défini dans la revendication 1, dans laquelle R² est choisi parmi hydrogène, méthyle et éthyle, R¹ est choisi parmi hydrogène, carboxy, cyano, méthyle, éthyle, acétyle, méthoxycarbonyle, carbamoyle, N-méthylcarbamoyle et N,N-diméthylcarbamoyle et R^{1a} et R^{2a} sont chacun hydrogène.

4. Dérivé de la quinazoline de formule I tel que défini dans la revendication 1, dans laquelle R¹ et R^{1a} sont chacun hydrogène, R² est choisi parmi hydrogène, carboxy, cyano, méthyle, éthyle, acétyle, méthoxycarbonyle, carbamoyle, N-méthylcarbamoyle et N,N-diméthylcarbamoyle et R^{2a} est choisi parmi hydrogène et (1-3C)alkyle.

5. Dérivé de la quinazoline de formule I tel que défini dans la revendication 1, dans laquelle R² et R^{2a} sont chacun hydrogène, R¹ est choisi parmi hydrogène, carboxy, cyano, méthyle, éthyle, acétyle, méthoxycarbonyle, carbamoyle, N-méthylcarbamoyle et N,N-diméthylcarbamoyle et R^{1a} est choisi parmi hydrogène et (1-3C)alkyle.

6. Dérivé de la quinazoline de formule I tel que défini dans l'une quelconque des revendications 1, 2, 3 et 5, dans laquelle R¹ est méthyle, et R², R^{1a} et R^{2a} sont chacun hydrogène.

7. Dérivé de la quinazoline de formule I tel que défini dans l'une quelconque des revendications 1 à 4, dans laquelle R² est méthyle et R¹, R^{1a} et R^{2a} sont chacun hydrogène.

8. Dérivé de la quinazoline de formule I tel que défini dans la revendication 1 ou la revendication 5, dans laquelle R¹ et R^{1a} sont chacun méthyle et R² et R^{2a} sont chacun hydrogène.

9. Dérivé de la quinazoline de formule I tel que défini dans la revendication 1 ou la revendication 4, dans laquelle R² et R^{2a} sont chacun méthyle et R¹ et R^{1a} sont chacun hydrogène.

10. Dérivé de la quinazoline de formule I tel que défini dans l'une quelconque des revendications précédentes, dans laquelle chacun de R³ et R⁴, qui peuvent être identiques ou différents, est choisi parmi (1-3C)alkyle, où tout CH ou CH₂ ou CH₃ dans l'un quelconque de R³ et R⁴ porte éventuellement sur chaque dit CH ou CH₂ ou CH₃ un ou plusieurs substituants choisis parmi hydroxy et (1-3C)alcoxy.

11. Dérivé de la quinazoline de formule I tel que défini dans l'une quelconque des revendications 1 à 9, dans laquelle chacun de R³ et R⁴, qui peuvent être identiques ou différents, est choisi parmi hydrogène, méthyle, éthyle, propényle, 2-méthoxyéthyle et 2-hydroxyéthyle.

12. Dérivé de la quinazoline de formule I tel que défini dans la revendication 11, dans laquelle chacun de R³ et R⁴, qui peuvent être identiques ou différents, est choisi parmi méthyle, éthyle, propényle, 2-méthoxyéthyle et 2-hydroxyéthyle.

13. Dérivé de la quinazoline de formule I tel que défini dans la revendication 11 ou la revendication 12, dans laquelle R³ est méthyle et R⁴ est choisi parmi méthyle, éthyle, 2-hydroxyéthyle, 2-méthoxyéthyle et propényle.

14. Dérivé de la quinazoline de formule I tel que défini dans l'une quelconque des revendications 10 à 13, dans laquelle R³ et R⁴ sont chacun méthyle.

15. Dérivé de la quinazoline de formule I tel que défini dans l'une quelconque des revendications 10 à 12, dans laquelle R³ est éthyle et R⁴ est 2-hydroxyéthyle.

16. Dérivé de la quinazoline de formule I tel que défini dans l'une quelconque des revendications précédentes, dans laquelle X est choisi parmi hydrogène, halogéno, (1-4C)alkyle et (1-4C)alcoxy.

17. Dérivé de la quinazoline de formule I tel que défini dans la revendication 16, dans laquelle X est choisi parmi hydrogène, fluoro, chloro, méthyle et méthoxy.

18. Dérivé de la quinazoline de formule I tel que défini dans la revendication 16 ou la revendication 17, dans laquelle X est choisi parmi méthyle et chloro.

19. Dérivé de la quinazoline de formule I tel que défini dans la revendication 18, dans laquelle X est chloro.

20. Dérivé de la quinazoline de formule I tel que défini dans la revendication 18, dans laquelle X est méthyle.

21. Dérivé de la quinazoline de formule I tel que défini dans l'une quelconque des revendications précédentes, dans laquelle Y est choisi parmi 0, S et OC(R⁷)₂ où chaque R⁷ est, indépendamment, hydrogène ou (1-4C) alkyle.

22. Dérivé de la quinazoline de formule I tel que défini dans la revendication 21, dans laquelle Y est choisi parmi 0, S et OCH₂.

23. Dérivé de la quinazoline de formule I tel que défini dans la revendication 21 ou la revendication 22, dans laquelle Y est O.

24. Dérivé de la quinazoline de formule I tel que défini dans la revendication 21 ou la revendication 22, dans laquelle Y est S.

25. Dérivé de la quinazoline de formule I tel que défini dans la revendication 21 ou la revendication 22, dans laquelle Y est OCH₂.

26. Dérivé de la quinazoline de formule I tel que défini dans l'une quelconque des revendications précédentes, dans laquelle n est 0.

27. Dérivé de la quinazoline de formule I tel que défini dans l'une quelconque des revendications précédentes, dans laquelle Q¹ est choisi parmi phényle, 2-pyridyle, 2-pyrazinyle, 1,3-thiazol-4-yle, 1,3-thiazol-5-yle, 1H-imidazol-2-yle et isoxazol-3-yle et dans laquelle Q¹ porte éventuellement un ou plusieurs substituants, qui peuvent être identiques ou différents, tels que définis dans la revendication 1.

28. Dérivé de la quinazoline de formule I tel que défini dans la revendication 27, dans laquelle Q¹ est choisi parmi phényle, 2-pyridyle, 2-pyrazinyle, 1,3-thiazol-4-yle, 1,3-thiazol-5-yle, 1H-imidazol-2-yle et 3- isoxazolyle et dans laquelle Q¹ porte éventuellement un ou plusieurs substituants, qui peuvent être identiques ou différents, choisis parmi fluoro et (1-4C)alkyle.

29. Dérivé de la quinazoline de formule I tel que défini dans la revendication 27 ou la revendication 28, dans laquelle Q¹ est choisi parmi 3-fluorophényle, 2-pyridyle, 2-pyrazinyle, 1-méthyl-1H-imidazol-2-yle, 1,3-thiazol-4-yle, 1,3-thiazol-5-yle et 5-méthyl-3-isoxazolyle.

30. Dérivé de la quinazoline de formule I tel que défini dans l'une quelconque des revendications précédentes, dans laquelle R⁶ est hydrogène.

31. Dérivé de la quinazoline selon la revendication 1, choisi parmi un ou plusieurs de ce qui suit :
la 4-(3-chloro-4-(2-pyridylméthoxy)anilino)-5-(2-diméthylaminoéthoxy)quinazoline ;
la 4-(3-chloro-4-(2-pyridylméthoxy)anilino)-5-(2-diméthylamino-1-méthyléthoxy)quinazoline ;
la 4-(3-chloro-4-(1-méthyl-1*H*-imidazol-2-ylthio)anilino)-5-(2-diméthylaminoéthoxy)quinazoline ;
la 4-(3-chloro-4-(1-méthyl-1*H*-imidazol-2-ylthio)anilino)-5-(2-diméthylamino-2-méthyléthoxy)quinazoline ;
la 4-(4-(3-fluorobenzyloxy)anilino)-5-(2-diméthylaminoéthoxy)quinazoline ;
la 4-(4-(3-fluorobenzyloxy)anilino)-5-(2-diméthylamino-1-méthyléthoxy)quinazoline ;
la 4-(3-chloro-4-(2-pyrazinylméthoxy)anilino)-5-(2-diméthylaminoéthoxy)quinazoline ;
la 4-(3-chloro-4-(2-pyrazinylméthoxy)anilino)-5-(2-diméthylamino-1-méthyléthoxy)quinazoline ;
la 4-(3-chloro-4-(5-méthylisoxazol-3-ylméthoxy)anilino)-5-(2-diméthylaminoéthoxy)quinazoline ;
la 4-(3-chloro-4-(5-méthylisoxazol-3-ylméthoxy)anilino)-5-(2-diméthylamino-1-méthyléthoxy)quinazoline ;
la 4-(3-chloro-4-(3-fluorobenzyloxy)anilino)-5-(2-(N-éthyl-N-méthylamino)éthoxy)quinazoline ;
la 4-(3-chloro-4-(3-fluorobenzyloxy)anilino)-5-(2-diméthylaminoéthoxy)quinazoline ;
la 4-(3-chloro-4-(3-fluorobenzyloxy)anilino)-5-[2-(N-(2-hydroxyéthyl)-N-méthylamino)éthoxy]quinazoline ;
la 4-(3-chloro-4-(2-pyridylméthoxy)anilino)-5-(2-(N-éthyl-N-méthylamino)éthoxy)quinazoline ;
la 4-(3-chloro-4-(2-pyridylméthoxy)anilino)-5-(2-(N-(2-hydroxyéthyl)-N-méthylamino)éthoxy)quinazoline ;
la 4-(3-chloro-4-(3-fluorobenzyloxy)anilino)-5-(2-diméthylamino-2-méthyléthoxy)quinazoline ;
la 4-(3-chloro-4-(2-pyridylméthoxy)anilino)-5-(2-diméthylamino-2-méthyléthoxy)quinazoline ;
la *N*-[3-chloro-4-(1,3-thiazol-4-ylméthoxy)phényl]-5-[2-(diméthylamino)éthoxy]quinazolin-4-amine ;
la *N*-[3-chloro-4-(pyridin-2-yloxy)phényl]-5-[2-(diméthylamino)éthoxy]quinazolin-4-amine ;
la *N*-[3-chloro-4-(pyrazin-2-ylméthoxy)phényl]-5-[(1*S*)-2-(diméthylamino)-1-méthyléthoxy]quinazolin-4-amine ;
la *N*-{3-chloro-4-[(3-fluorobenzyl)oxy]phényl}-5-[(1*S*)-2-(diméthylamino)-1-méthyléthoxy]quinazolin-4-amine ;
la *N*-[3-chloro-4-(pyridin-2-ylméthoxy)phényl]-5-[(1*R*)-2-(diméthylamino)-1-méthyléthoxy]quinazolin-4-amine ;
la *N*-[3-chloro-4-(1,3-thiazol-4-ylméthoxy)phényl]-5-[(1*R*)-2-(diméthylamino)-1-méthyléthoxy]quinazolin-4-amine ;
la *N*-[3-chloro-4-(pyrazin-2-ylméthoxy)phényl]-5-[(1*R*)-2-(diméthylamino)-1-méthyléthoxy]quinazolin-4-amine ;
la *N*-{3-chloro-4-[(3-fluorobenzyl)oxy]phényl}-5-[(1*R*)-2-(diméthylamino)-1-méthyléthoxy]quinazolin-4-amine ;
la *N*-[3-chloro-4-(pyridin-2-ylméthoxy)phényl]-5-[2-(diméthylamino)-2-méthylpropoxy]quinazolin-4-amine ;
la *N*-[3-chloro-4-(1,3-thiazol-4-ylméthoxy)phényl]-5-[2-(diméthylamino)-2-méthylpropoxy]quinazolin-4-amine ;
la *N*-{3-chloro-4-[(5-méthylisoxazol-3-yl)méthoxy]phényl}-5-[2-(diméthylamino)-2-méthylpropoxy]quinazolin-4-amine ;
la 5-[2-(diméthylamino)éthoxy]-*N*-[3-méthyl-4-(pyridin-2-ylméthoxy)phényl]quinazolin-4-amine ;
la 5-[2-(diméthylamino)éthoxy]-*N*-[3-méthyl-4-(1,3-thiazol-4-ylméthoxy)phényl]quinazolin-4-amine ;
la 5-[2-(diméthylamino)éthoxy]-*N*-{3-méthyl-4-[(5-méthylisoxazol-3-yl)méthoxy]phényl}quinazolin-4-amine ;
la 5-[(1*R*)-2-(diméthylamino)-1-méthyléthoxy]-*N*-[3-méthyl-4-(pyridin-2-ylméthoxy)phényl]quinazolin-4-amine ;
la 5-[(1*R*)-2-(diméthylamino)-1-méthyléthoxy]-*N*-[3-méthyl-4-(pyrazin-2-ylméthoxy)phényl]quinazolin-4-amine ;
la 5-[(1*R*)-2-(diméthylamino)-1-méthyléthoxy]-*N*-[3-méthyl-4-(1,3-thiazol-4-ylméthoxy)phényl]quinazolin-4-amine ;
la 5-[(1*R*)-2-(diméthylamino)-1-méthyléthoxy]-*N*-{3-méthyl-4-[(5-méthylisoxazol-3-yl)méthoxy]phényl}quinazolin-4-amine ;
la 5-[2-(diméthylamino)-2-méthylpropoxy]-*N*-[3-méthyl-4-(1,3-thiazol-4-ylméthoxy)phényl]quinazolin-4-amine ;
la 5-[2-(diméthylamino)éthoxy]-*N*-{3-méthoxy-4-[(5-méthylisoxazol-3-yl)méthoxy]phényl}quinazolin-4-amine ;
la 5-[2-(diméthylamino)éthoxy]-*N*-[3-méthoxy-4-(pyrazin-2-ylméthoxy)phényl]quinazolin-4-amine ; la 5-[2-(diméthylamino)éthoxy]-*N*-[3-fluoro-4-(1,3-thiazol-5-ylméthoxy)phényl]quinazolin-4-amine ;
la *N*-[3-chloro-4-(pyridin-2-ylméthoxy)phényl]-5-[(1*S*)-2-(diméthylamino)-1-méthyléthoxy]quinazolin-4-amine ;
la *N*-[3-chloro-4-(pyridin-2-ylméthoxy)phényl]-5-{[(2*S*)-2-(diméthylamino)propyl]oxy}quinazolin-4-amine ;
la *N*-[3-chloro-4-(pyridin-2-ylméthoxy)phényl]-5-{[(2*R*)-2-(diméthylamino)propyl]oxy}quinazolin-4-amine ;
la 5-{2-[allyl(méthyl)amino]éthoxy}-*N*-[3-chloro-4-(pyridin-2-ylméthoxy)phényl]quinazolin-4-amine ;
le 2-[{2-[(4-{[3-chloro-4-(pyridin-2-ylméthoxy)phényl]amino}quinazolin-5-yl)oxy]éthyl}(éthyl)amino]éthanol ;
la *N*-[3-chloro-4-(pyridin-2-ylméthoxy)phényl]-5-{(1*S*)-2-[(2-méthoxyéthyl(méthyl)amino]-1-méthyléthoxy}quinazolin-4-amine ;
la *N*-[3-chloro-4-(pyridin-2-ylméthoxy)phényl]-5-{(1*R*)-2-[éthyl(méthyl)amino]-1-méthyléthoxy}quinazolin-4-amine ;
la 5-{(1*R*)-2-[allyl(méthyl)amino]-1-méthyléthoxy}-*N*-[3-chloro-4-(pyridin-2-ylméthoxy)phényl]quinazolin-4-amine ;
la 5-{(1*S*)-2-[allyl(méthyl)amino]-1-méthyléthoxy}-*N*-[3-chloro-4-(pyridin-2-ylméthoxy)phényl]quinazolin-4-amine ;
la *N*-{3-chloro-4-[(3-fluorobenzyl)oxy]phényl}-5-{[(2*S*)-2-(diméthylamino)propyl]oxy}quinazolin-4-amine ;
la *N*-{3-chloro-4-[(3-fluorobenzyl)oxy]phényl}-5-{[(2*R*)-2-(diméthylamino)propyl]oxy}quinazolin-4-amine ;
la *N*-{3-chloro-4-[(1-méthyl-1*H*-imidazol-2-yl)thio]phényl}-5-{[(2*S*)-2-(diméthylamino)propyl]oxy}quinazolin-4-amine ;
la *N*-{3-chloro-4-[(1-méthyl-1*H*-imidazol-2-yl)thio]phényl}-5-{[(2*R*)-2-(diméthylamino)propyl]oxy}quinazolin-4-amine ;
la *N*-{3-chloro-4-[(1-méthyl-1*H*-imidazol-2-yl)thio]phényl}-5-[(1*R*)-2-(diméthylamino)-1-méthyléthoxy]quinazolin-4-amine ;
la 5-[2-(diméthylamino)-1-méthyléthoxy]-*N*-(3-méthoxy-4-phénoxyphényl)quinazolin-4-amine ;
la 5-[2-(diméthylamino)-1-méthyléthoxy]-*N*-(3-méthoxy-4-phénoxyphényl)quinazolin-4-amine et
la *N*-[3-chloro-4-(pyridin-2-ylméthoxy)phényl]-5-[2-(diméthylamino)-1,1-diméthyléthoxy]quinazolin-4-amine ;
ou un sel pharmaceutiquement acceptable de ceux-ci.

32. Composition pharmaceutique qui comprend un dérivé de la quinazoline de formule I, ou un sel pharmaceutiquement acceptable de celui-ci, tel que défini dans l'une quelconque des revendications 1 à 31, en association avec un diluant ou un support pharmaceutiquement acceptable.

33. Dérivé de la quinazoline de formule I, ou un sel pharmaceutiquement acceptable de celui-ci, tel que défini dans l'une quelconque des revendications 1 à 31, destiné à être utilisé comme médicament.

34. Dérivé de la quinazoline de formule I, ou un sel pharmaceutiquement acceptable de celui-ci, tel que défini dans l'une quelconque des revendications 1 à 31, destiné à être utilisé dans la production d'un effet anti-prolifératif, lequel effet est produit uniquement ou en partie par l'inhibition du récepteur tyrosine kinase erbB2 chez un animal à sang chaud tel que l'homme.

35. Dérivé de la quinazoline de formule I, ou un sel pharmaceutiquement acceptable de celui-ci, tel que défini dans l'une quelconque des revendications 1 à 31, destiné à être utilisé dans la production d'un effet inhibiteur du récepteur tyrosine kinase erbB2 chez un animal à sang chaud tel que l'homme.

36. Dérivé de la quinazoline de formule I, ou un sel pharmaceutiquement acceptable de celui-ci, tel que défini dans l'une quelconque des revendications 1 à 31, destiné à être utilisé dans la production d'un effet inhibiteur du récepteur tyrosine kinase erbB2 sélectif chez un animal à sang chaud tel que l'homme.

37. Procédé de préparation d'un dérivé de la quinazoline de formule I, ou d'un sel pharmaceutiquement acceptable de celui-ci, tel que défini dans la revendication 1, qui comprend :
(a) la réaction, commodément en présence d'une base convenable, d'une quinazoline de formule **II** : 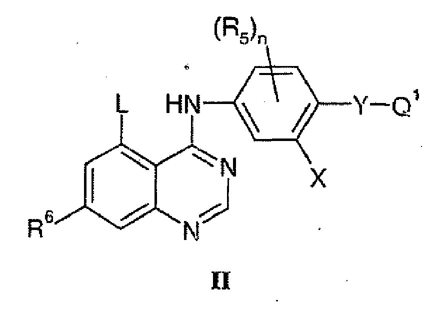 dans laquelle R⁵, R⁶, Q¹, X, Y et n sont tels que définis dans la revendication 1 sauf que toute fonction est protégée le cas échéant, et L est un groupement déplaçable, avec un alcool de formule **III** 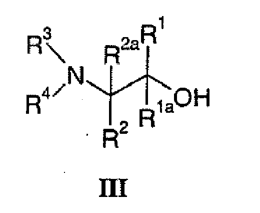 dans laquelle R¹, R^{1a}, R², R^{2a}, R³ et R⁴ sont tels que définis dans la revendication 1 sauf que toute fonction est protégée le cas échéant ;
ou
(b) pour la préparation des composés de formule **I**
dans laquelle Y est OC(R⁷)₂, SC(R⁷)₂ ou N(R⁷)C(R⁷)₂, la réaction, commodément en présence d'une base convenable, d'une quinazoline de formule **IV** : 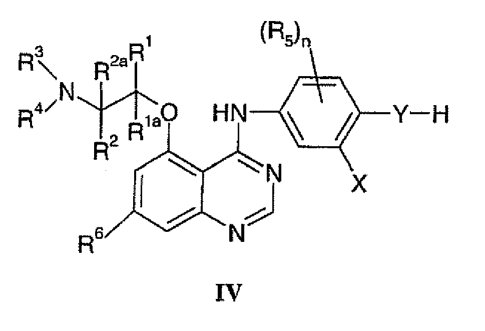 dans laquelle Y est 0, S ou N(R⁷), et X, R¹, R^{1a}, R², R^{2a}, R³, R⁴, R⁵, R⁶, R⁷ et n sont tels que définis dans la revendication 1, sauf que toute fonction est protégée le cas échéant, avec un composé de formule **V** :
Q¹-C(R⁷)₂-L**^{V}** **V**
dans laquelle **L¹** est un groupement déplaçable convenable et Q¹ et R⁷ sont tels que définis dans la revendication 1 sauf que toute fonction est protégée le cas échéant ;
ou
(c) la réaction d'une quinazoline de formule **VI** : 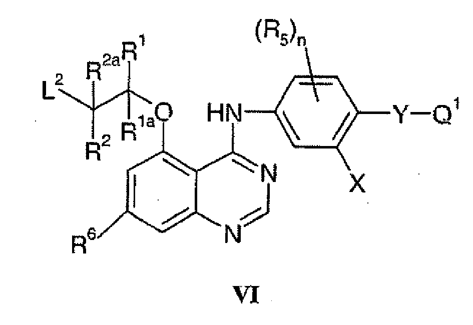 dans laquelle **L²** est un groupement déplaçable convenable et Q¹, X, Y, R¹, R^{1a}, R², R^{2a}, R⁵, R⁶ et n sont tels que définis dans la revendication 1 sauf que toute fonction est protégée le cas échéant, avec une amine de formule **VII** :
NHR³R⁴ **V**
dans laquelle R³ et R⁴ sont tels que définis dans la revendication 1 sauf que toute fonction est protégée le cas échéant ;
ou
(d) pour la préparation des composés de formule **I**
dans laquelle R^{2a} est hydrogène, l'amination réductrice en présence d'un agent de réduction convenable de l'aldéhyde ou de la cétone de formule **VII** : 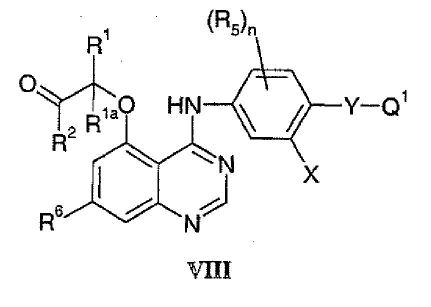 dans laquelle Q¹, X, Y, R¹, R^{1a}, R², R⁵, R⁶ et n sont tels que définis dans la revendication 1 sauf que toute fonction est protégée le cas échéant, avec une amine de formule **VII** :
NHR³R⁴ **VII**
dans laquelle R³ et R⁴ sont tels que définis dans la revendication 1 sauf que toute fonction est protégée le cas échéant ;
ou
(e) pour la préparation des composés de formule **I**
dans laquelle Y est 0 ou N(R⁷) et Q¹ est 2-pyridyle
ou 4-pyridyle, la réaction, en présence d'un catalyseur convenable, d'une quinazoline de formule **IV** : 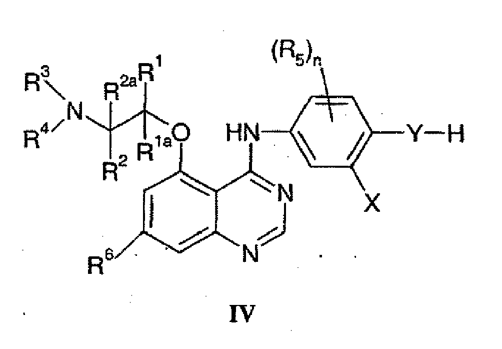 dans laquelle **Y** est O ou N(R⁷) et X, R¹, R^{1a}, R², R^{2a}, R³, R⁴, R⁵, R⁶, et n sont tels que définis dans la revendication 1 sauf que toute fonction est protégée le cas échéant, avec une amine de formule **IVa** ou de formule **IVb :** 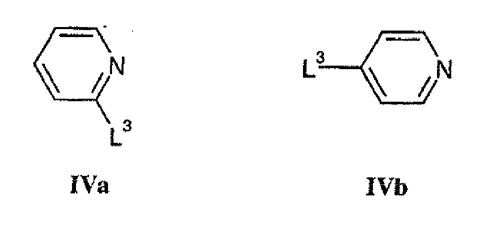 dans lesquelles L³ est un groupement déplaçable convenable ;
ou
(f) la réaction, commodément en présence d'une phosphine convenable ou d'un composé diazo convenable, d'une quinazoline de formule **II :** 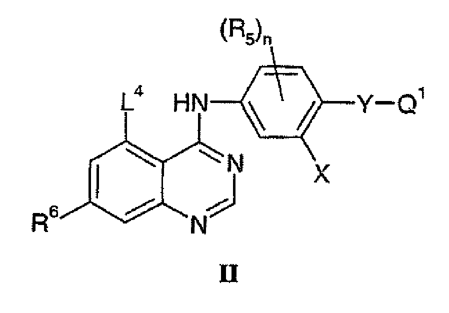 dans laquelle R⁵, R⁶, Q¹, X, Y et n sont tels que définis dans la revendication 1 sauf que toute fonction est protégée le cas échéant, et L⁴ est hydroxy, avec un alcool de formule **III :** 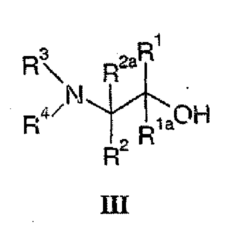 dans laquelle R¹, R^{1a}, R², R^{2a}, R³ et R⁴ sont tels que définis dans la revendication 1 sauf que toute fonction est protégée le cas échéant ; et ensuite, le cas échéant :
(i) la transformation d'un dérivé de la quinazoline de formule I en un autre dérivé de la quinazoline de formule I ;
(ii) l'élimination de tout groupement protecteur qui est présent par des moyens classiques ;
(iii) la formation d'un sel pharmaceutiquement acceptable.
